# EUROPÄISCHE PATENTSCHRIFT

(11) **EP 0 282 042 B1**
(45) Veröffentlichungstag und Bekanntmachung des Hinweises auf die Patenterteilung: **08.06.1994**
(21) Anmeldenummer: 88103740.2
(22) Anmeldetag: 09.03.1988
(51) Int. Cl.: C07K 13/00, C07K 3/18, C12P 21/02, C12N 15/00, C07K 15/26, C12N 9/02, C12P 21/06, A61K 37/02

(54) **Neue Fusionsproteine und deren Reinigung**
Fusion proteins and their purification
Protéines fusionnées et leur purification

(30) Priorität: 10.03.1987 CH 895/87
(43) Veröffentlichungstag der Anmeldung: 14.09.1988
(73) Patentinhaber: F. HOFFMANN-LA ROCHE AG, 4002 Basel (CH)
(72) Erfinder: Döbeli, Heinz, Dr., CH-4417 Ziefen (CH); Eggimann, Bernhard, Dr., CH-4051 Basel (CH); Gentz, Reiner, Dr., D-7888 Rheinfelden (DE); Hochuli, Erich, Dr., CH-4411 Arisdorf (CH); Stüber, Dietrich, Dr., D-7889 Grenzach-Wyhlen (DE)
(74) Vertreter: Lederer, Franz, Dr.

(56) Entgegenhaltungen:
- EP-A- 0 184 355
- EP-A- 0 253 303
- US-A- 4 569 794
- TRENDS IN BIOTECHNOLOGY, Band 3, Nr. 1, Januar 1985, Seiten 1-7, Elsevier Science Publishers B.V., Amsterdam, NL; E. SULKOWSKI: "Purification of proteins by IMAC"
- BIOCHEMISTRY, Band 22, 1983, Seiten 1621-1630, American Chemical Society, Washington, DC, US; J. PORATH et al.: "Immobilized metal ion affinity adsorption and immobilized metal ion affinity chromatography of biomaterials. Serum protein affinities for gel-immobilized iron and nickel ions"
- BIOTECHNOLOGY, Band 6, Nr. 11, November 1988, Seiten 1321-1325; E. HOCHULI et al.: "Genetic approach to facilitate purification of recombinant proteins with a novel metal chelate adsorbent"
- Biologically Active Molecules, E. Hochuli, Springer-Verlag, Berlin Heidelberg 1989, pp. 217-239
- Journal of Chromatography, 516, 333-354, 1990, M. Belew et al.

## Beschreibung

Die Möglichkeiten der Gentechnologie, Hybridgene herzustellen, eröffnen neue Wege zur Aufarbeitung von rekombinanten Proteinen. Durch Verknüpfung der kodierenden Gensequenz eines gewünschten Proteins mit der kodierenden Gensequenz eines Proteinfragments mit hoher Affinität für einen Liganden (Affinitätspeptid) ist es möglich, gewünschte rekombinante Proteine in Form von Fusionsproteinen in einem Schritt unter Ausnutzung des Affinitätspeptids zu reinigen. Durch gezielte Mutagenese ("site-directed mutagenesis") ist es ferner möglich, spezifische chemische oder enzymatische Schnittstellen am Verknüpfungspunkt von Affinitätspeptid und gewünschtem rekombinantem Protein einzuführen, so dass nach der Reinigung des Fusionsproteins mittels eines geeigneten Affinitätsharzes das gewünschte rekombinante Protein durch chemische oder enzymatische Spaltung erhältlich ist. Derartige Reinigungsmethoden sind beispielsweise bekannt aus Science 198, 1056-1063 (1977) (Itakura et al.), Proc. Natl. Acad. Sci. U.S.A. 80, 6848-6852 (1983) (Germino et al.), Nucleic Acids Res. 13, 1151-1162 (1985) (Nilsson et al.), Gene 32, 321-327 (1984) (Smith et al.), sowie aus den europäischen Patentanmeldungen, Publikations-Nummern 150126 und 184 355.

Die Europäische Patentanmeldung, Publikations-Nummer184 355, offenbart ein Verfahren zur Reinigung von Fusionsproteinen, die aus einem Affinitätspeptid, welches mindestens einen Histidinrest aufweist, und einem biologisch aktiven Protein bestehen. Verschiedene Beispiele für bevorzugte Affinitätspeptide werden offenbart, die u.a. die Peptidsequenz His-Gly-His mitumfassen. Als Harze für die Reinigung der Fusionsproteine werden Chelatharze genannt, insbesondere Chelatharze mit Iminodiessigsäure (IDA) als komplexbildender Komponente.

Die Reinigung von Proteinen mit nicht-benachbarten Histidinresten mittels Chelatharzen mit IDA als komplexbildender Komponente wird auch in Trends in Biotechnology 3, 1-7 (1985) (Sulkowski) beschrieben.

Affinitätspeptide mit möglichst grosser Affinität zu Metallchelatharzen, die die problemlose Reinigung von nativen und denaturierten Proteinen ermöglichen, sind aus keiner der vorstehend genannten Publikationen bekannt.

Erfindungsgemäss wurde nun gefunden, dass Affinitätspeptide mit mindestens zwei benachbarten Histidinresten für die Reinigung von rekombinanten Proteinen mittels Metallchelat Affinitäts-Chromatographie an Nitrilotriessigsäure (NTA)-Harzen besonders geeignet sind. Diese Affinitätspeptide zeichnen sich gegenüber den bekannten vor allem dadurch aus, dass sie die problemlose Reinigung von nativen und denaturierten Proteinen mittels NTA-Harzen ermöglichen.

Die vorliegende Erfindung betrifft daher Fusionsproteine, die aus einem oder zwei Affinitätspeptiden, welche benachbarte Histidinreste enthalten, und einem an diese Affinitätspeptide direkt oder indirekt gebundenen biologisch aktiven Polypeptid oder Protein bestehen, Verfahren zu deren Herstellung mittels rekombinanter DNA-Technologie sowie Verfahren zu deren Reinigung mittels Metallchelat-Affinitätschromatographie an NTA-Harzen. Die vorliegende Erfindung betrifft ferner Gene, die für diese Fusionsproteine kodieren, Expressionsvektoren, die diese Gene enthalten, mit diesen Expressionsvektoren transformierte Mikroorganismen sowie Verfahren zur Herstellung solcher Gene, Expressionsvektoren und transformierter Mikroorganismen.

Die Affinitätspeptide der erfindungsgemässen Fusionsproteine sind durch die allgemeinen Formel

R¹-(His)₂₋₆-R²

definiert,
worin R¹ Wasserstoff, eine Aminosäure oder eine Sequenz von mehreren Aminosäuren ist, R² Q, Q-Ile-Glu-Gly-Arg- oder Q-Asp-Asp-Asp-Asp-Lys- darstellt und Q eine Peptidbindung, eine Aminosäure oder eine Sequenz von mehreren, max. 30 Aminosäuren ist.
Besonders bevorzugte Affinitätspeptide der erfindungsgemässen Fusionsproteine sind solche mit den Peptidsequenzen der Formeln

Met-His-His,
Met-His-His-His,
Met-His-His-His-His,
Met-His-His-His-His-His,
Met-His-His-His-His-His-His,
Met-His-His-Ala-Gly-Ile-Glu-Gly-Arg
und Met-His-His-Ala-Gly-Asp-Asp-Asp-Asp-Lys.

Die Affinitätspeptide der erfindungsgemässen Fusionsproteine können direkt oder indirekt an die biologisch aktiven Polypeptide oder Proteine gebunden sein. Bei der Verwendung eines einzelnen Affinitätspeptids kann dieses entweder an die aminoterminale oder an die carboxyterminale Aminosäure der biologisch aktiven Polypeptide oder Proteine gebunden sein. Bei der Verwendung von zwei Affinitätspeptiden wird das eine an die aminoterminale und das andere an die carboxyterminale Aminosäure der biologisch aktiven Polypeptide oder Proteine gebunden.

Bei indirekter Bindung enthalten die Affinitätspeptide eine geeignete, selektive Schnittstelle über die sie an das gewünschte biologisch aktive Polypeptid oder Protein gebunden sind. Als geeignete, selektive Schnittstellen kommen vorzugsweise die Aminosäuresequenzen -(Asp)ₙ-Lys-, worin n 2, 3 oder 4 bedeutet, oder -Ile-Glu-Gly-Arg- in Frage, die von den Proteasen Enterokinase bzw. Koagulations-Faktor Xₐ spezifisch erkannt werden. Solche Affinitätspeptide werden dann in an sich bekannter Weise enzymatisch abgespalten.

Bei direkter Bindung bleiben die Affinitätspeptide mit dem gewünschten biologisch aktiven Polypeptid oder Protein verbunden, d.h. die Affinitätspeptide können nicht chemisch oder enzymatisch abgespalten werden. Diese Art der Bindung ist dann vorteilhaft, wenn durch die Anwesenheit des Affinitätspeptids die Aktivität des gewünschten Polypeptids oder Proteins nicht nachteilig beeinflusst wird. Solche erfindungsgemässen Fusionsproteine können für eine Anzahl von immunologischen Verfahren verwendet werden. Sie können beispielsweise als Reagenzien zur Bestimmung von Infektionskrankheiten verwendet werden. Indem man sie einem physiologisch verträglichen Trägermaterial beimischt, können sie auch als Impfstoffe zur Verhütung von Krankheiten verwendet werden.

Der Ausdruck "biologisch aktive Polypeptide oder Proteine" der im Zusammenhang mit den erfindungsgemässen Fusionsproteinen verwendet wird, bezieht sich auf solche Polypeptide oder Proteine, die selbst biologisch aktiv sind oder aber auf Polypeptide oder Proteine, die für die Herstellung von biologisch aktiven Polypeptiden oder Proteinen verwendet werden können.

Als biologisch aktive Polypeptide oder Proteine kommen beispielsweise Malaria-Oberflächenantigene, insbesondere das 5.1-Oberflächenantigen, das CS-Protein und das p190-Protein von Plasmodium falciparum, Lymphokine, Interferone, Insulin und Insulin-Vorläufer, HIV-1 und HIV-2 Hüll- und Strukturproteine, Wachstumshormone und Wachstumshormon-Releasing-Faktoren in Frage. Besonders bevorzugte biologisch aktive Polypeptide oder Proteine der erfindungsgemässen Fusionsproteine sind solche mit der Aminosäuresequenz von Human-Immun-Interferon und Teilsequenzen von Human-Immun-Interferon, insbesondere solche mit den Aminosäuresequenzen der Formeln: 
Gln-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Ser-Gln-Met-Leu-
Phe-Arg-Gly-Arg-Arg-Ala-Ser-Gln,

Gln-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Ser-Gln-Met-Leu
und

Gln-Asn-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Ser-Gln-Met-Leu
sowie solche mit der Aminosäuresequenz der Dihydrofolatreduktase der Maus.

Die Herstellung der erfindungsgemässen Fusionsproteine kann nach bekannten, in der Literatur beschriebenen Methoden der rekombinanten DNA Technologie erfolgen. Vorzugsweise werden zunächst die für die gewünschten Affinitätspeptide kodierenden Nukleotidsequenzen synthetisiert und diese dann mit einer für das gewünschte biologisch aktive Polypeptid oder Protein kodierenden Nukleotidsequenz verknüpft.

Der Einbau des so erhaltenen Hybridgens in Expressionsvektoren, beispielsweise die Plasmide pDS8/RBSII,SphI; pDS5/RBSII,3A+5A; pDS78/RBSII oder pDS56/RBSII und andere handelsübliche bzw. allgemein zugängliche Plasmide erfolgt ebenfalls in an sich bekannter Weise. Hierzu kann auf das Lehrbuch von Maniatis et al. ("Molecular Cloning", Cold Spring Harbor Laboratory, 1982) verwiesen werden.

Die Methoden zur Expression der erfindungsgemässen Fusionsproteine sind ebenfalls an sich bekannt und in dem vorstehend genannten Lehrbuch eingehend beschrieben. Sie umfassen die folgenden Massnahmen:
(a) Transformation eines geeigneten Wirtsorganismus, vorteilhaft E. coli, mit einem Expressionsvektor in welchem ein vorstehend genanntes Hybridgen operativ an eine Expressionskontrollsequenz gebunden ist;
(b) Kultivierung des so erhaltenen Wirtsorganismus unter geeigneten Wachstumsbedingungen; und
(c) Extraktion und Isolierung des gewünschten Fusionsproteins aus dem Wirtsorganismus.

Als Wirtsorganismen kommen gram-negative und gram-positive Bakterien in Frage, beispielsweise E. coli und B. subtilis Stämme. Ein besonders bevorzugter Wirtsorganismus der vorliegenden Erfindung ist der E. coli Stamm M15 (s. S.17). Ausser dem oben genannten E. coli Stamm können aber auch andere allgemein zugängliche E. coli Stämme, beispielsweise E. coli 294 (ATCC No. 3144), E. coli RR1 (ATCC No. 31343) und E. coli W3110 (ATCC No. 27325) verwendet werden.

Ideale Metallchelatharze für die Reinigung der erfindungsgemässen Fusionsproteine sind Nitrilotriessigsäure-(NTA)-Harze der allgemeinen Formel

Trägermatrix-Spacer-NH-(CH₂)ₓ-CH(COOH)-N(CH₂COO⁻)₂ Ni²⁺,

worin x 2, 3 oder 4 bedeutet.

Als Trägermatrix kommen Materialien in Frage, wie sie in der Affinitäts- und Gelchromatographie verwendet werden, beispielsweise vernetzte Dextrane, Agarose (insbesondere in der unter dem Warenzeichen Sepharose® bekannten Form) oder Polyacrylamide.

Als Spacer kommen die aus der Affinitäts-Chromatographie bereits bekannten Spacer-Gruppen in Frage, wobei die Gruppen -O-CH₂-CH(OH)-CH₂- und -O-CO- bevorzugt sind.

Ein besonders bevorzugtes NTA-Harz für die Reinigung der erfindungsgemässen Hybridproteine ist das der Formel

[Sepharose®CL 6B]-O-CH₂-CH(OH)-CH₂-NH-(CH₂)₄-CH(COOH)-N(CH₂COO⁻)₂ Ni²⁺

dessen Herstellung wie in Beispiel 10 beschrieben erfolgen kann.

Für die Reinigung der erfindungsgemässen Fusionsproteine können die vorstehend genannten NTA-Harze batch-weise oder in kontinuierlich zu betreibenden Säulen verwendet werden. Die NTA-Harze werden vor der Beladung mit erfindungsgemässem Fusionsprotein zweckmässigerweise mit einem wässrigen Puffer, der selber keine Chelate mit Nickel bildet, vorzugsweise einem Tris·HCl Puffer, pH 7,5, äquilibriert. Der Aequilibrierungspuffer (wie auch die Elutionspuffer) kann ein Denaturierungsmittel bzw. ein Detergenz, beispielsweise Guanidin·HCl, Harnstoff oder Triton enthalten. Der Zusatz eines solchen Denaturierungsmittels oder Detergenzes ermöglicht problemloses Arbeiten selbst mit erfindungsgemässen Fusionsproteinen, die in wässriger Lösung extrem schwer löslich sind. Die Elution der erfindungsgemässen Fusionsproteine kann bei konstantem pH-Wert oder mit linear oder diskontinuierlich fallenden pH-Gradienten durchgeführt werden. Die optimalen Elutionsbedingungen hängen von der Menge und Art der vorhandenen Verunreinigungen, der Menge des zu reinigenden Materials, den Säulendimensionen usw. ab und sind zweckmässigerweise von Fall zu Fall zu bestimmen.

Die folgenden Beispiele illustrieren die Herstellung erfindungsgemässer Fusionsproteine, deren Reinigung mittels Metallchelatchromatographie sowie die Herstellung von biologisch aktiven Polypeptiden oder Proteinen durch enzymatische Spaltung der gereinigten, erfindungsgemässen Fusionsproteine.

Diese Beispiele können besser verstanden werden, wenn sie im Zusammenhang mit den begleitenden Abbildungen gelesen werden. In diesem Abbildungen treten die folgenden Abkürzungen und Symbole auf:
B, Bg, E, H, N, Na, Nd, P, S, Sa, Sc, X und Xb bezeichnen Schnittstellen für die Restriktionsenzyme BamHI, BglII, EcoRI, HindIII, NaeI, NarI, NdeI, PstI, SphI, SalI, ScaI, XhoI bzw. XbaI. 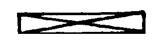
repräsentiert die Promotoren der Gene bla, lacI und neo; 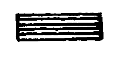
repräsentiert die ribosomalen Bindungsstellen der Gene bla, cat, neo und lacI; 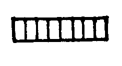
repräsentiert die Terminatoren tₒ und T1; 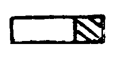
repräsentiert das regulierbare Promotor/Operator Element P_{N25}x_{/O} bzw. N250PSN25OP29; 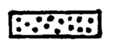
repräsentiert die ribosomalen Bindungsstellen RBSII,SphI und RBSII,3A+5A; → repräsentiert die kodierenden Regionen unter Kontrolle dieser ribosomalen Bindungsstellen; 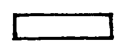
repräsentiert die Regionen, die für die erfindungsgemässen Affinitätspeptide sowie die selektiven Schnittstellen kodieren; 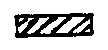
repräsentiert die Regionen, die für 2, 4 bzw. 6 Histidinreste kodieren; 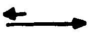
repräsentiert die für die Replikation benötigte Region (repl.); 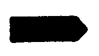
repräsentiert kodierende Regionen für Dihydrofolatreduktase (dhfr), Chloramphenicolacetyltransferase, lac-Repressor (lacI), β-Lactamase (bla), Neomycinphosphotransferase (neo) und die verschiedenen Derivate des γ-Interferons.

### Abbildung 1

Schematische Darstellung des Plasmids pDS8/RBSII,SphI.

### Abbildung 2

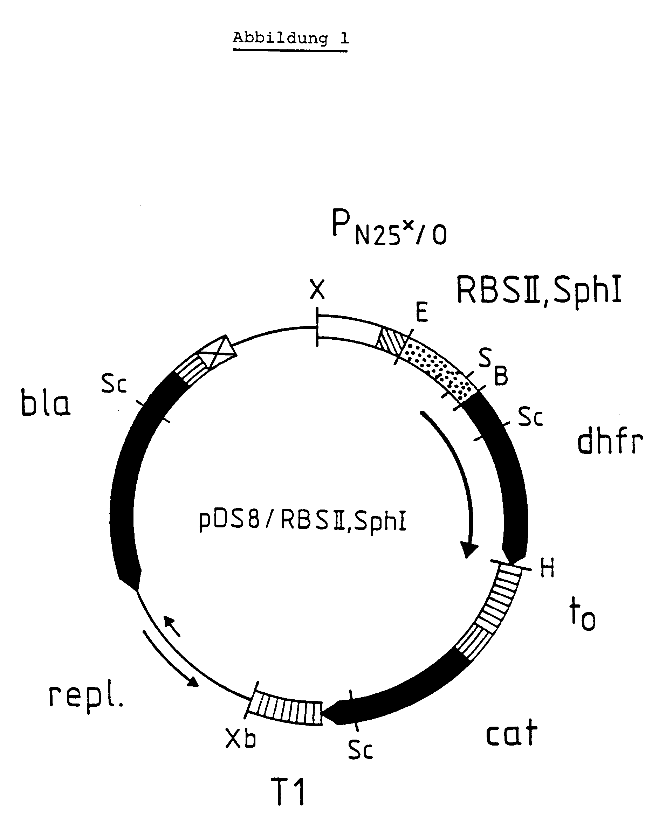

Nukleotidsequenz des XhoI/XbaI Fragments des Plasmids pDS8/RBSII,SphI. Dieses Fragment enthält das regulierbare Promotor/Operator Element P_{N25}x_{/O}, die ribosomale Bindungsstelle RBSII,SphI, das dhfr-Gen, den Terminator tₒ, das cat-Gen sowie den Terminator T1. Die in Abbildung 1 aufgeführten Schnittstellen für Restriktionsenzyme sind überstrichen während die unter der Kontrolle der RBSII,SphI stehende Region, die für eine Variante der Dihydrofolatreduktase kodiert, unterstrichen ist. Zusätzlich ist der aus dem Plasmid pRB322 stammende Teil des Plasmids pDS8/RBSII,SphI schematisch dargestellt, wobei sich die angegebenen Zahlen auf die Sequenz von pBR322 beziehen (J.G. Sutcliffe, Cold Spring Harbor Symp. Quant. Biol. 43, pp. 77-90 [1979]).

### Abbildung 3

Schematische Darstellung des Plasmids pDS5/RBSII,3A+5A.

### Abbildung 4

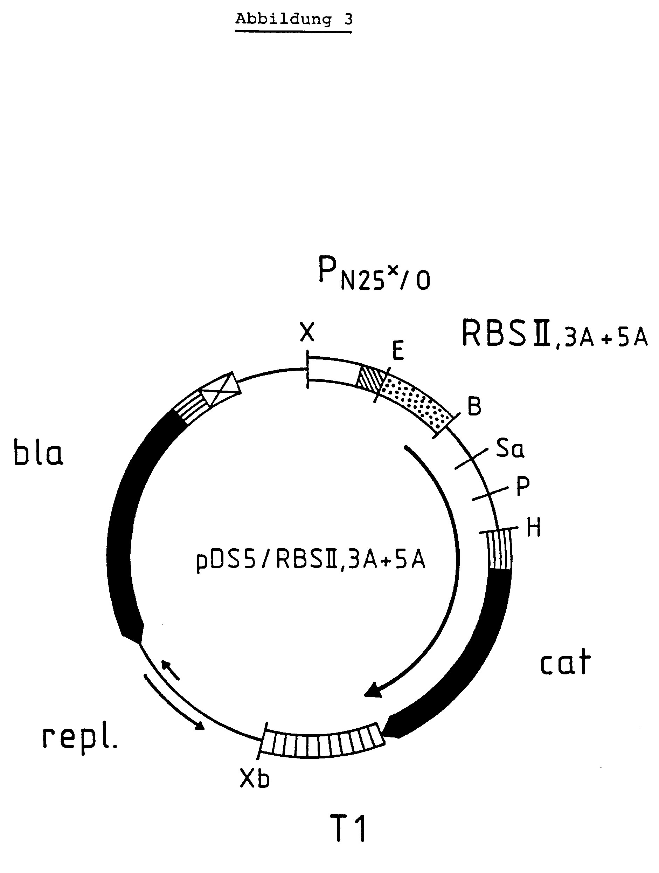

Nukleotidsequenz des Plasmids pDS5/RBSII,3A+5A. Die in Abbildung 3 aufgeführten Schnittstellen für Restriktionsenzyme sind überstrichen während die unter Kontrolle der RBSII,3A+5A stehende Region, die für eine Variante der Chloramphenicoltransferase kodiert, unterstrichen ist.

### Abbildung 5

Schematische Darstellung des Plasmids pDS78/RBSII.

### Abbildung 6

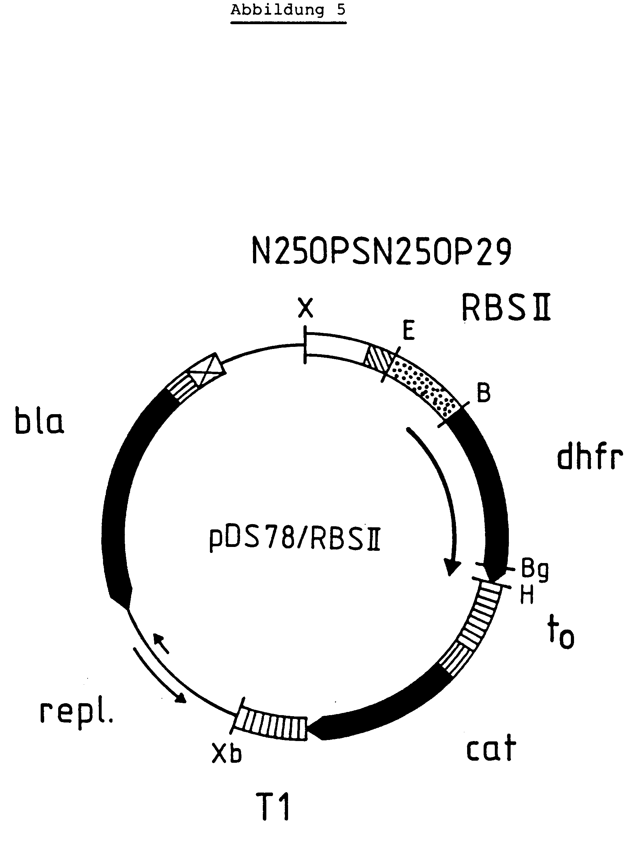

Nukleotidsequenz des Plasmids pDS78/RBSII. Die in Abbildung 5 aufgeführten Schnittstellen für Restriktionsenzyme sind überstrichen während die unter Kontrolle der RBSII stehende Region, die für eine Variante der Dihydrofolatreduktase kodiert, unterstrichen ist.

### Abbildung 7

Schematische Darstellung des Plasmids pDS56/RBSII.

### Abbildung 8

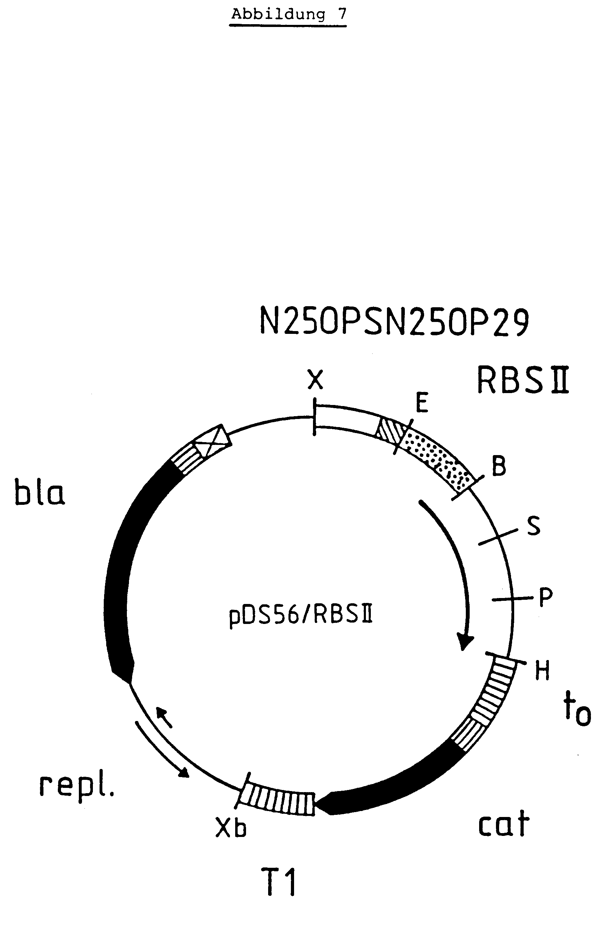

Nukleotidsequenz des Plasmids pDS56/RBSII. Die in Abbildung 7 aufgeführten Schnittstellen für Restriktionsenzyme sind überstrichen während die unter Kontrolle der RBSII stehende Region unterstrichen ist.

### Abbildung 9

Schematische Darstellung des Plasmids pDMI,1.

### Abbildung 10

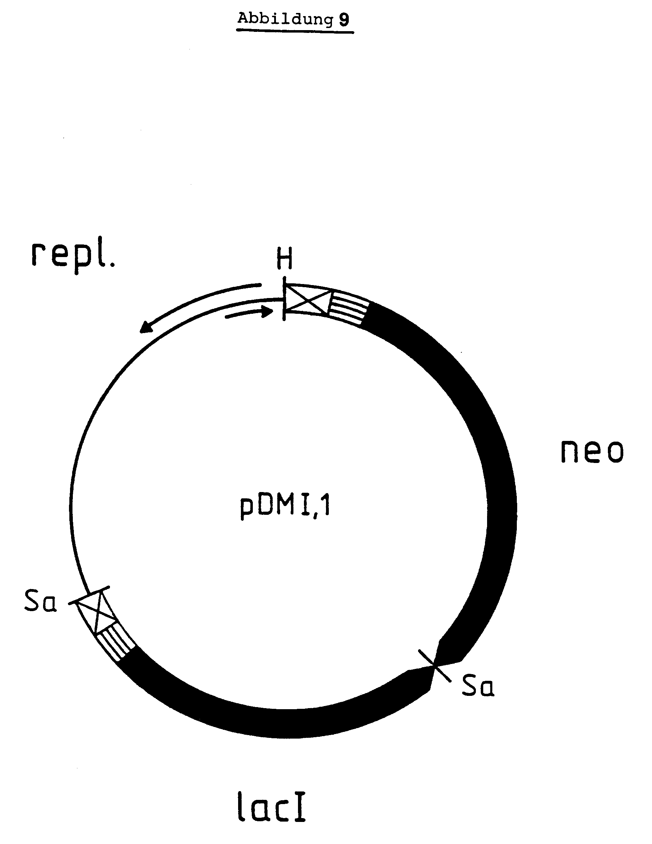

Nukleotidsequenz des Plasmids pDMI,1. Die in Abbildung 9 aufgeführten Schnittstellen für Restriktionsenzyme sind überstrichen während die für Neomycinphosphotransferase (neo) bzw. lac-Repressor (lacI) kodierenden Regionen unterstrichen sind.

### Abbildung 11

Nukleotidsequenzen der zur Konstruktion der in den Beispielen verwendeten Plasmide eingesetzten Oligonukleotide. Jeweils zwei solcher Oligonukleotide wurden zusammengefasst und als Adaptor bezeichnet. Die Schnittstellen für die Restriktionsenzyme NaeI, NarI und BglII sind überstrichen.

### Abbildung 12

Schematische Darstellung der Konstruktion und Isolierung des Fragments 1. Dieses Fragment wurde aus dem Plasmid pRC23/IFI-900 isoliert und enthält das Gen für das rekombinante humane Interferon mit Cys-Tyr-Cys als N-terminale Aminosäuren.

### Abbildung 13

Schematische Darstellung der Konstruktion des Plasmids pGLS durch Einbau des Fragments 1 in das Plasmid pDS8/RBSII,SphI. In der schematischen Darstellung von pGLS bezeichnet (Sc) die Position an der das Fragment 1 über die Schnittstelle für das Restriktionsenzym ScaI mit dem Plasmid pDS8/RBSII,SphI verknüpft wurde.

### Abbildung 14

Schematische Darstellung der Konstruktion und Isolierung des Fragments 2. Dieses Fragment kodiert für ein humanes Interferon, das am C-Terminus um 8 Aminosäuren verkürzt ist und als IFN-γ(-8) bezeichnet wird. In den angegebenen Nukleotidsequenzen ist das entsprechende Terminationscodon unterstrichen.

### Abbildung 15

Schematische Darstellung der Konstruktion des Plasmids pIFN-γ(-8) durch Einbau des Fragments 2 in das Plasmid pDS8/RBSII,SphI über die Schnittstellen für die Restriktionsenzyme EcoRI und HindIII.

### Abbildung 16

Schematische Darstellung der Konstruktion und Isolierung des Fragments 3. Dieses Fragment trägt das regulierbare Promotor/Operator Element P_{N25}x_{/O}, die ribosomale Bindungsstelle RBSII,SphI und den Adator 3, der für die Aminosäuresequenz Met-His-His-Ala-Gly-Ile-Glu-Gly-Arg-Leu-Gly-Ser kodiert.

### Abbildung 17

Schematische Darstellung der Konstruktion des Plasmids pDS8/RBSII,SphI-His,His-Xa-BamHI durch Einbau des Fragments 3 in das Plasmid pDS8/RBSII,SphI über die Schnittstellen für die Restriktionsenzyme XhoI und BamHI.

### Abbildung 18

Schematische Darstellung der Konstruktion und Isolierung des Fragments 4, welches bei der Konstruktion des Plasmids pHis,His-Xa-IFN-γ eingesetzt wurde.

### Abbildung 19

Schematische Darstellung der Isolierung des Fragments 5, welches bei der Konstruktion der Plasmide pHis,His-Xa-IFN-γ, pHis,His-Ek-IFN-γ(-8) und pHis,His-Xa-IFN-γ(-8)(Asn) eingesetzt wurde.

### Abbildung 20

Schematische Darstellung der Isolierung des Fragments 6, welches bei der Konstruktion des Plasmids pHis,His-Xa-IFN-γ eingesetzt wurde.

### Abbildung 21

Schematische Darstellung der Konstruktion des Plasmids pHis,His-Xa-IFN-γ durch Verknüpfen der Fragmente 4, 5 und 6. Das Plasmid pHis,His-Xa-IFN-γ kodiert für ein IFN-γ Fusionsprotein mit Met-His-His-Ala-Gly-Ile-Glu-Gly-Arg als zusätzlicher N-terminaler Aminosäuresequenz (His,His-Xa-IFN-γ).

### Abbildung 22

Schematische Darstellung der Konstruktion und Isolierung des Fragments 7, welches bei der Konstruktion des Plasmids pHis,His-Ek-IFN-γ(-8) eingesetzt wurde.

### Abbildung 23

Schematische Darstellung der Isolierung des Fragments 8, welches bei der Konstruktion der Plasmide pHis,His-Ek-IFN-γ(-8) und pHis,His-Xa-IFN-γ(-8)(Asn) eingesetzt wurde.

### Abbildung 24

Schematische Darstellung der Konstruktion des Plasmids pHis-His-Ek-IFN-γ(-8) durch Verknüpfen der Fragmente 5, 7 und 8. Das Plasmid pHis,His-Ek-IFN-γ(-8) kodiert für ein am C-Terminus um 8 Aminosäuren verkürztes IFN-γ Fusionsprotein mit Met-His-His-Ala-Gly-Asp-Asp-Asp-Asp-Lys als zusätzlicher N-terminaler Aminosäuresequenz (His,His-Ek-IFN-γ(-8)).

### Abbildung 25

Schematische Darstellung der Konstruktion und Isolierung des Fragments 9, welches bei der Konstruktion des Plasmids pHis,His-Xa-IFN-γ(-8)(Asn) eingesetzt wurde.

### Abbildung 26

Schematische Darstellung der Konstruktion des Plasmids pHis,His-Xa-IFN-γ(-8)(Asn) durch Verknüpfen der Fragmente 5, 8 und 9. Dieses Plasmid kodiert für ein IFN-γ Fusionsprotein, das am C-Terminus um 8 Aminosäuren verkürzt ist, das am N-Terminus um die Aminosäuresequenz Met-His-His-Ala-Gly-Ile-Glu-Gly-Arg verlängert ist und bei dem zusätzlich an der Position 2 die Aminosäure Asp durch die Aminosäure Asn ersetzt ist (His,His-Xa-IFN-γ(-8)(Asn)).

### Abbildung 27

Schematische Darstellung der Konstruktion und Isolierung des Fragments 10, welches bei der Konstruktion des Plasmids p6xHis-DHFR eingesetzt wurde.

### Abbildung 28

Schematische Darstellung der Konstruktion des Plasmids p6xHis-DHFR durch Verknüpfen des Fragments 10 mit dem die Replikationsregion enthaltenden XhoI/BamHI-Fragment des Plasmids pDS78/RBSII. Das Plasmid p6xHis-DHFR kodiert für ein DHFR-Fusionsprotein mit 6 Histidinen am N-Terminus [(His)₆-mDHFR].

### Abbildung 29

Schematische Darstellung der Konstruktion und Isolierung des Fragments 11, welches bei der Konstruktion des Plasmids p4xHis-DHFR eingesetzt wurde.

### Abbildung 30

Schematische Darstellung der Konstruktion des Plasmids p4xHis-DHFR durch Verknüpfen des Fragments 11 mit dem die Replikationsregion enthaltenden XhoI/BamHI-Fragment des Plasmids pDS78/RBSII. Das Plasmid p4xHis-DHFR kodiert für ein DHFR-Fusionsprotein mit 4 Histidinen am N-Terminus [(His)₄-mDHFR].

### Abbildung 31

Schematische Darstellung der Konstruktion und Isolierung des Fragments 12, welches bei der Konstruktion des Plasmids pRBSII-6xHis eingesetzt wurde.

### Abbildung 32

Schematische Darstellung der Konstruktion des Plasmids pRBSII-6xHis durch Verknüpfen des Fragments 12 mit dem die Replikationsregion enthaltenden XbaI/BamHI-Fragment des Plasmids pDS56/RBSII.

### Abbildung 33

Schematische Darstellung der Konstruktion und Isolierung des Fragments 13, welches bei der Konstruktion des Plasmids pRBSII-4xHis eingesetzt wurde.

### Abbildung 34

Schematische Darstellung der Konstruktion des Plasmids pRBSII-4xHis durch Verknüpfen des Fragments 13 mit dem die Replikationsregion enthaltenden XbaI/BamHI-Fragment des Plasmids pDS56/RBSII.

### Abbildung 35

Schematische Darstellung der Konstruktion und Isolierung des Fragments 14, welches bei der Konstruktion des Plasmids pRBSII-2xHis eingesetzt wurde.

### Abbildung 36

Schematische Darstellung der Konstruktion des Plasmids pRBSII-2xHis durch Verknüpfen des Fragments 14 mit dem die Replikationsregion enthaltenden XbaI/BamHI-Fragment des Plasmids pDS56/RBSII.

### Abbildung 37

Schematische Darstellung der Konstruktion des Plasmids pDHRF-6xHis durch Verknüpfen des die Replikationsregion enthaltenden XbaI/BglII-Fragments des Plasmids pDS78/RBSII mit dem das cat-Gen enthaltenden BglII/XbaI-Fragment des Plasmids pRBSII-6xHis. Das Plasmid pDHFR-6xHis kodiert für ein DHFR-Fusionsprotein mit 6 Histidinen am C-Terminus [Met-mDHFR-(His)₆].

### Abbildung 38

Schematische Darstellung der Konstruktion des Plasmids pDHFR-2xHis durch Verknüpfen des die Replikationsregion enthaltenden XbaI/BglII-Fragments des Plasmids pDS78/RBSII mit dem das cat-Gen enthaltenden BglII/XbaI-Fragment des Plasmids pRBSII-2xHis. Das Plasmid pDHFR-2xHis kodiert für ein DHFR-Fusionsprotein mit 2 Histidinen am C-Terminus [Met-mDHFR-(His)₂].

### Abbildung 39

Schematische Darstellung der Konstruktion des Plasmids p4xHis-DHFR-4xHis durch Verknüpfen des die Replikationsregion enthaltenden XbaI/BglII-Fragments des Plasmids p4xHis-DHFR mit dem das cat-Gen enthaltenden BglII/XbaI-Fragment des Plasmids pRBSII-4xHis. Das Plasmid p4xHis-DHFR-4xHis kodiert für ein DHFR-Fusionsprotein mit je 4 Histidinen am N- und am C-Terminus [(His)₄-mDHFR-(His)₄].

### Abbildung 40

Nukleotidsequenz des vom Plasmid pGLS kodierten IFN-γ-Gens und die davon abgeleitete Aminosäuresequenz.

### Abbildung 41

Nukleotidsequenz des vom Plasmid pHis,His-Xa-IFN-γ kodierten IFN-γ Fusionsgens und die davon abgeleitete Aminosäuresequenz.

### Abbildung 42

Nukleotidsequenz des vom Plasmid pHis,His-Ek-IFN-γ(-8) kodierten IFN-γ Fusionsgens und die davon abgeleitete Aminosäuresequenz.

### Abbildung 43

Nukleotidsequenz des vom Plasmid pHis,His-Xa-IFN-γ(-8)(Asn) kodierten IFN-γ Fusionsgens und die davon abgeleitete Aminosäuresequenz.

### Beispiel 1

### Beschreibung der Plasmide, welche bei der Konstruktion der Plasmide pGLS, pHis,His-Xa-IFN-γ, pHis,His-Ek-IFN-γ(-8), pHis,His-Xa-IFN-γ(-8)(Asn), p6xHis-DHFR, p4xHis-DHFR-4xHis, pDHFR-2xHis und pDHFR-6xHis verwendet wurden

### A. Prinzipien

Die Plasmide pDS8/RBSII,SphI (Abb. 1 und 2), pDS5/RBSII,3A+5A (Abb. 3 und 4), pDS78/RBSII (Abb. 5 und 6) und pDS56/RBSII (Abb. 7 und 8) wurden zur Konstruktion der aufgeführten Plasmide verwendet. Mit diesen Plasmiden transformierte E. coli Zellen wurden bei der Deutschen Sammlung von Mikroorganismen in Göttingen (seit 21.11.87 in Braunschweig) am 03.10.85 [E. coli M15 (pDS5/RBSII,3A+5A; pDMI,1), DSM-Nr.: 3517], am 06.08.86 [E. coli M15 (pDS8/RBSII,SphI; pDMI,1), DSM-Nr.: 3809], am 03.09.1987 [E. coli M15 (pDS78/RBSII; pDMI,1), DSM-Nr.: 4232], bzw. am 23.12.87 [E. coli M15 (pDS56/RBsII; pDMI,1), DSM-Nr.: 4330] nach dem Budapester Vertrag hinterlegt.

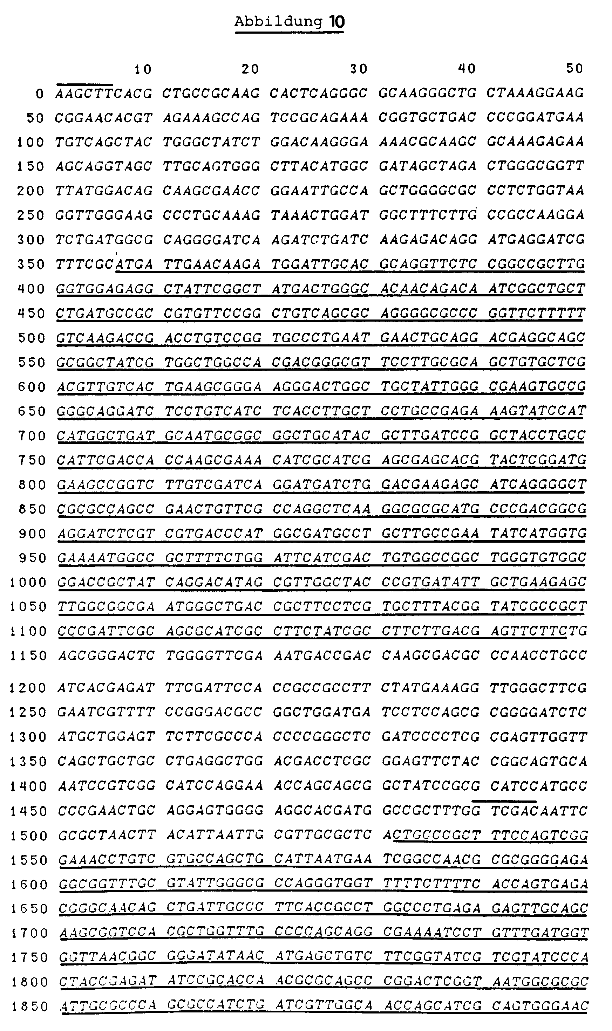

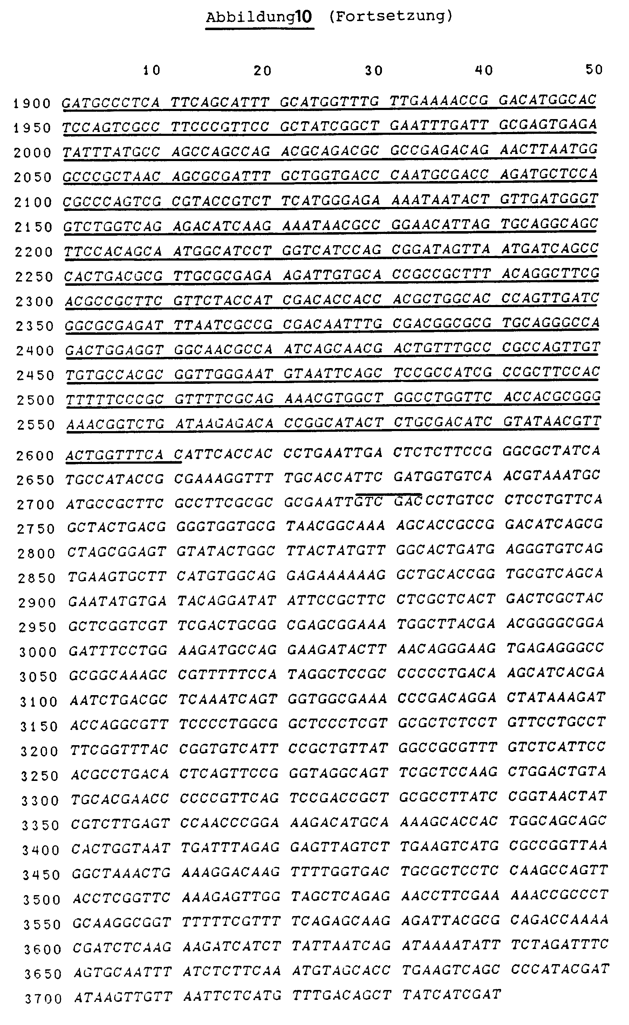

Die oben erwähnten Vektoren enthalten das regulierbare Promotor/Operator-Element P_{N25}x_{/O} (Stüber et al., EMBO J. 3, 3143-3148 [1984] oder N25OPSN25OP29 und die ribosomalen Bindungsstellen RBSII,SphI, RBSII,3A+5A oder RBSII. Diese ribosomalen Bindungsstellen wurden von der ribosomalen Bindungsstelle des Promotors P_{G25} des E. coli-Phagen T5 (R. Gentz, Dissertation, Universität Heidelberg, BRD [1984]) abgeleitet und über DNA-Synthese erhalten. Auf Grund der hohen Effizienz dieser Expressionssignale können die oben erwähnten Plasmide nur dann stabil in E. coli-Zellen gehalten werden, wenn das Promotor/Operator Element durch das Binden eines lac-Repressors an den Operator reprimiert wird. Der lac-Repressor ist im lacI-Gen kodiert. P_{N25}x_{/O} und N25OPSN25OP29 können nur dann effizient reprimiert werden, wenn eine ausreichende Zahl von Repressormolekülen in den Zellen vorhanden ist. Daher wurde das lacI^{q}-Allel benutzt, das eine Promotormutante enthält, die zu einer erhöhten Expression des Repressorgens führt. Dieses lacI^{q}-Allel ist im Plasmid pDMI,1 (Abb. 9 und 10) enthalten. Dieses Plasmid trägt zusätzlich zum lac-I-Gen das Neo-Gen, das den Bakterien Kanamycinresistenz verleiht, welche als Selektionsmarker benutzt wird. pDMI,1 ist mit den oben erwähnten Plasmiden kompatibel. E. coli-Zellen, die mit solchen Expressionsvektoren transformiert werden, müssen pDMI,1 enthalten, um sicherzustellen, dass der Expressionsvektor stabil in den Zellen gehalten wird. Eine Induktion dieses Systems wird durch Zugabe von IPTG ins Medium bei der gewünschten Zelldichte erreicht.

### B. Plasmid pDS8/RBSII,SphI

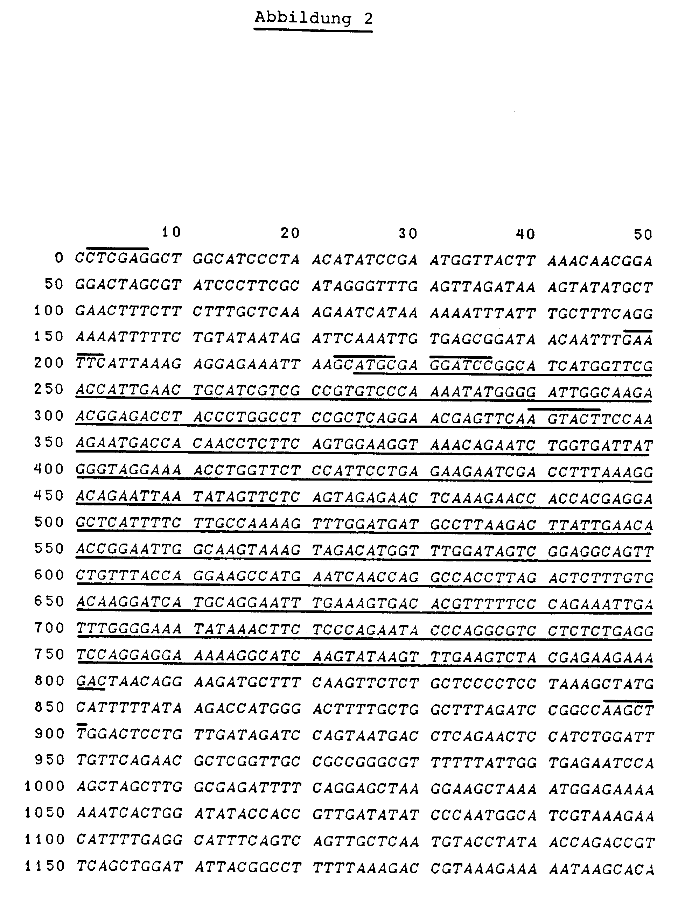

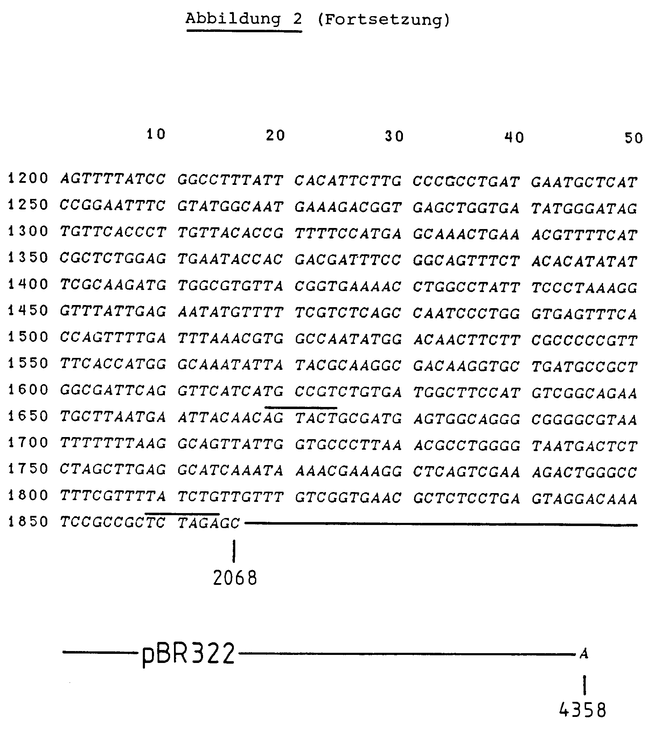

Der Anteil von pDS8/RBSII, SphI (Abb. 1 und 2), der zwischen den Restriktionsschnittstellen für XbaI und XhoI liegt und die Replikationsregion sowie das Gen für β-Lactamase, das den Zellen Ampicillinresistenz verleiht, enthält, stammt aus dem Plasmid pBR322 (Bolivar et al., Gene 2, 95-113 [1977]); Sutcliffe, Cold Spring Harbor Symp. Quant. Biol. 43, 77-90 [1979]). Der verbleibende Teil des Plasmids trägt das regulierbare Promotor/Operator-Element P_{N25}x_{/O} (Stüber et al., supra) Gefolgt von der Ribosomenbindungsstelle RBSII,SphI, die Teil eines EcoRI/BamHI-Fragments ist. Es folgt das Gen der Dihydrofolatreduktase (DHFR) der Maus-Zellinie AT-3000 (Chang et al., Nature 275, 617-624 [1978]; Masters et al., Gene 21, 59-63 [1983]), der Terminator tₒ des E. coli-Phagen Lambda (Schwarz et al., Nature 272, 410-414 [1978]), das Promotor-freie Gen der Chloramphenicolacetyltransferase (Marcoli et al., FEBS Letters, 110, 11-14 [1980]) und der Teminator T1 des E. coli rrnB Operons (Brosius et al., J. Mol. Biol., 148, 107-127 [1981]).

### C. Plasmid pDS5/RBSII,3A+5A

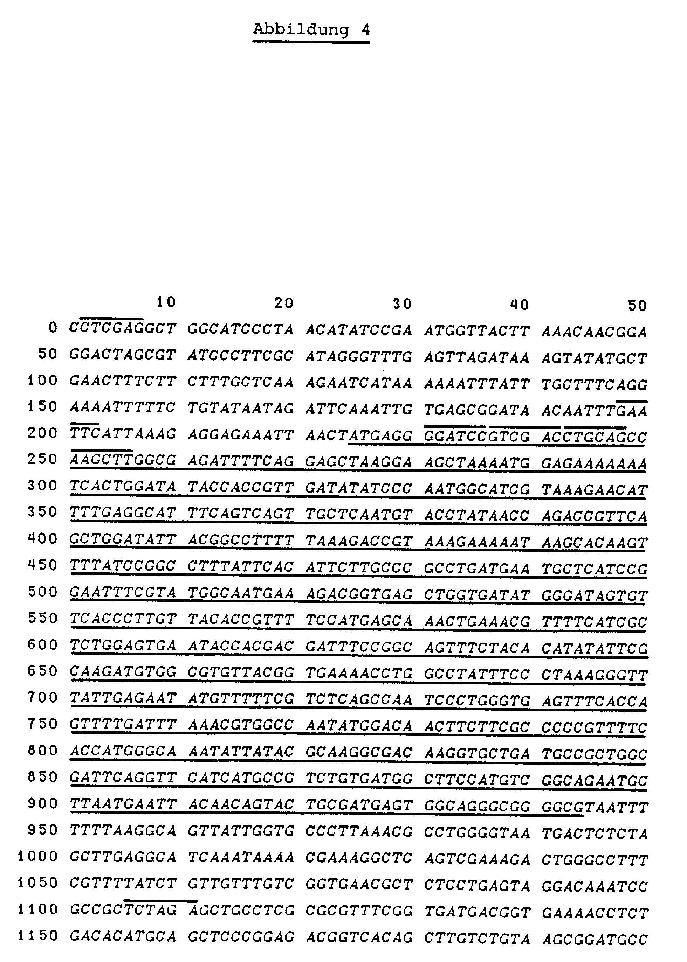

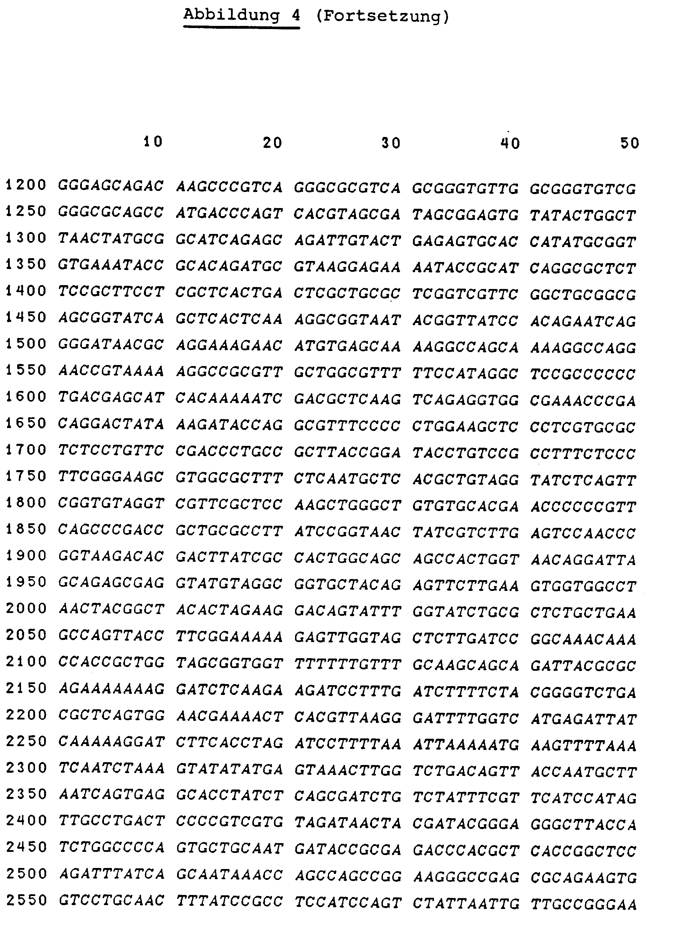

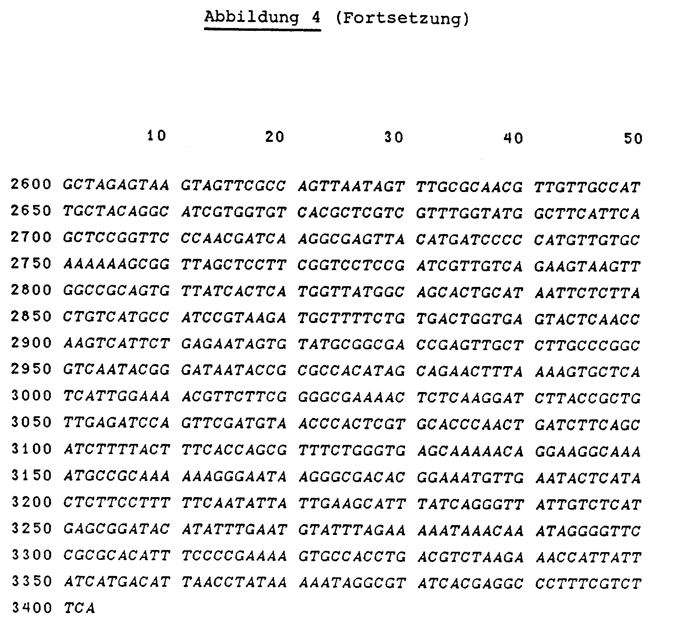

Der Anteil von pDS5/RBSII,3A+5A (Abb. 3 und 4), der zwischen den Schnittstellen für die Restriktionsenzyme XbaI und XhoI liegt und die Replikationsregion sowie das Gen für die β-Lactamase, das den Zellen Ampicillinresistenz verleiht, enthält, stammt ursprünglich aus dem Plasmid pBR322 (Bolivar et al., supra; Sutcliffe, supra). Jedoch ist das Gen für die β-Lactamase dahingegen modifiziert, dass die Schnittstellen für die Restriktionsenzyme HincII und PstI eliminiert sind. Diese Veränderungen in der DNA-Sequenz wirken sich aber nicht auf die Aminosäuresequenz der β-Lactamase aus. Der verbleibende Teil des Plasmids trägt das regulierbare Promotor/Operator-Element P_{N25}x_{/O} (Stüber et al., supra) gefolgt von der Ribosomenbindungsstelle RBSII,3A+5A, die Teil eines EcoRI/BamHI-Fragments ist. Es folgen Schnittstellen für die Restriktionsenzyme SalI, PstI und HindIII, das Promotor-freie Gen der Chloramphenicolacetyltransferase (Marcoli et al,, supra) und der Terminator T1 des E. coli rrnB Operons (Brosius et al., supra).

### D. Plasmid pDS78/RBSII

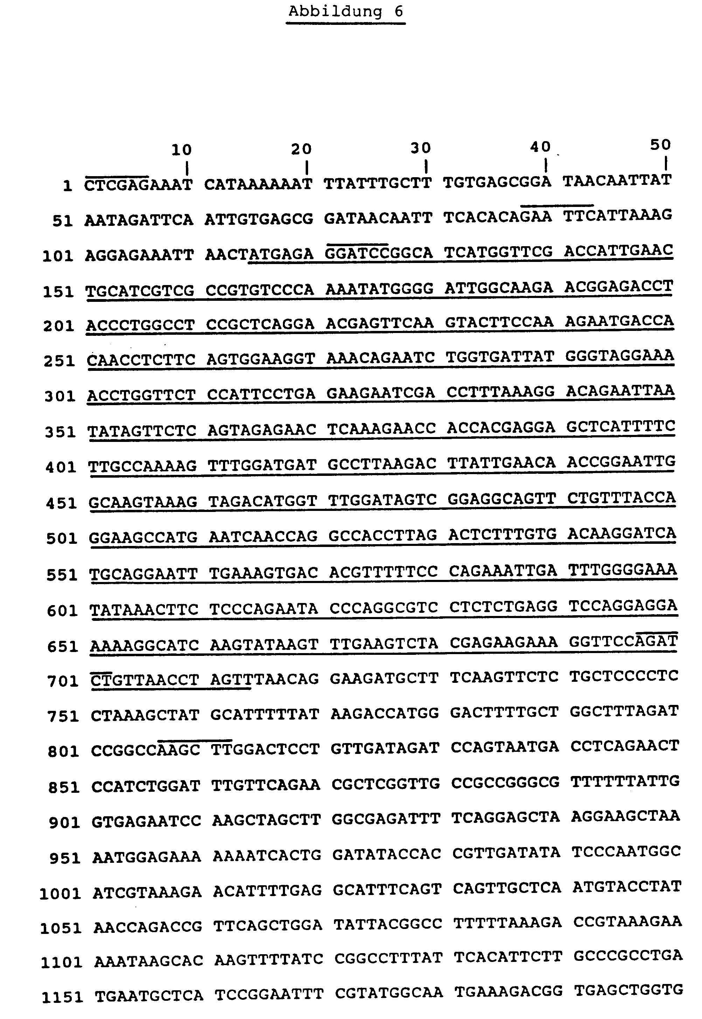

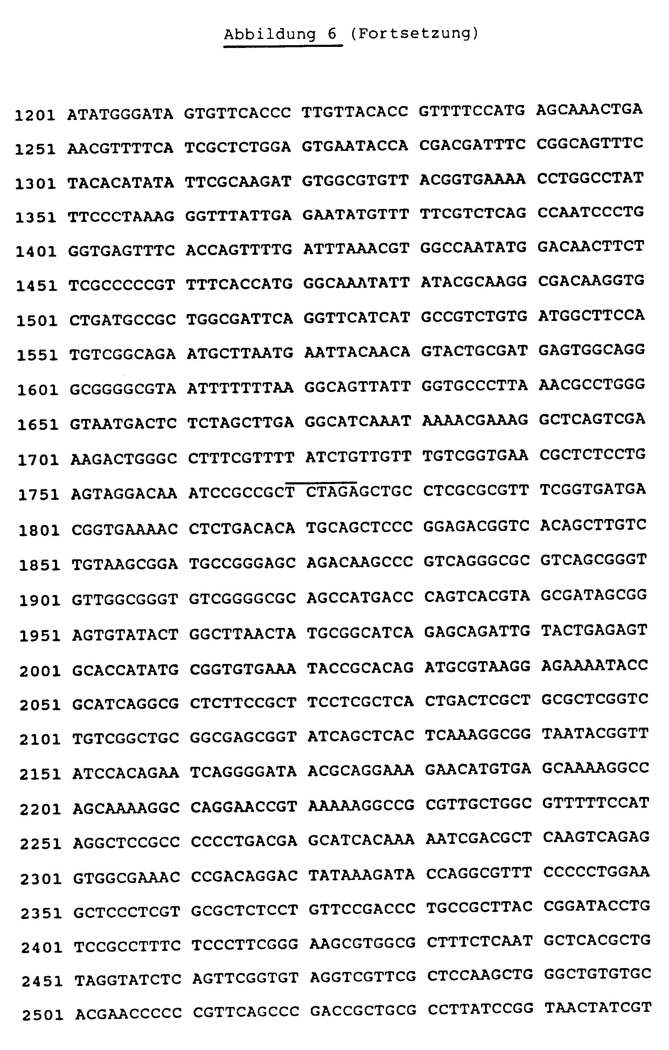

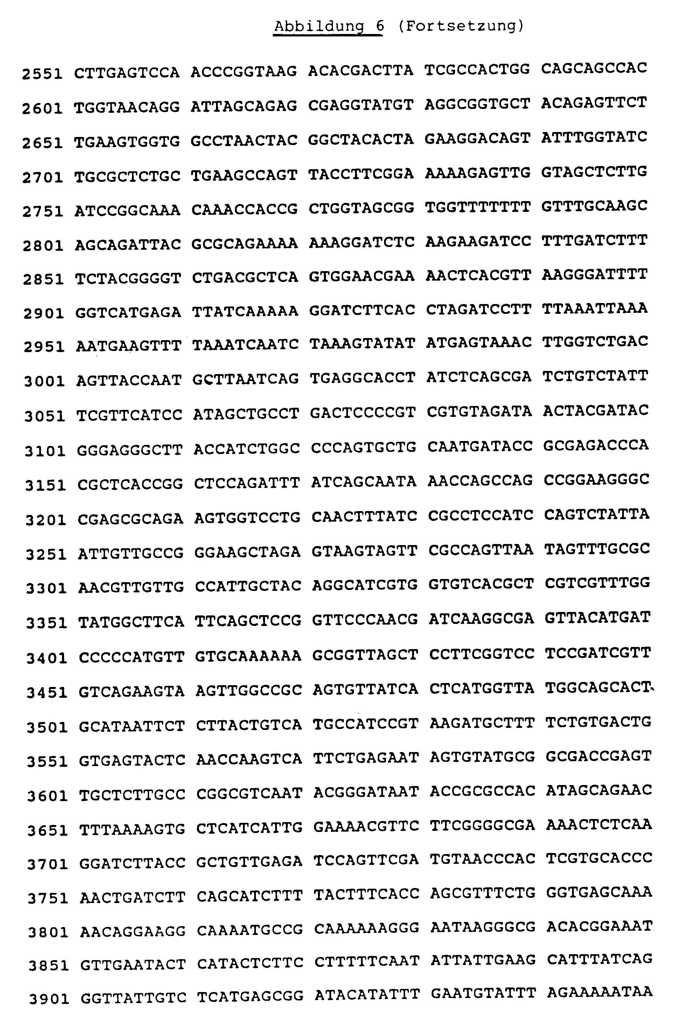

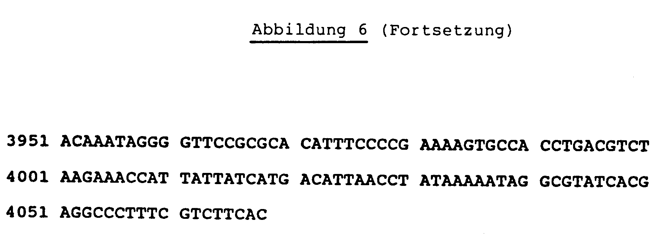

Der Anteil von pDS78/RBSII (Abb. 5 und 6), der zwischen den Restriktionsschnittstellen für XbaI und XhoI liegt und die Replikationsregion sowie das Gen für die β-Lactamase, das den Zellen Ampicillinresistenz verleiht, enthält, stammt ursprünglich aus dem Plasmid pBR322 (Bolivar et al., supra; Sutcliffe, supra). Jedoch ist das Gen für β-Lactamase in der für das Plasmid pDS5/RBSII,3A+5A beschriebenen Weise modifiziert. Der verbleibende Teil des Plasmids trägt das regulierbare Promotor/Operator-Element N25OPSN25OP29 gefolgt von der Ribosomenbindungsstelle RBSII, die Teil eines EcoRI/BamHI-Fragments ist. Es folgen das Gen der Dihydrofolatreduktase der Maus-Zellinie AT-3000 (Chang et al., supra; Masters et al., supra), welches so verändert wurde, dass eine Schnittstelle für das Restriktionsenzym BglII unmittelbar vor dem Ende des Strukturgens eingeführt wurde, der Terminator tₒ (Schwarz et al., supra), das Promotor-freie Gen der Chloramphenicolacetyltransferase (Marcoli et al., supra) und der Terminator T1 (Brosius et al., supra).

### E. Plasmid pDS56/RBSII

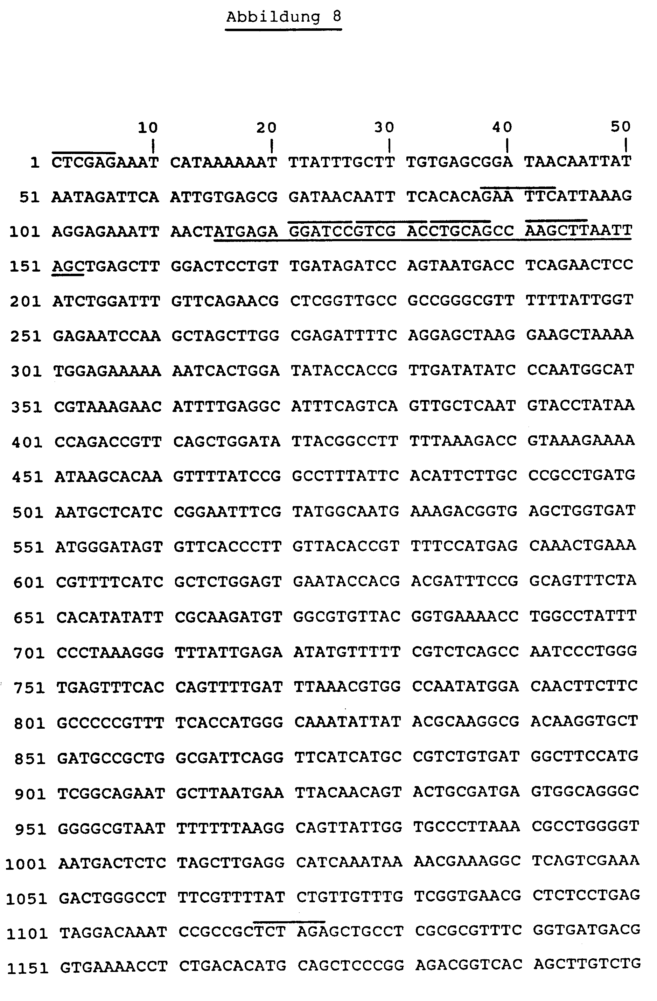

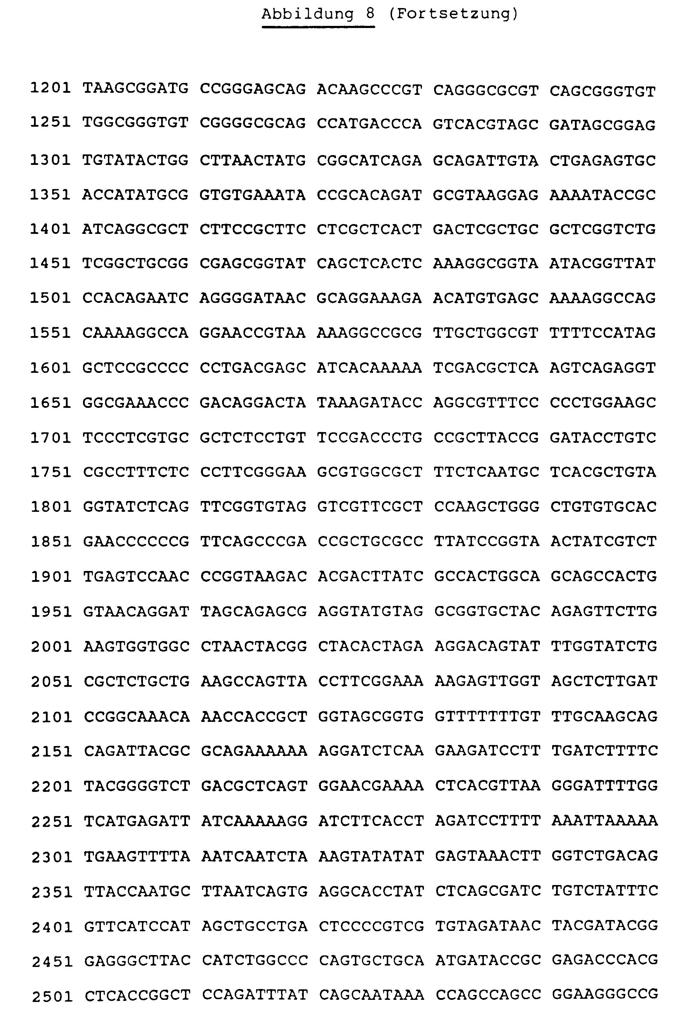

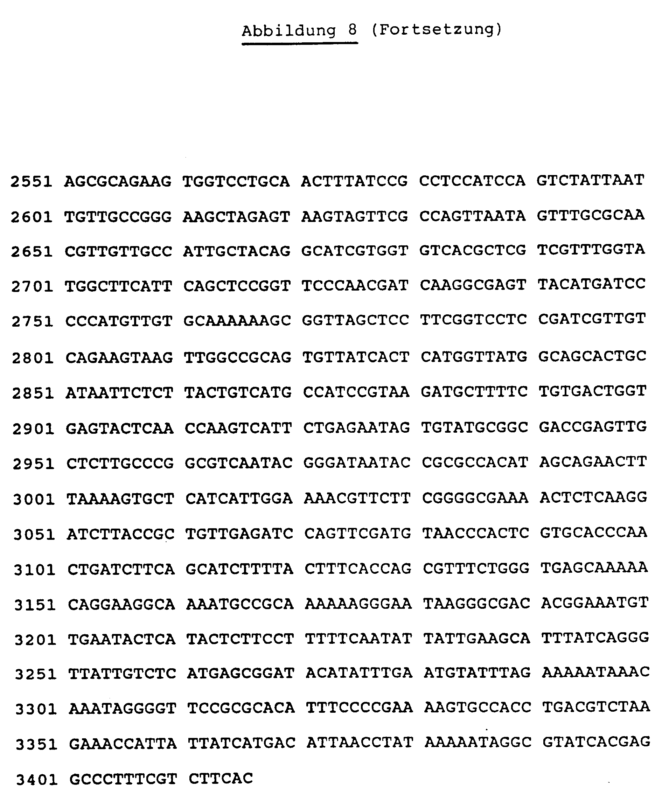

Das Plasmid pDS56/RBSII (Abb. 7 und 8) ist dem Plasmid pDS5/RBSII,3A+5A sehr ähnlich. Im Gegensatz zu diesem enthält das Plasmid pDS56/RBSII jedoch als Expressionssignale das regulierbare Promotor/Operator-Element N25OPSN25OP29 und die Ribosomenbindungsstelle RBSII. Zusätzlich enthält pDS56/RBSII den Terminator tₒ des E. coli-Phagen Lambda (Schwarz et al., supra).

### F. Plasmid pDMI,1

Das Plasmid pDMI,1 (Abb. 9 und 10) trägt das Gen der Neomycinphosphotransferase aus dem Transposon Tn5 (Beck et al., Gene 19, 327-336 [1982]), das E. coli-Zellen Kanamycinresistenz verleiht und das lac I-Gen (Farabough, Nature 274, 765-769 [1978]) mit der Promotormutation I^{q} (Calos, Nature 274, 762-765 [1978]), das den lac-Repressor kodiert. Ausserdem enthält das Plamid pDMI,1 eine Region des Plasmids pACYC184 (Chang und Cohen, J. Bacteriol. 134, 1141-1156 [1978]), die alle Informationen enthält, die für die Replikation und stabile Weitergabe an die Tochterzellen notwendig sind.

### Beispiel 2

### Beschreibung der DNA-Adaptoren, welche bei der Konstruktion der verschiedenen Plasmide verwendet wurden

### A. Prinzipien

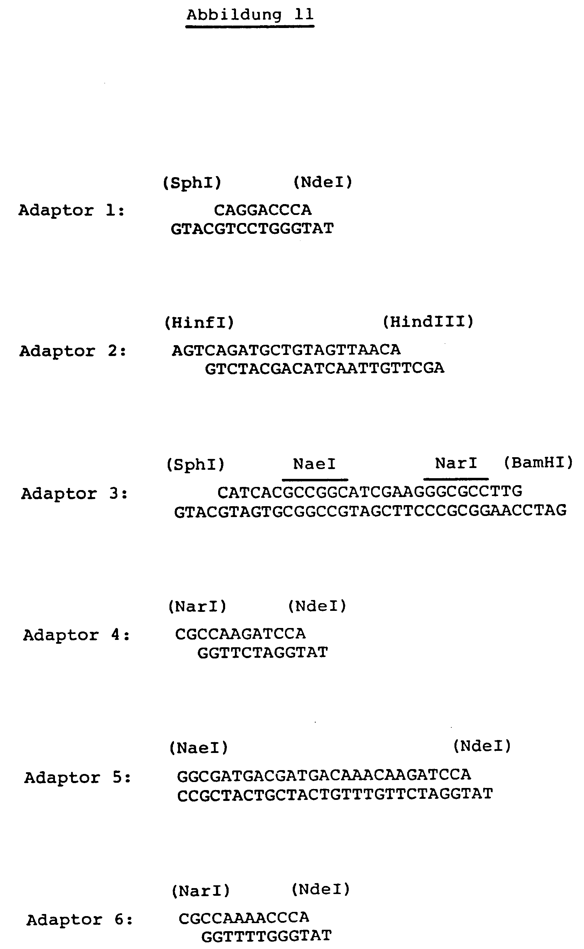

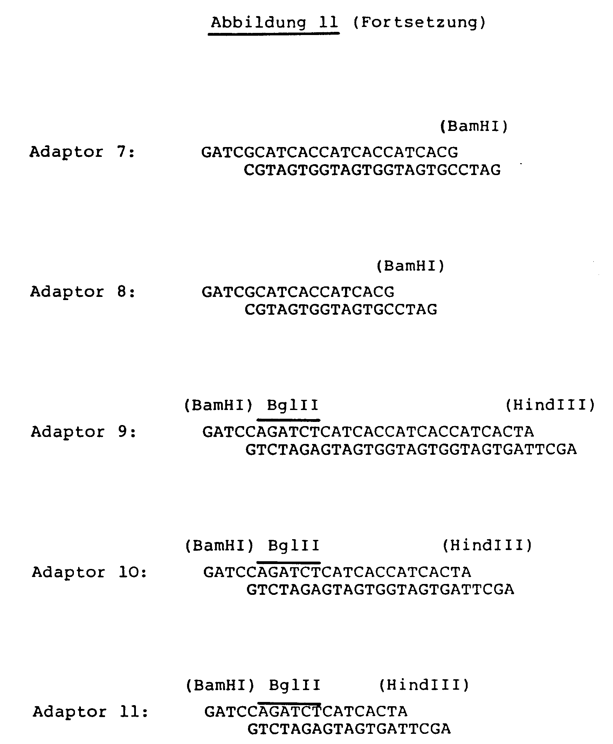

Zur Adaptierung der ribosomalen Bindungsstelle RBSII,SphI auf das Gen für Immuninterferon (IFN-γ), zur Verkürzung dieses Gens, zur Verknüpfung von IFN-γ und IFN-γ Bruchstücken, wie z.B. IFN-γ (-8), mit einem Affinitätspeptid sowie zur Expression von DHFR-Fusionsproteinen mit mindestens zwei benachbarten Histidinresten wurden Oligonukleotide chemisch synthetisiert und nach ihrer Aufarbeitung phosphoryliert. Die Nukleotidsequenzen der verwendeten Adaptoren sind als doppelsträngige DNA-Sequenzen in Abbildung 11 dargestellt.

### B. Synthese und Aufarbeitung der Oligonukleotide

Die Oligonukleotide wurden simultan auf einem Multisyntheseapparat (beschrieben in der Europäischen Patentanmeldung Nr. 181, veröffentlicht am 21.05.85) hergestellt, wobei Glas definierter Porengrösse (CPG) als Trägermaterial verwendet wurde (Kiefer et al., Immunol. Meth. 3, 69-83 [1985]; Sproat et al., Tetrahedr. Lett. 24,5771-5774 [1983]; Adams et al., J. Amer. Chem. Soc., 105, 661-663 [1985]. Die lyophilisierten Oligonukleotide wurden in Wasser aufgenommen und 1 Stunde bei 4°C gelöst. Die DNA-Konzentration betrug 100 nmol/ml.

### C. Phosphorylierung der Oligonukleotide

In getrennten Ansätzen wurden jeweils 150 pmol der Oligonukleotide in 20 »l 50 mM Tris-HCl, pH 8,5 und 10 mM MgCl₂ mit 2 pmol γ-[³²P]-ATP (Amersham, Braunschweig; 5000 Ci/mMol) und 1 Einheit (E) T4-Polynukleotidkinase (Gibco-BRL, Basel) während 20 Minuten bei 37°C inkubiert. Anschliessend wurde 5 nmol ATP zugegeben und nach weiteren 20 Minuten bei 37°C wurden die Reaktionen durch Erhitzen auf 65°C beendet. Die so erhaltenen phosphorylierten Oligonukleotide wurden ohne weitere Aufarbeitung verwendet.

### Beispiel 3

### Konstruktion des Plasmids pGLS

### A. Prinzipien

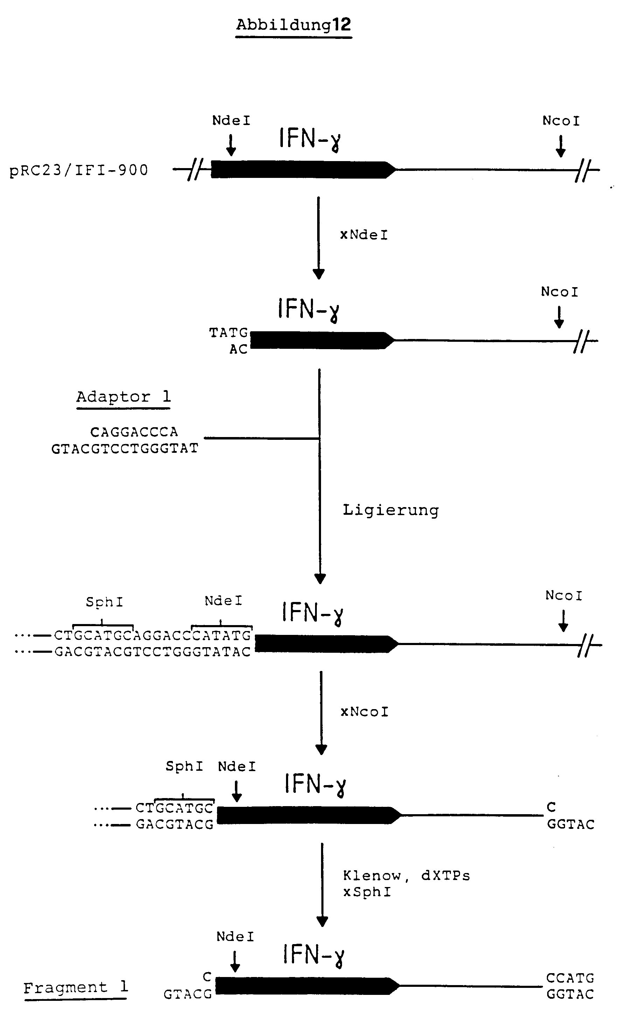

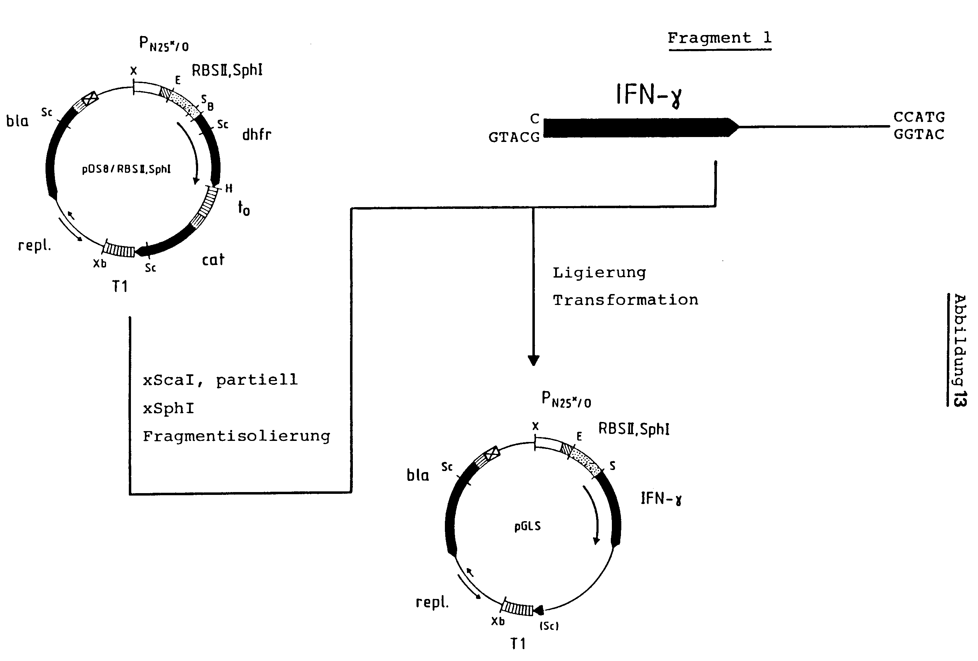

Zur Konstruktion des Plasmids pGLS wurde zunächst das IFN-γ Gen mit dem Adaptor 1 (Abb.11) verbunden (Abb.12) und isoliert. Anschliessend wurde das resultierende Fragment 1 in das Plasmid pDS8/RBSII,SphI integriert (Abb.13).

### B. Präparation des Fragments 1

4 »g des Plasmids pRC23/IFI-900 (Europ. Patentanmeldung Publ.-Nr. 99084, veröffentlicht am 25.1.84) mit einer DNA-Konzentration von 400 »g/ml wurden mit 10 Einheiten der Restriktionsendonuklease NdeI in core buffer (50mM Tris-HCl, pH 8, 10mM MgCl₂, 50mM NaCl), für 1 Stunde bei 37°C (Volumen 20 »l) verdaut. Anschliessend wurde die Probe einmal mit Phenol extrahiert, Reste von Phenol mit Aether entfernt und die DNA schliesslich mit 66% Alkohol und 0,3M K-Acetat gefällt. Das Sediment wurde 2 Minuten im Speed-vac-concentrator getrocknet und in T4-Ligasepuffer (50mM Tris-HCl, pH 7,8, 10mM MgCl₂, 10mM DTT, 500 »M ATP) gelöst. 25 pmol des phosphorylierten Adaptors 1 (Abb.11) wurden in 1x Ligasepuffer gelöst und dieser Reaktionsmischung zugegeben, so dass ein Gesamtvolumen von 25 »l erreicht wurde. Die Ligierung wurde für 3 Stunden bei 22°C durchgeführt, wobei 1 »l DNA-Ligase (1 Weiss-Einheit, Boehringer Mannheim) eingesetzt wurde. Die Ligierung wurde durch Erhitzen der Probe für 7 Minuten auf 65°C beendet. Die DNA wurde mit Alkohol gefällt, wie oben beschrieben getrocknet und dann in 50 »l NcoI-Verdauungspuffer (50mM Tris-HCl, pH 8, 10mM MgCl₂, 50mM NaCl, 50mM KCl) gelöst. 10 Einheiten NcoI wurden hinzugegeben und die Probe für 1 Stunde bei 37°C inkubiert. Das Enzym wurde anschliessend durch Erhitzen der Probe für 7 Minuten auf 65°C inaktiviert. Nach einer Phenolextraktion wurde die DNA wie beschrieben ausgefällt und das Sediment getrocknet. Die DNA wurde in Klenow-Puffer (50mM Tris-HCl, pH 7,2, 10mM MgSO₄, 100 »M DTT) gelöst, dATP, dGTP, dCTP und dTTP (Endkonzentration jeweils 100 »M) und 1 Einheit Klenow-Enzym (Boehringer, Mannheim) hinzugegeben und die Probe für 1 Stunde bei 22°C gehalten. Die Reaktion wurde durch Zugabe von 2 »l 0,25M EDTA beendet, die Probe mit Phenol extrahiert, die DNA wie beschrieben mit Alkohol ausgefällt und in SphI-Verdauungspuffer (50mM Tris-HCl, pH 7,5, 6mM MgCl₂, 50mM NaCl, 6mM 2-Mercaptoäthanol) gelöst. Nach Zugabe von 10 Einheiten SphI wurde die Probe für 1 Stunde bei 37°C inkubiert, die Verdauung wie beschrieben durch Erhitzen beendet, eine Phenolextraktion durchgeführt und die DNA mit Alkohol ausgefällt und getrocknet. Das DNA-Sediment wurde in 10 »l Probenpuffer gelöst und die DNA-Fragmente in einem 6%igen Polyacrylamidgel aufgetrennt (Laufpuffer; 40mM Tris-HCl, 20mM Na-acetat, 2mM EDTA, pH 7,8). Als Molekulargewichtsstandard diente mit HaeIII verdaute DNA des Phagen ØX (Gibco-BRL, Basel). Die DNA wurde mit Ethidiumbromid (0,5 »g/ml) angefärbt, mit UV-Licht (300 nm Wellenlänge) sichtbar gemacht und die IFN-γ kodierende Bande mit einem Skalpell aus dem Gel ausgeschnitten. Das Gelstück wurde in eine 4x4 mm grosse Tasche in einem Agarosegel überführt (0,7% Agarosegel, Laufpuffer: 90mM Tris-Borat, 90mM Borsäure, 3mM EDTA, pH 8,3). Die Tasche wurde mit 0,7% flüssiger Agarose in 1xTBE verschlossen, um ein homogenes elektrisches Feld zu erreichen. Vor die Probe wurde ein Stück einer NA45 Membran (Schleicher und Schüll, Dassel, BRD) plaziert und die DNA auf die Membran elektrophoretisiert (5 Minuten, 15 V/cm). Nach der Waschung mit destilliertem Wasser wurde die Membran in ein Eppendorfröhrchen überführt, das 250 »l 1,5M Lithiumacetat, 50mM Tris-HCl, pH 8 und 10mM EDTA enthielt. Die DNA wurde nun für 20 Minuten bei 65°C eluiert. Der Membranstreifen wurde aus dem Röhrchen entfernt und die Probe einmal mit 200 »l Phenol (pH 8) extrahiert. Die DNA wurde nach Zugabe von 20 »l 5M Lithiumacetat und 440 »l Isopropanol gefällt und das Sediment mit 80% Aethanol gewaschen und anschliessend getrocknet. Abschliessend wurde das Sediment in 10 »l TE-Puffer gelöst (10mM Tris-HCl, pH 7,6, 1mM EDTA). Das so erhaltene DNA-Fragment erhielt die Bezeichnung Fragment 1 (Abb.12).

### C. Präparation des Plasmids pDS8/RBSII,SphI

2 pmol des Plasmids pDS8/RBSII,SphI wurden zunächst mit dem Restriktionsenzym SphI geschnitten. Danach wurde die resultierende lineare Plasmid DNA mit einer limitierenden Menge des Restriktionsenzyms ScaI inkubiert, wodurch die DNA nur an ungefähr 50% der vorhandenen ScaI Schnittstellen geschnitten wurde. Nun wurde die Probe mit Phenol extrahiert, ausgeäthert und die DNA wie beschrieben ausgefällt. Das Sediment wurde getrocknet, in 20 »l Puffer (50mM Tris-HCl, pH 8) gelöst und 1E CIP (calf intestinal phosphatase, Boehringer Mannheim) zugegeben. Die Probe wurde für 1 Stunde bei 37°C inkubiert, das Enzym durch eine Phenolextraktion entfernt und die DNA ausgefällt. Nachdem die DNA gelöst worden war, wurde das ScaI/SphI-Fragment, das Teile des cat-Gens, den Terminator T1, die Replikationsregion, das bla-Gen, den Promotor N25x/o und die ribosomale Bindungsstelle RBSII,SphI enthielt aus einem 1%igen Agarosegel isoliert und elektrophoretisch, wie oben beschrieben, auf eine NA45-Membran transferiert. Die Elution der DNA, Alkoholfällung und Auflösen in 10 »l TE-Puffer wurde ebenfalls, wie oben beschrieben, durchgeführt. Ungefähr 1 pmol des gewünschten Vektorfragments wurden erhalten.

### D. Zusammensetzen des Plasmids pGLS

0,05 pmol des Vektorfragments wurden mit 0,05 pmol Insert-DNA (Fragment 1) in Ligasepuffer ligiert (s.o.). Eine Kontrolligierung ohne Insert-DNA wurde parallel dazu durchgeführt. E. coli M15 Zellen enthaltend Plasmid pDMI,1 wurden nach der Methode von Morrison (Methods Enzymol. 68, 326-331 [1979]) für die Transformation vorbereitet. Die Ligierungs- mischungen wurden nach Erhitzen für 7 Minuten bei 65°C zu 200 »l dieser kompetenten Zellen hinzugefügt. Die Proben wurden für 30 Minuten auf Eis gehalten, dann für 2 Minuten bei 42°C inkubiert, und nach der Zugabe von 0,5 ml LB-Medium während 90 Minuten bei 37°C inkubiert. Die Zellen wurden dann auf LB-Agarplatten, die 100 »g/ml Ampicillin und 25 »g/ml Kanamycin enthielten, ausplattiert und über Nacht im Brutschrank bei 37°C inkubiert. Die Transformation der Kontrolligierung ergab keine Transformanten. Die Ligierung mit Fragment 1 dagegen etwa 200 Kolonien. Einzelne Kolonien wurden mit einem sterilen Zahnstocher gepickt, in ein Röhrchen überführt, das 10 ml LB-Medium mit 100 »g/ml Ampicillin und 25 »g/ml Kanamycin enthielt, und 12 Stunden im Schüttelinkubator gehalten. Danach wurden die Zellen sedimentiert und die Plasmid-DNA nach der Methode von Birnboim und Doly (Nucleic Acids Res. 7, 1515-1523 [1979]) isoliert.

Je 1 »g der isolierten Plasmide wurde mit SphI und XbaI verdaut, um nachzuprüfen, ob ein Fragment, welches das IFN-γ Gen und den Terminator T1 enthält, in diesen Plasmiden enthalten ist. Alle analysierten DNA-Proben enthielten das erwartete DNA-Fragment von ca. 1 kb. Diese Plasmide erhielten die Bezeichnung pGLS (Abb.13).

### E. Sequenzanalyse des in pGLS integrierten IFN-γ Gens

Um nachzuweisen, dass die richtige IFN-γ Sequenz in dem Plasmid pGLS enthalten ist, wurde die doppelsträngige zirkuläre Plasmid-DNA sequenziert, wobei ein mit [γ-³²P]-ATP markierte Startersequenz (primer) benutzt wurde. Diese Startersequenz enthält die Nukleotide von Position 199-218 des Plasmids pDS8/RBSII,SphI und endet 6 Nukleotide vor dem ATG der SphI-Schnittstelle.

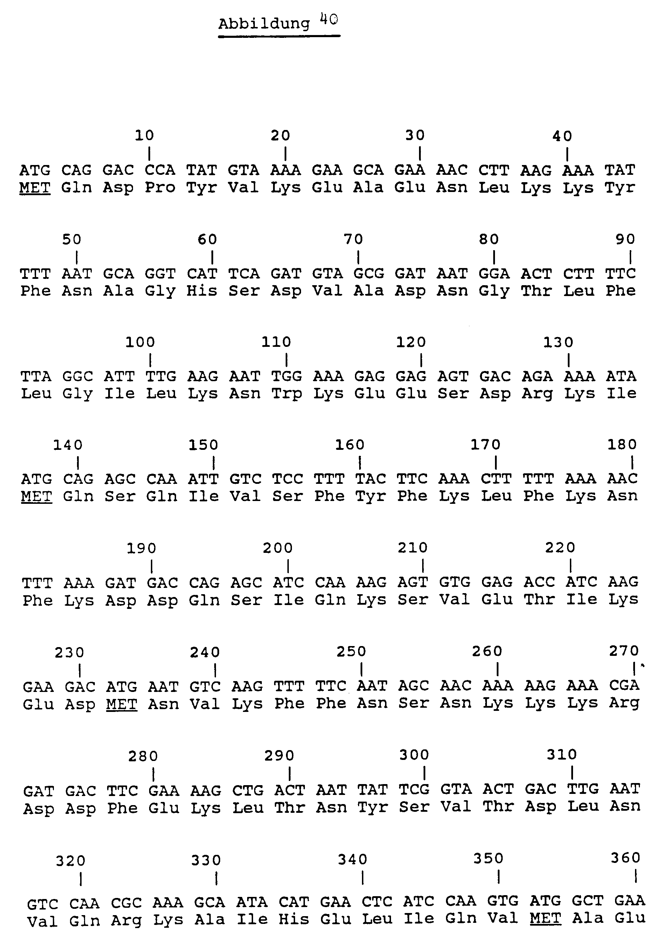

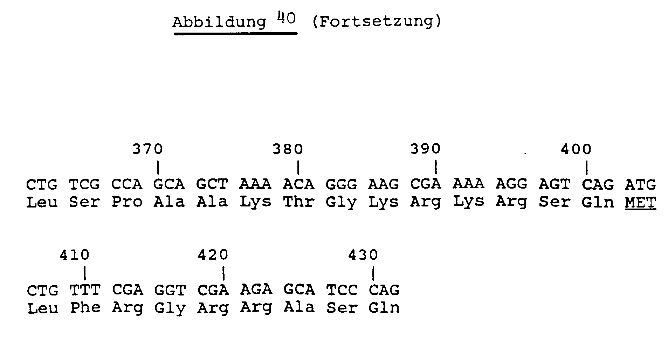

0,3 pmol der isolierten Plasmid-DNA wurden mit Alkohol gefällt, das Sediment einmal mit 80% Aethanol gewaschen, getrocknet und schliesslich in 8 »l 1/4 TE-Puffer gelöst. Nach Zugabe von 2 pmol der Startersequenz wurde die Probe 5 Minuten bei 42°C inkubiert. Dann wurde die DNA nach der Methode von Sanger et al. (Proc. Natl. Acad. Sci. USA 74, 5463-6567 [1977]) sequenziert. Da ein radioaktiver markierter "Primer" verwendet wurde, wurden alle Reaktionen mit unmarkierten Deoxynukleotidtriphosphaten durchgeführt. Die DNA-Sequenzanalyse ergab, dass die korrekte IFN-γ-Sequenz in das Plasmid pGLS integriert worden war (Aminosäuresequenz siehe Abb.40).

### Beispiel 4

### Konstruktion des Plasmids pIFN-γ(-8)

### A. Prinzipien

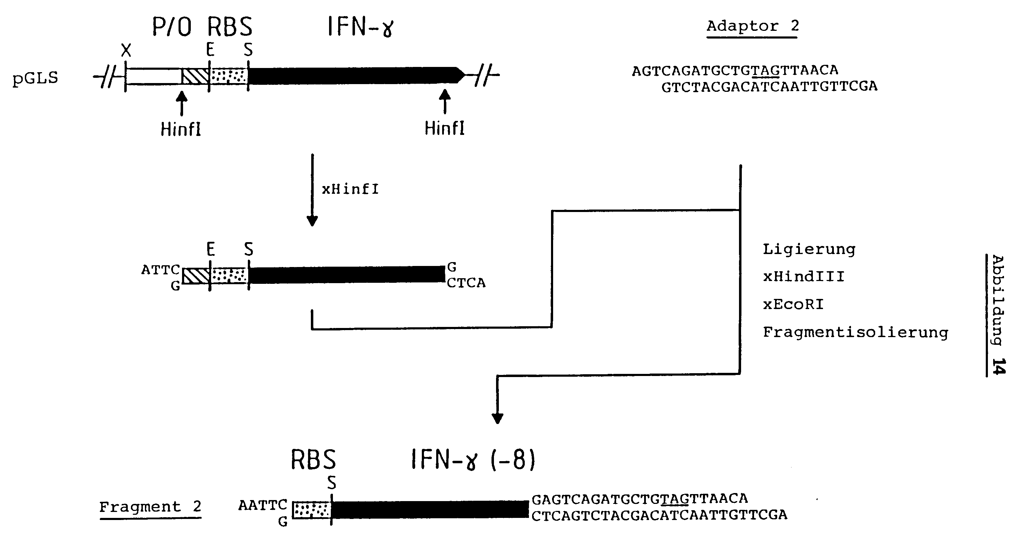

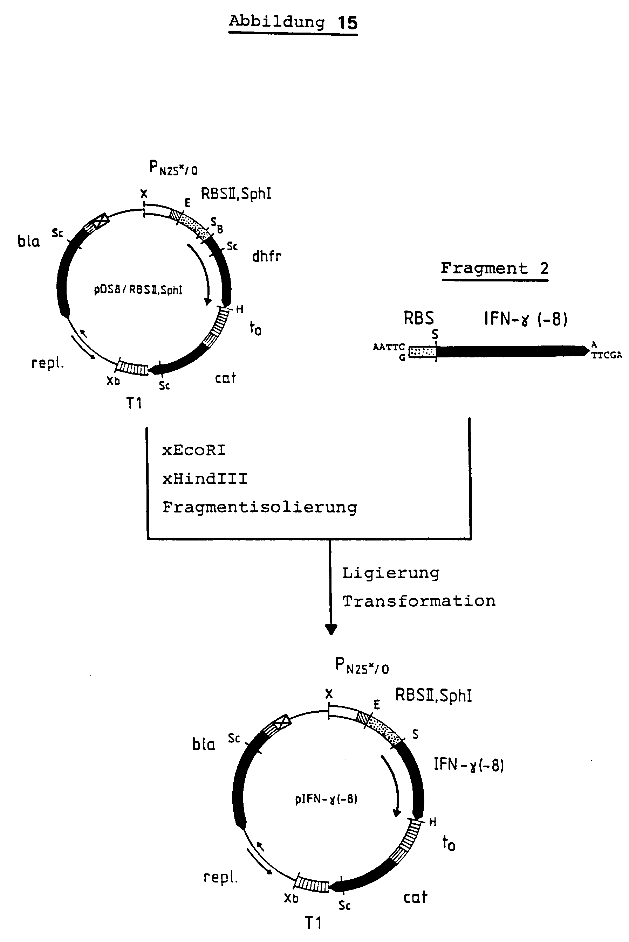

Zur Konstruktion des Plasmids pIFN-γ(-8) wurde zunächst der Adaptor 2 (Abb.11) mit der einzigen HinfI-Schnittstelle im IFN-γ Gen verbunden. Aufgrund eines Translationsstopcodons in diesem Adaptor wird somit die C-terminale Region des IFN-γ Proteins um 8 Aminosäuren verkürzt (Abb.14). Anschliessend wurde das resultierende Fragment 2 in das Plasmid pDS8/RBSII,SphI integriert (Abb.15).

### B. Präparation des Fragments 2

3 pmol des Plasmids pGLS wurden mit 15E HinfI verdaut (50 »l Volumen, 1 h, 37°C). Anschliessend wurde das Restriktionsenzym inaktiviert (7 Min. bei 65°C), die Probe mit Phenol extrahiert, ausgeäthert, mit K-acetat und Alkohol gefällt und getrocknet. Das Sediment wurde in 50 »l Ligasepuffer gelöst. 100 pmol phosphorylierte Oligonukleotide (Adaptor 2) wurden mit 10 »l HinfI geschnittenem Plasmid pGLS vermischt und nach Zugabe von 25 »l Ligasepuffer und 1E T4-DNA-Ligase während 12 Stunden bei 22°C inkubiert. Die Reaktion wurde, wie oben beschrieben, durch Erhitzen der Probe beendet und die DNA gefällt. Nun folgte eine Verdauung mit den Restriktionsenzymen EcoRI (15E) und HindIII (20E) für 2 Stunden bei 37°C. Nach Hitzeinaktivierung, Phenolextraktion, ausäthern und Alkoholfällung wurde die Probe in 10 »l Probenpuffer gelöst und die DNA-Fragmente in einem 6%igen Polyacrylamidgel aufgetrennt. Die DNA-Banden wurden unter UV-Licht (300 nm), nach Anfärbung mit Ethidiumbromid, sichtbar gemacht. Die Bande, die das IFN-γ Gen enthielt wurde aus dem Gel mit einem sterilen Skalpell ausgeschnitten und, wie oben beschrieben, auf eine NA45-Membran elektrophoretisiert. Die DNA wurde wie beschrieben, eluiert und erhielt die Bezeichnung Fragment 2 (Abb.14).

### C. Präparation des Plasmids pDS8/RBSII,SphI

2 pmol des Plasmids pDS8/RBSII,SphI wurden mit 10E EcoRI und 10E HindIII für 1 Stunde bei 37°C in einem Volumen von 50 »l verdaut. Nach Hitzeinaktivierung der Enzyme und einer Alkoholfällung wurde das DNA-Sediment in 10 »l Probenpuffer gelöst. Nach Elektrophorese in einem 1%igen Agarosegel wurde das EcoRI/HindIII-Fragment, das den Terminator tₒ, das cat-Gen, den Terminator T1, die Replikationsregion, das bla-Gen und den Promotor P_{N25}x/_{O} enthält aus dem Gel ausgeschnitten und, wie oben beschrieben, eluiert.

### D. Zusammensetzen des Plasmids pIFN-γ(-8)

10 »l des isolierten EcoRI/HindIII-Vektorfragments und die Hälfte des isolierten Fragments 2 wurden mit 1E T4-Ligase inkubiert (22°C, 3 Stunden). Eine Kontrolligierung ohne Hinzufügen des Fragments 2 wurde parallel dazu durchgeführt. Die Ligierungen wurden, wie beschrieben, durch Erhitzen der Probe beendet.

Die Transformationen wurden nach der Methode von Morrison (supra) durchgeführt, wobei der E. coli-Stamm M15, der das Plasmid pDMI.1 enthielt, verwendet wurde. Die Zellen wurden auf LB-Agarplatten, die 100 »g/ml Ampicillin und 25 »g/ml Kanamycin enthielten, ausplattiert. Die Platten wurden für 15 Stunden bei 37°C im Brutschrank gehalten.

Auf den Kontrollplatten (=Kontrolligierung) wurden keine Transformanten gefunden. Die Ligierung, bei der Vektor-DNA und Fragment 2 eingesetzt wurden, ergab etwa 500 Kolonien. Einzelne Kolonien wurden mit einem sterilen Zahnstocher gepickt, in 10 ml LB-Medium überführt und, wie beschrieben, wachsen gelassen. Die Plasmid-DNA wurde nach der Methode von Birnboim and Doly (supra) isoliert. Je 4 »l der isolierten und in TE-Puffer gelöstem Plasmid-DNA wurden mit je 2E EcoRI und HindIII, wie beschrieben, geschnitten. Aus allen getesteten Plasmiden konnte ein Fragment mit der erwarteten Länge von ca. 450 bp ausgeschnitten werden. Diese Plasmide erhielten die Bezeichnung pIFN-γ(-8) (Abb.15).

### E. Sequenzanalyse des Plasmids pIFNγ(-8)

Die Sequenzanalyse wurde, wie in Beispiel 3 beschrieben, durchgeführt. Als Startersequenz wurde jedoch ein Oligonukleotid verwendet, das die Nukleotide von Position 928-896 des Plasmids pDS8/RBSII,SphI enthält und somit die Sequenzierung von DNA-Fragmenten erlaubt, die vor dem Terminator tₒ integriert wurden. Die Sequenzanalyse bestätigte die gewünschte Sequenz des IFN-γ Gens, welches für ein um 8 Aminosäuren (am Carboxylende) verkürztes IFN-γ Protein kodiert.

### Beispiel 5

### Konstruktion des Plasmids pDS8/RBSII,SphI-His,His-Xa-BamHI

### A. Prinzipien

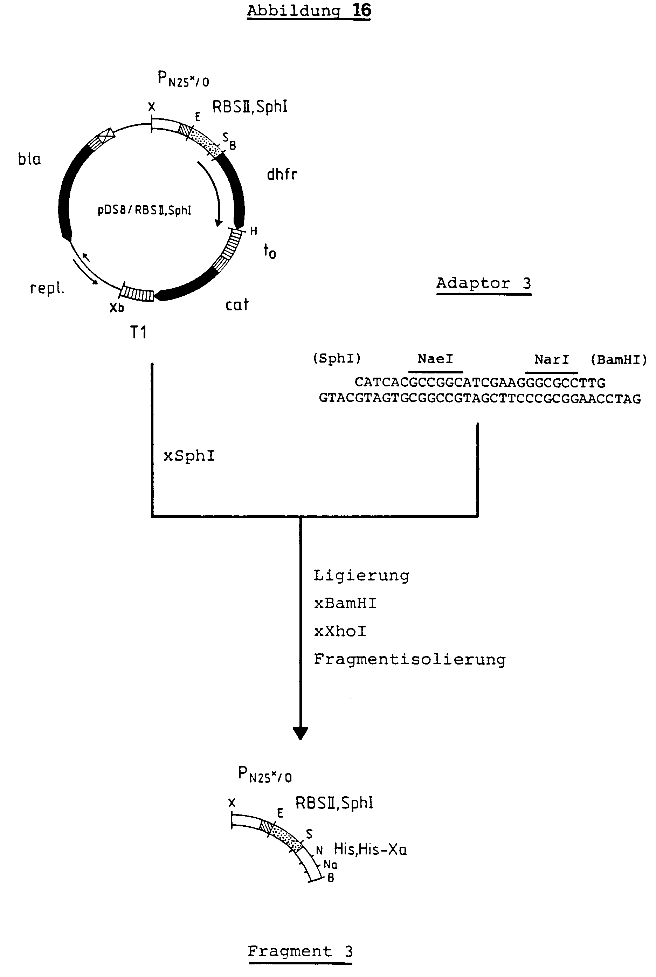

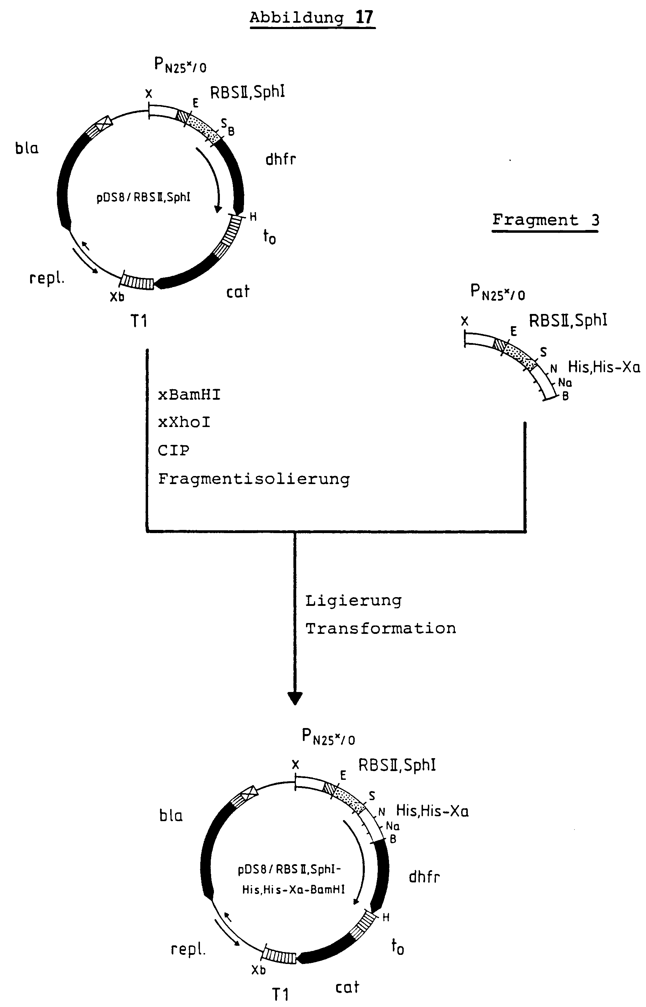

Zur Konstruktion des Plasmids pDS8/RBSII,SphI-His,His-Xa-BamHI wurde zunächst der Adaptor 3 (Abb.11), der für ein Affinitätspeptid kodiert, welches zwei benachbarte Histidine und eine Schnittstelle des Faktors Xa enthält, mit der ribosomalen Bindungsstelle RBSII,SphI verbunden. Anschliessend wurde das Fragment 3 (Abb.16), enthaltend den Promotor P_{N25}x_{/O} sowie genanntes Affinitätspeptid, isoliert und in das Plasmid pDS8/RBSII,SphI integriert (Abb.17).

### B. Präparation des Fragments 3

2 pmol des Plasmids pDS8/RBSII,SphI wurden mit dem Restriktionsenzym SphI geschnitten. Die Reaktion wurde durch Inkubation bei 65°C für 7 Minuten beendet, die Probe mit Phenol extrahiert, ausgeäthert und die DNA mit Alkohol und K-acetat ausgefällt. Das Sediment wurde in 10 »l Ligasepuffer aufgenommen, 25 pmol des phosphorylierten Adaptors 3 (Abb. 11), gelöst in Ligasepuffer, zugegeben und nach Zugabe von 1E T4-DNA-Ligase für 3 Stunden bei 22°C inkubiert. Die Reaktion wurde durch Erhitzen (7 Minuten, 65°C) beendet und die DNA, nach einer Phenolextraktion und anschliessender Behandlung mit Aether, mit Alkohol und K-acetat ausgefällt. Das Sediment wurde in 30 »l Puffer gelöst, je 10E der Restriktionsenzyme BamHI und XhoI wurden zugegeben und die Mischung bei 37°C für 2 Stunden inkubiert. Anschliessend wurden 3,5 »l 10-fach konzentrierter Probenpuffer für Polyacrylamidgele zugegeben und die Mischung für 7 Minuten bei 65°C inkubiert. Die DNA wurde in einem 6%igen Polyacrylamidgel aufgetrennt und das durch XhoI und BamHI freigesetzte Fragment mit einem Skalpell ausgeschnitten. Die DNA wurde, wie beschrieben, eluiert und erhielt die Bezeichnung Fragment 3 (Abb.16).

### C. Präparation des Plasmids pDS8/RBSII,SphI

2 pmol des Plasmids pDS8/RBSII,SphI wurden mit je 10E der Restriktionsenzyme BamHI und XhoI geschnitten. Nach Hitzeinaktivierung der Enzyme wurde die Probe mit Phenol extrahiert, ausgeäthert und die DNA mit Alkohol und K-acetat gefällt. Das Sediment wurde in 50 »l 50 mM Tris-HCl, pH 8, resuspendiert. 1E CIP (siehe oben) wurde zugegeben und die Probe bei 37°C für 30 Minuten inkubiert. Nach Hitzeinaktivierung des Enzyms wurde die DNA, nach Zugabe von Probenpuffer, in einem 6%igen Polyacrylamidgel aufgetrennt und der Plasmidrumpf, wie beschrieben, aus dem Gel eluiert.

### D. Zusammensetzen des Plasmids pDS8/RBSII,SphI-His,His-Xa-BamHI

Das oben beschriebene Fragment 3 wurde mit dem Vektorrumpf ligiert (22°C, 2E T4-DNA-Ligase, 25 »l Ligasepuffer). Eine Kontrolligierung ohne Zugabe von Fragment 3 wurde parallel dazu durchgeführt. Die Ligierungsansätze wurden, wie oben beschrieben, in den E. coli-Stamm M15, der das Plasmid pDMI,1 enthielt, transformiert und auf LB-Platten mit 100 »g/ml Ampicillin und 25 »g/ml Kanamycin ausplattiert. Die Transformation der Kontrolligierung ergab keine Transformanten. Die Transformation des Ligierungsansatzes mit Fragment 3 ergab etwa 100 Kolonien. Einzelne Kolonien wurden in 10 ml LB-Medium, wie oben beschrieben, aufgewachsen und die Plasmid-DNA nach der Methode von Birnboim und Doly (supra) isoliert. Alle Plasmide enthielten die durch den Adaptor neu eingeführten Schnittstellen für NaeI und NarI (siehe Abb.17). Die Sequenzanalyse der Plasmid-DNA wurde, wie oben beschrieben (Beispiel 3,F), durchgeführt und bestätigte, dass der Adaptor 3 korrekt in den Vektor integriert wurde. Diese Plasmide erhielten die Bezeichnung pDS8/RBSII,SphI-His,His-Xa-BamHI (Abb.17).

### Beispiel 6

### Konstruktion des Plasmids pHis,His-Xa-IFN-γ

### A. Prinzipien

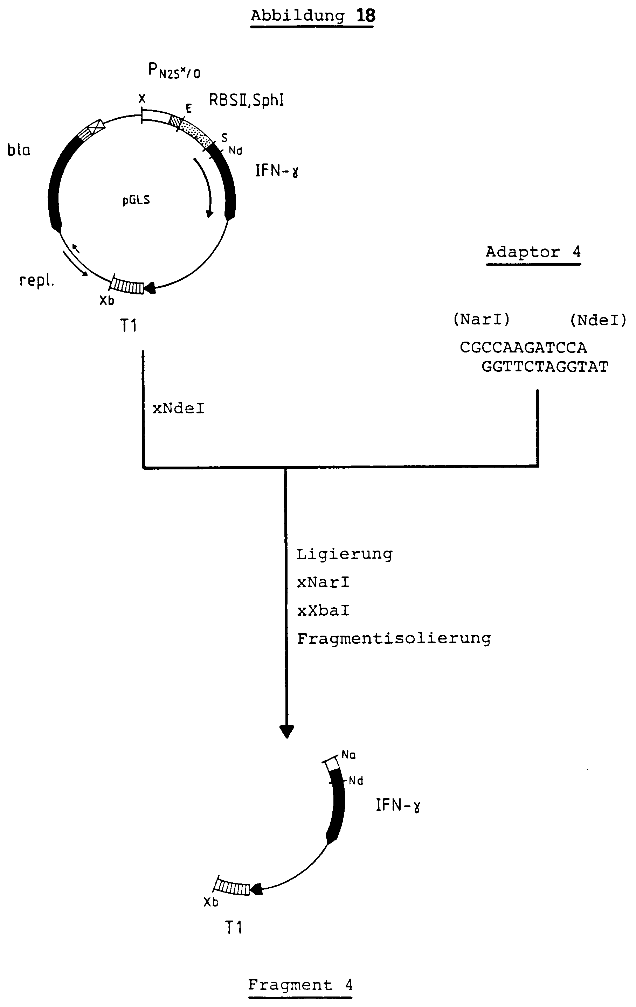

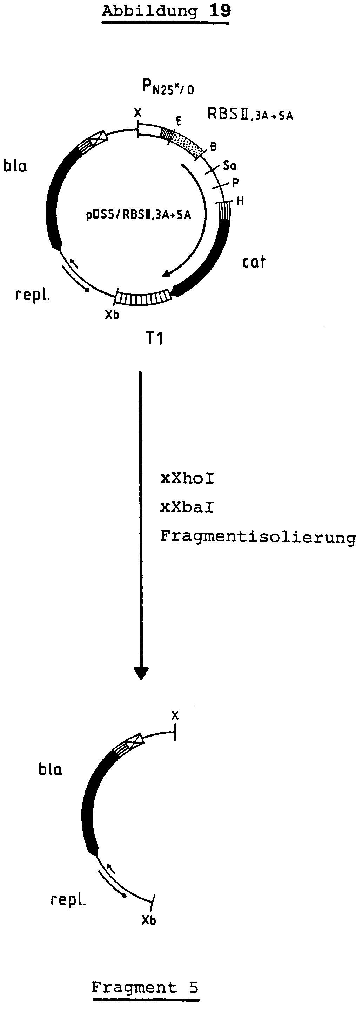

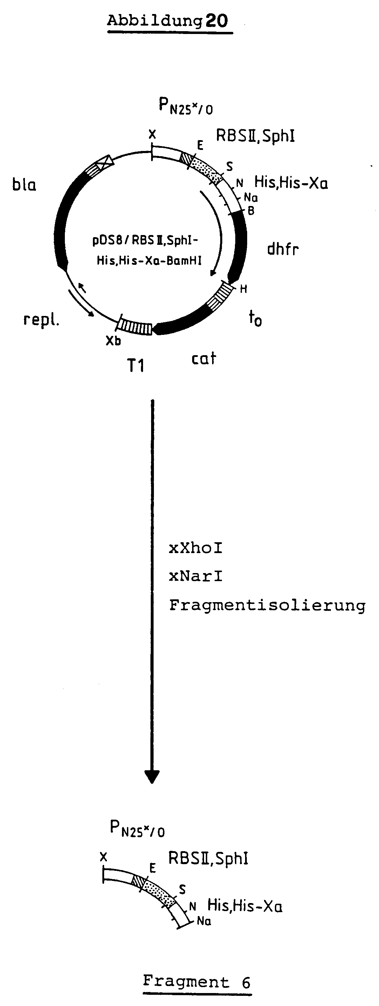

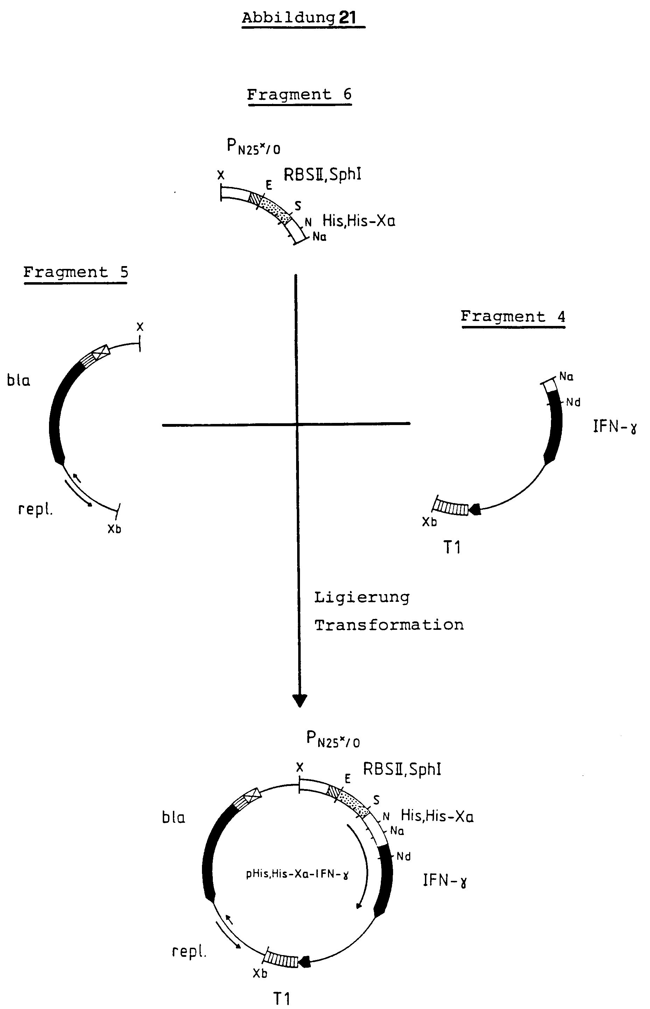

Zur Konstruktion des Plasmids pHis,His-Xa-IFN-γ wurden die folgenden DNA-Fragmente isoliert und miteinander verbunden (Abb.21): 1), das mit dem Adaptor 4 (Abb.11) verbundene IFN-γ Gen des Plasmids pGLS (Fragment 4, Abb.18); 2), die Signaleinheit des Plasmids pDS8/RBSII,SphI-His,His-Xa-BamHI, die den Promoter P_{N25}x_{/O}, die ribosomale Bindungsstelle RBSII,SphI und die für die benachbarten Histidine und für die Erkennungsstelle des Faktors Xa kodierende Region enthält (Fragment 6, Abb.20); und 3), die Replikationsregion mit dem β-Lactamase Gen aus dem Plasmid pDS5/RBSII,3A+5A (Fragment 5, Abb.19).

### B. Präparation des Fragments 4

2 pmol des Plasmids pGLS wurden mit dem Restriktionsenzym NdeI geschnitten. Nach Hitzeinaktivierung des Enzyms wurde die Probe mit Phenol extrahiert, ausgeäthert und die DNA, wie beschrieben, ausgefällt. Das Sediment wurde in 10 »l Ligasepuffer gelöst. 50 pmol des phosphorylierten Adaptors 4 (Abb.11), gelöst in Ligasepuffer, wurden dem NdeI geschittenen Plasmid pGLS zugegeben und die Probe mit 2E Ligase inkubiert (22°C, 3 Stunden). Nach Hitzeinaktivierung der Ligase wurde die Probe mit Phenol extrahiert, ausgeäthert und die DNA, wie beschrieben, ausgefällt. Das Sediment wurde gelöst und die DNA mit den Restriktionsenzymen NarI und XbaI geschnitten. Nach Zugabe von Probenpuffer, 7-minütigem Erhitzen der Mischung bei 65°C und Auftrennung der DNA in einem 6%igen Polyacrylamidgel wurde das NarI/XbaI-Fragment, das das IFN-γ-Gen enthielt, wie beschrieben, isoliert. Dieses Fragment erhielt die Bezeichnung Fragment 4 (Abb.18).

### C. Präparation des Fragments 5

2 pmol des Plasmids pDS5/RBSII,3A+5A wurden mit den Restriktionsenzymen XhoI und XbaI geschnitten. Die Mischung wurde, wie oben beschrieben, aufgearbeitet und die DNA in einem 6%igen Polycrylamidgel aufgetrennt. Das Fragment, welches das bla-Gen und die Replikationsregion enthielt, wurde, wie beschrieben, aus dem Gel isoliert. Dieses Fragment erhielt die Bezeichnung Fragment 5 (Abb.19).

### D. Präparation des Fragments 6

2 pmol des Plasmids pDS8/RBSII,SphI-His,His-Xa-BamHI wurden mit den Restriktionsenzymen XhoI und NarI geschnitten. Nach Aufarbeitung der Probe und Gelelektrophorese wurde das Fragment 6 isoliert, welches den Promotor P_{N25}x_{/O}, die ribosomale Bindungsstelle RBSII,SphI sowie den Bereich enthält, der für die benachbarten Histidine und die Erkennungsstelle für den Faktor Xa kodiert (Abb.20).

### E. Zusammensetzen des Plasmids pHis,His-Xa-IFN-γ

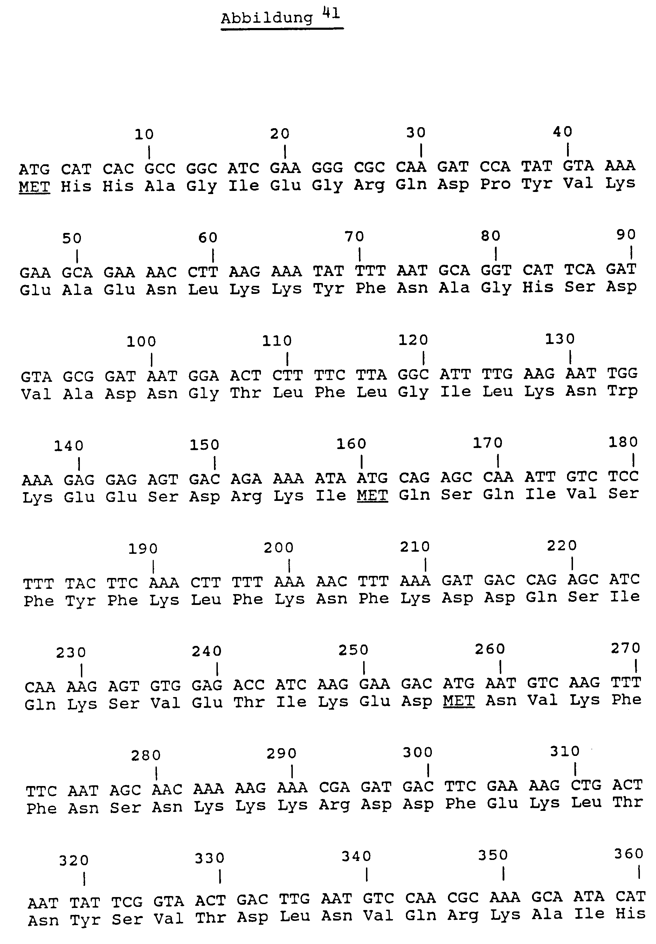

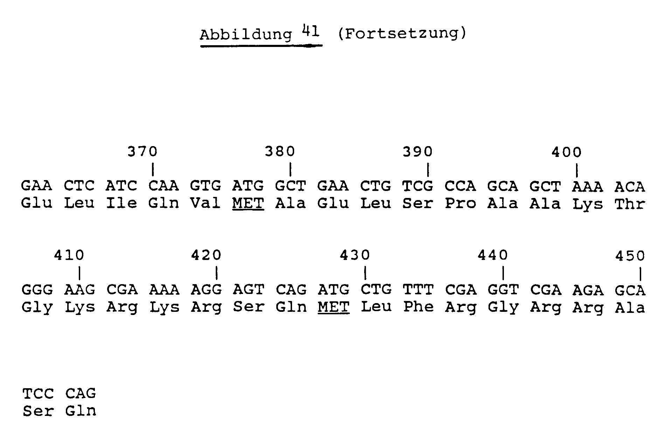

Je 0,5 pmol der Fragmente 4 (Abb.18), 5 (Abb.19) und 6 (Abb.20) wurden in Ligasepuffer mit 2E T4-DNA-Ligase inkubiert (22°C, 5 Stunden). Nach Hitzeinaktivierung des Enzyms wurde der Ansatz, wie oben beschrieben, in den E. coli-Stamm M15, der das Plasmid pDMI.1 enthielt, transformiert und das Transformationsgemisch auf LB-Agarplatten, die 100 »g/ml Ampicillin und 25 »g/ml Kanamycin enthielten, ausplattiert. Etwa 100 Transformanten wurden nach Inkubation bei 37°C über Nacht erhalten. Einzelne Kolonien wurden in 10 ml LB-Medium, wie beschrieben, aufgewachsen und die Plasmide nach der Methode von Birnboim und Doly (supra) isoliert. Alle Plasmide wurden mit den Restriktionsenzymen XhoI, BamHI und XbaI geschnitten und die Fragmente in 6%igen Polyacrylamidgelen analysiert. Die Restriktionsenzymanalyse ergab, dass die Plasmide die 3 gewünschten Fragmente enthielten. Die Sequenzanalysen wurden wie beschrieben (Beispiel 3,F) durchgeführt und zeigten, dass der Adaptor 4 korrekt mit dem IFN-γ-Gen fusioniert worden war. Diese Plasmide erhielten den Namen pHis,His-Xa-IFN-γ (Abb.21). Das von diesen Plasmiden kodierte IFN-γ Fusionsprotein (Aminosäuresequenz siehe Abb.41) erhielt die Bezeichnung His,His-Xa-IFN-γ.

### Beispiel 7

### Konstruktion des Plasmids pHis,His-Ek-IFN-γ(-8)

### A. Prinzipien

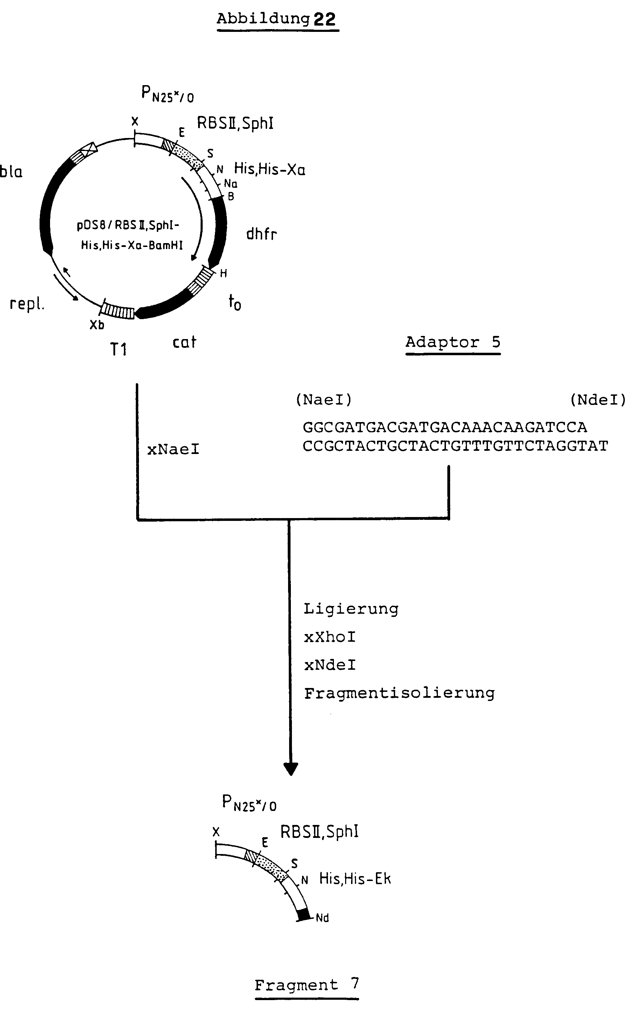

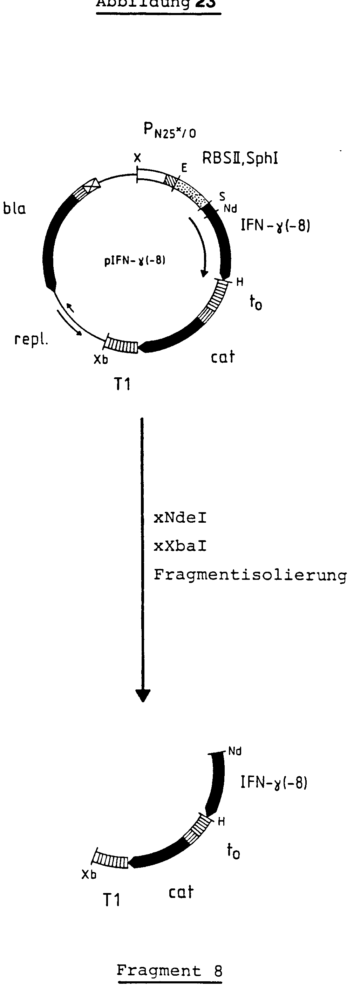

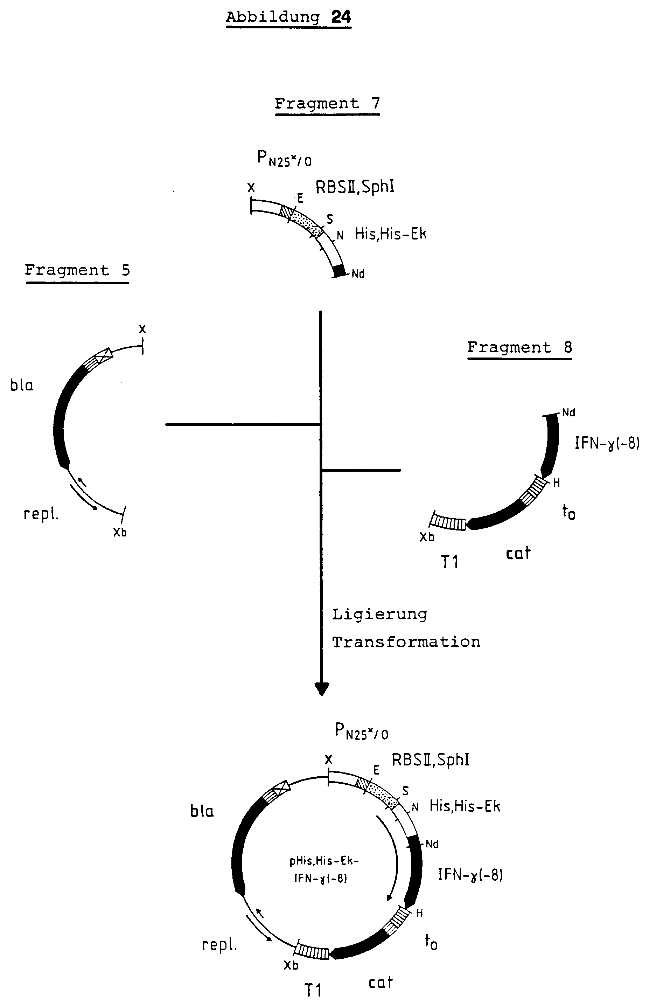

Zur Konstruktion des Plasmids pHis,His-Ek-IFN-γ(-8) wurden die folgenden 3 DNA Fragmente miteinander verbunden (Abb.24): 1), ein Fragment aus dem Plasmid pDS8/RBSII,SphI-His,His-Xa-BamHI enthaltend den Promotor P_{N25}x_{/O}, die ribosomale Bindungsstelle RBSII,SphI und die für die benachbarten Histidine kodierende Region, welche mit Hilfe des Adaptors 5 (Abb.11) um eine für die Erkennungsstelle der Enterokinase (EK)-kodierende Region verlängert wurde (Fragment 7, Abb.22); 2), ein Fragment aus dem Plasmid pIFN-γ(-8), welches das Gen für IFN-γ(-8) enthält (Fragment 8, Abb.23); und 3), ein Fragment aus dem Plasmid pDS5/RBSII,3A+5A mit der Replikationsregion und dem β-Lactamase Gen (Fragment 5, Abb.19). Die Präparation des letztgenannten Fragments wurde in Beispiel 6 beschrieben.

### B. Präparation des Fragments 7

4 pmol des Plasmids pDS8/RBSII,SphI-His,His-Xa-BamHI wurden mit dem Restriktionsenzym NaeI geschnitten. Anschliessend wurde das Enzym hitzeinaktiviert, die Probe mit Phenol extrahiert, ausgeäthert und die DNA, wie beschrieben, gefällt. Das Sediment wurde in 50 »l TE-Puffer aufgenommen. 1,5 pmol der geschnittenen DNA wurden in einem Volumen von 200 »l mit 30 pmol des phosphorylierten Adaptors 5 (Abb.11) und 7E T4-DNA-Ligase während 14 Stunden in Ligasepuffer inkubiert. Nach Hitzeinaktivierung des Enzyms wurde die DNA mit den Restriktionsenzymen NdeI und XhoI geschnitten. Anschliessend wurden die Enzyme hitzeinaktiviert, die Probe mit Phenol extrahiert, ausgeäthert und die DNA, wie beschrieben, gefällt, Das Sediment wurde in Probenpuffer aufgenommen und die Mischung für 7 Minuten bei 65°C inkubiert. Danach wurde die DNA in einem 6%igen Polyacrylamidgel aufgetrennt. 0,2 pmol des XhoI/NdeI-Fragments, enthaltend den Promotor P_{N25}x_{/O}, die ribosomalen Bindungsstelle RBSII,SphI sowie den für die benachbarten Histidine und für die Erkennungsstelle der Enterokinase kodierenden Bereich, wurden, wie beschrieben, aus dem Gel isoliert. Dieses DNA-Fragment erhielt die Bezeichnung Fragment 7 (Abb.22).

### C. Präparation des Fragments 8

0,5 pmol des Plasmids pIFN-γ(-8) wurden mit den Restriktionsenzymen NdeI und XbaI geschnitten. Anschliessend wurden die Enzyme hitzeinaktiviert, die Probe mit Phenol extrahiert, ausgeäthert und die DNA, wie beschrieben, gefällt. Das Sediment wurde in Probenpuffer aufgenommen und die Mischung für 7 Minuten bei 65°C inkubiert. Die DNA wurde dann in einem 6%igen Polyacrylamidgel aufgetrennt. 0,05 pmol des NdeI-XbaI-Fragments, enthaltend das IFN-γ(-8) Gen, den Terminator tₒ, das cat-Gen sowie den Terminator T1 wurden, wie beschrieben, aus dem Gel isoliert. Dieses DNA-Fragment erhielt die Bezeichnung Fragment 8 (Abb.23).

### D. Zusammensetzen des Plasmids pHis,His-Ek-IFN-γ(-8)

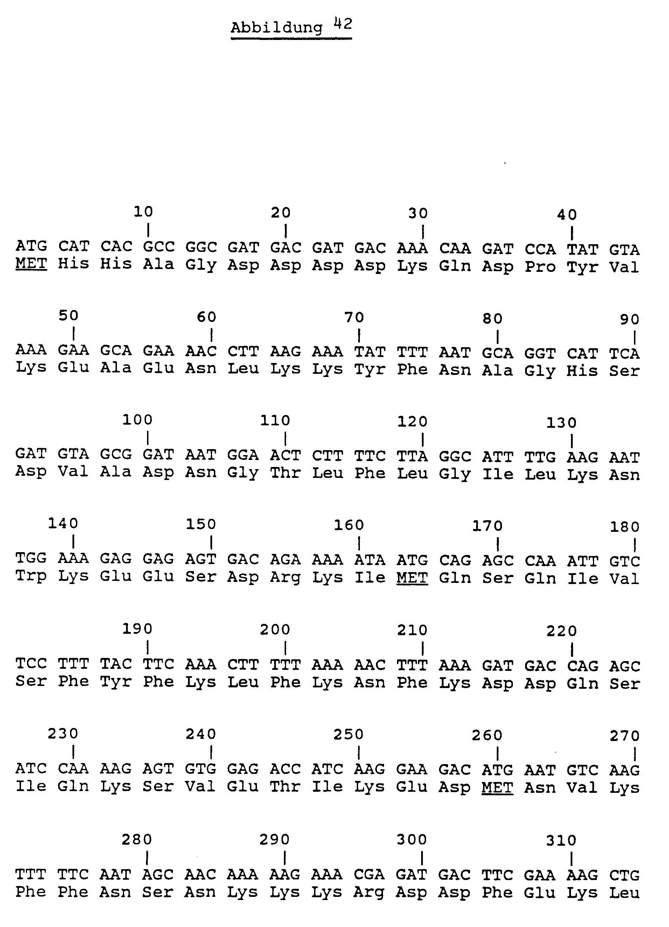

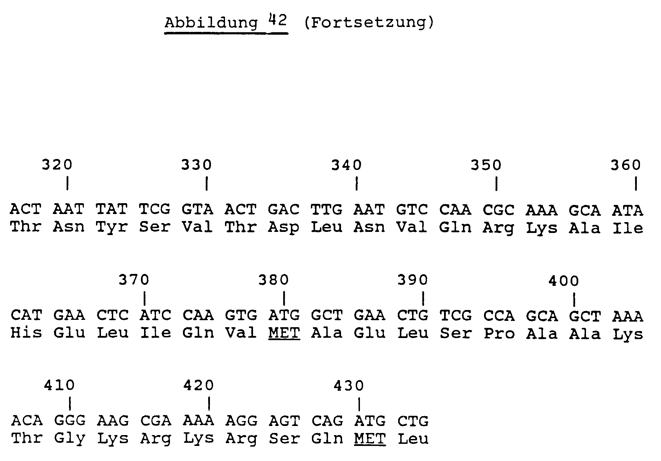

0,006 pmol des Fragments 5 (Abb.19), 0,02 pmol des Fragments 7 (Abb.22) und 0,005 pmol des Fragments 8 (Abb.23) wurden in Ligasepuffer in einem Volumen von 30 »l mit 0,5 E T4-DNA-Ligase für 3 Stunden bei 15°C inkubiert. Nach Hitzeinaktivierung des Enzyms wurde der Ansatz, wie oben beschrieben, in den E. coli Stamm M15¸ der das Plasmid pDMI,1 enthielt, transformiert und das Transformationsgemisch auf LB-Platten, die 100 »g/ml Ampicillin, 25 »g/ml Kanamycin und 5 »g/ml Chloramphenicol enthielten, ausplattiert. Nach Inkubation der Platten für 24 Stunden bei 37°C, wurden 2 der erhaltenen Transformanten in 10 ml LB-Medium, enthaltend 100 »g/ml Ampicillin, 25 »g/ml Kanamycin und 5 »g/ml Chloramphenicol, aufgewachsen und die Plasmide nach der Methode von Birnboim und Doly (supra) isoliert. Die Plasmide wurden hinsichtlich ihrer Grösse in 0,7%igen Agarosegelen und hinsichtlich ihrer Zusammensetzung mit Hilfe der Restriktionsenzyme HindIII, HinfI, NdeI, SphI, XbaI und XhoI in 6%igen Polyacrylamidgelen analysiert. Beide Plasmide enthielten die gewünschten 3 DNA-Fragmente in der richtigen Orientierung zueinander. Die Sequenzanalysen, die wie in Beispiel 3,F beschrieben, durchgeführt wurden, ergaben, dass die ribosomale Bindungsstelle RBSII,SphI mit den nachfolgenden Elementen korrekt mit IFN-γ(-8) Gen verbunden worden waren. Diese Plasmide erhielten die Bezeichnung pHis,His-Ek-IFN-γ(-8) (Abb.24). Das von diesen Plasmiden kodierte IFN-γ-Fusionsprotein (Aminosäuresequenz, Abb.42) erhielt die Bezeichnung His,His-Ek-IFN-γ(-8).

### Beispiel 8

### Konstruktion des Plasmids pHis,His-Xa-IFN-γ(-8)(Asn)

### A. Prinzipien

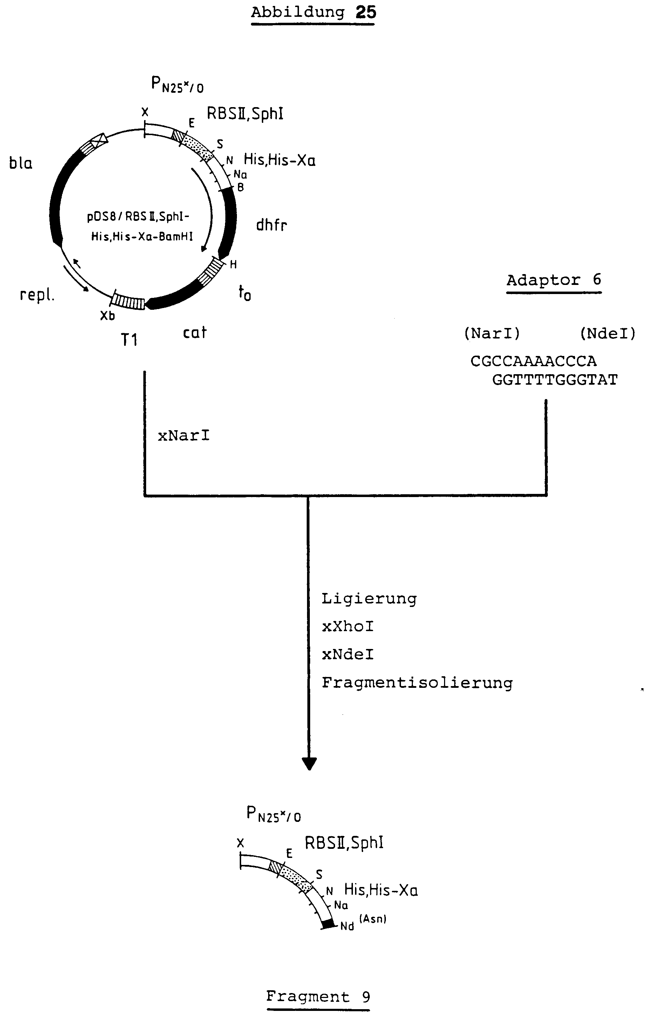

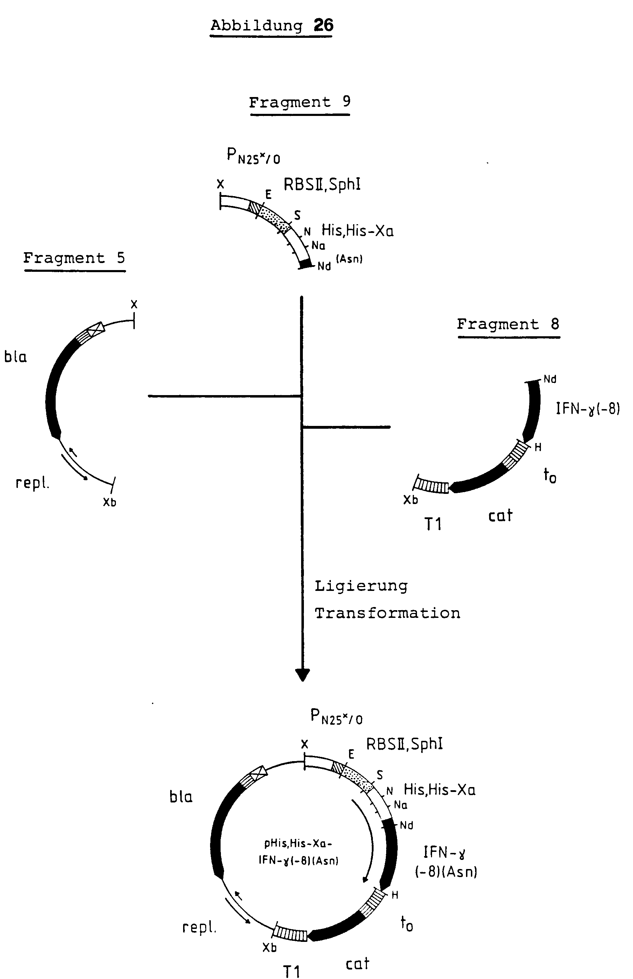

Zur Konstruktion des Plasmids pHis,His-Xa-IFN-γ(-8)(Asn) wurden die folgenden 3 DNA-Fragmente miteinander verbunden (Abb.26): 1., ein Fragment aus dem Plasmid pIFN-γ(-8), welches das IFN-γ(-8), Gen enthielt (Fragment 8, Abb.23, dessen Herstellung in Beispiel 7 beschrieben wurde); 2., ein Fragment aus dem Plasmid pDS8/RBsII,SphI-His,His-Xa-BamHI enthaltend den Promotor P_{N25}x_{/O}, die ribosomale Bindungsstelle RBSII,SphI sowie die für die benachbarten Histidine und die für die Erkennungsstelle des Faktors Xa kodierenden Region, welche mit Hilfe des Adaptors 6 (Abb.11) verlängert wurde, so dass durch Verknüpfung mit dem unter 1) beschriebenen Fragment das IFN-γ(-8)-Derivat IFN-γ(-8)(Asn) kodiert wird (Fragment 9, Abb.25); und 3., ein Fragment aus dem Plasmid pDS5/RBSII,3A+5A mit der Replikationsregion und dem Gen für β-Lactamase (Fragment 5, Abb.19, dessen Herstellung in Beispiel 6 beschrieben wurde).

### B. Präparation des Fragments 9

2 pmol des Plasmids pDS8/RBSII,SphI-His,His-Xa-BamHI wurden mit dem Restriktionsenzym NarI geschnitten. Anschliessend wurde das Enzym hitzeinaktiviert, die Probe mit Phenol extrahiert, ausgeäthert und die DNA, wie beschrieben, gefällt. Das Sediment wurde in 50 »l TE-Puffer aufgenommen. 1 pmol der geschnittenen DNA wurde in einem Volumen von 150 »l mit 30 pmol des phosphorylierten Adaptors 6 (Abb.11) und 5 E T4-DNA-Ligase für 14 Stunden bei 20°C in Ligasepuffer inkubiert. Nach Hitzeinaktivierung des Enzyms wurde die DNA mit den Restriktionsenzymen NdeI und XhoI geschnitten. Anschliessend wurden die Enzyme hitzeinaktiviert, die Probe mit Phenol extrahiert, ausgeäthert und die DNA, wie beschrieben, gefällt. Das Sediment wurde in Probenpuffer aufgenommen und der Ansatz für 7 Minuten bei 65°C inkubiert. Die resultierende DNA-Mischung wurde dann in einem 6%igen Polyacrylamidgel aufgetrennt. 0,25 pmol des XhoI/NdeI-Fragments mit dem Promotor P_{N25}x_{/O}, der ribosomalen Bindungsstelle RBSII,SphI und der Region, die für die benachbarten Histidine, für die Erkennungsstelle des Faktors Xa sowie für die Aminosäure Asn kodiert, wurden, wie beschrieben, aus dem Gel isoliert. Dieses DNA-Fragment erhielt die Bezeichnung Fragment 9 (Abb.25).

### C. Zusammensetzen des Plasmids pHis,His-Xa-IFN-γ(-8)(Asn)

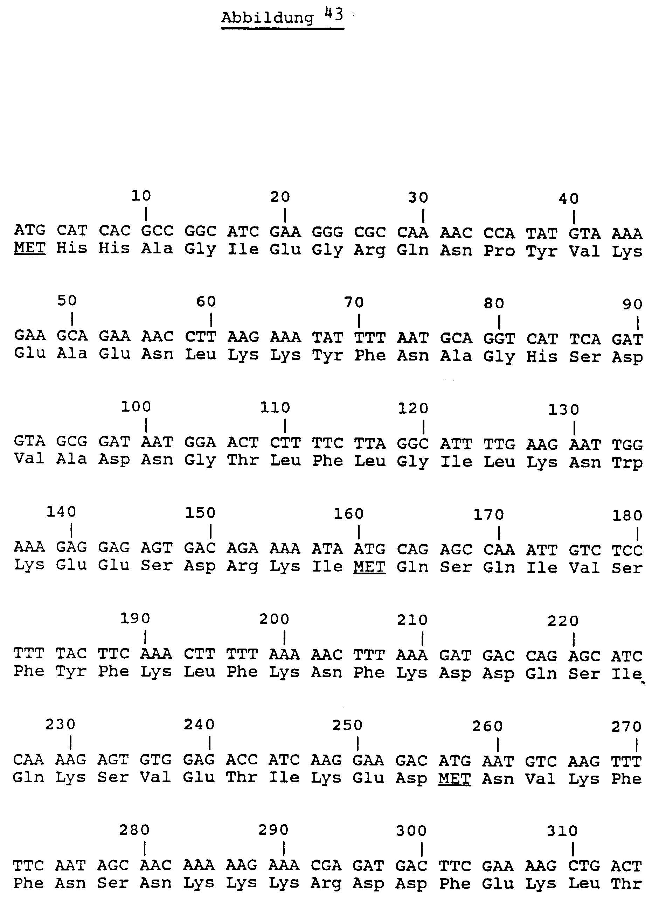

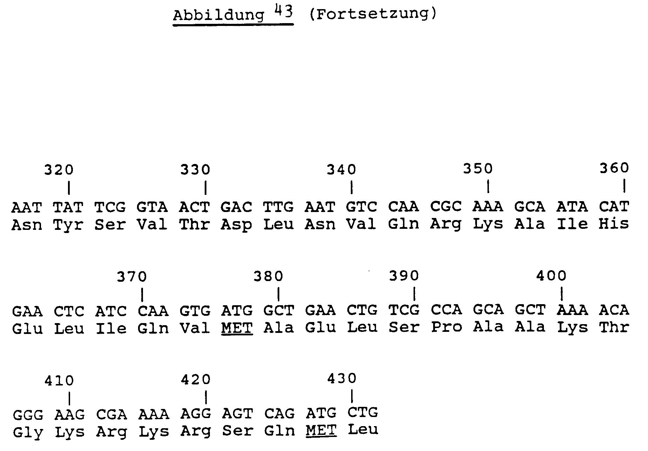

0,006 pmol des Fragments 5 (Abb.19), 0,005 pmol des Fragments 8 (Abb.23) und 0,02 pmol des Fragments 9 (Abb.25) wurden in 30 »l Ligasepuffer und 0,5 E T4-DNA-Ligase für 3 Stunden bei 15°C inkubiert. Nach Hitzeinaktivierung des Enzyms wurde der Ansatz, wie oben beschrieben, in den E. coli Stamm M15, der das Plasmid pDMI,1 enthielt, transformiert und das Transformationsgemisch auf LB-Platten enthaltend 100 »g/ml Ampicillin und 25 »g/ml Kanamycin ausplattiert. Nach Inkubation der Platten bei 37°C über Nacht wurden 2 Kolonien, wie beschrieben, in 10 ml LB-Medium, enthaltend 100 »g/ml Ampicillin und 25 »g/ml Kanamycin, aufgewachsen und die Plasmide nach der Methode von Birnboim und Doly (supra) isoliert. Die Plasmide wurden hinsichtlich ihrer Grösse in 0,7%igen Agarosegelen und hinsichtlich ihrer Zusammensetzung mit Hilfe der Restriktionsenzyme HindIII, HinfI, NdeI, SphI, XbaI und XhoI in 6%igen Polyacrylamidgelen analysiert. Eines der beiden Plasmide enthielt die gewünschten 3 DNA-Fragmente in der richtigen Orientierung zueinander. Die Sequenzanalyse dieses Plasmids wurde, wie beschrieben (Beispiel 3,F), durchgeführt, und sie ergab, dass die ribosomale Bindungsstelle RBSII,SphI mit den nachfolgenden Elementen korrekt mit der Region, die für IFN-γ(-8)(Asn) kodiert, verknüpft worden war. Dieses Plasmid erhielt die Bezeichnung pHis,His-Xa-IFN-γ(-8)(Asn) (Abb.26). Das von diesem Plasmid kodierte IFN-γ Fusionsprotein (Aminosäuresequenz siehe Abb.43) erhielt die Bezeichnung His,His-Xa-IFN-γ(-8)(Asn).

### Beispiel 9

### Konstruktion des Plasmids p6xHis-DHFR

### A. Prinzipien

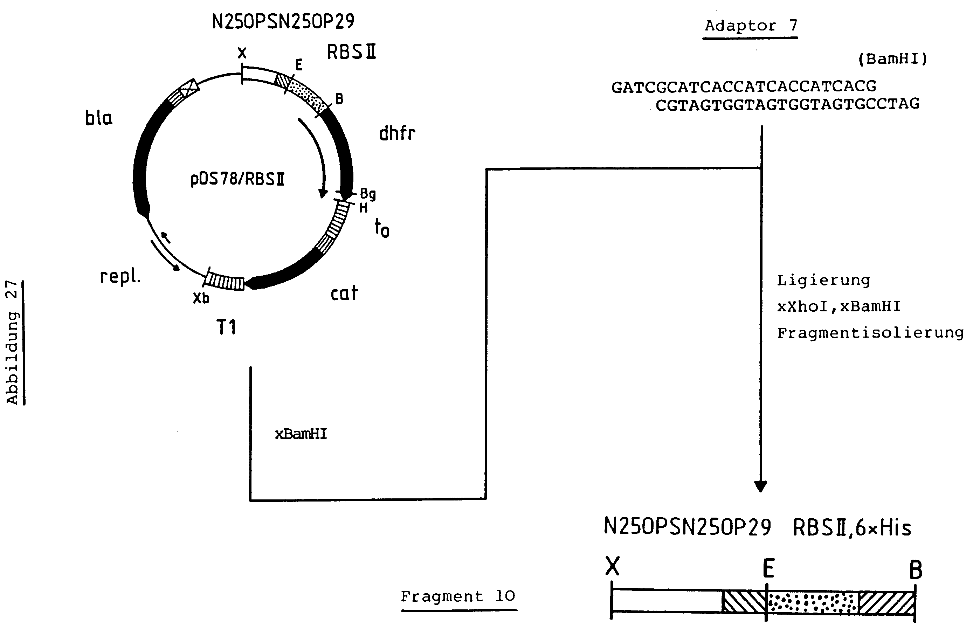

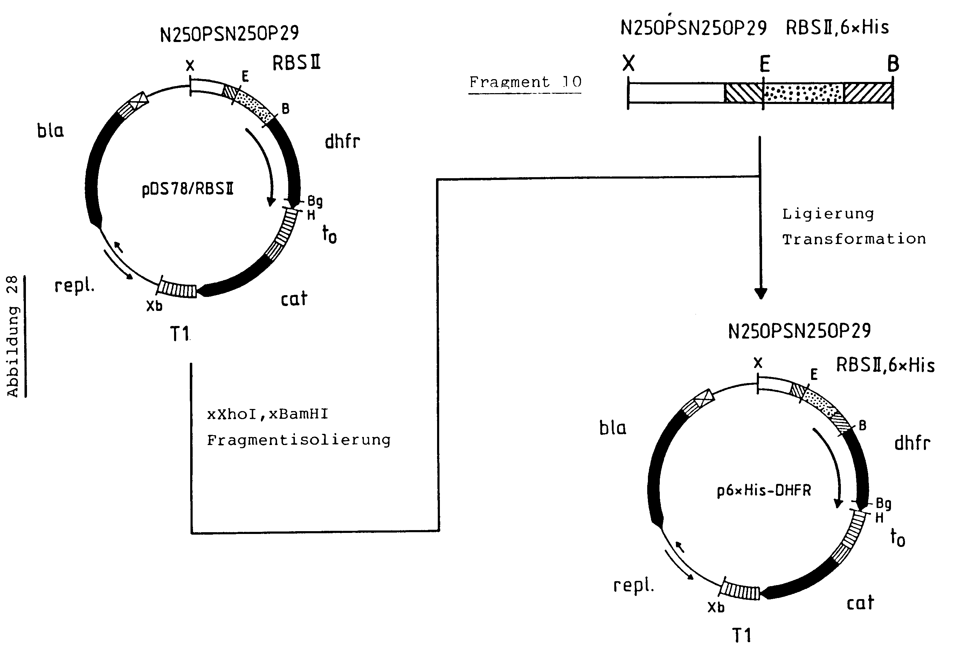

Zur Konstruktion des Plasmids p6xHis-DHFR wurden die folgenden DNA-Fragmente isoliert und miteinander verbunden (Abb.28): 1., die mit dem Adaptor 7 (Abb.11) verbundene Signaleinheit des Plasmids pDS78/RBSII mit dem Promotor N25OPSN25OP29 sowie die Ribosomenbindungsstelle RBSII (Fragment 10, Abb.27) und 2., das grössere der beiden XhoI/BamHI-Fragmente des Plasmids pDS78/RBSII (Abb.28).

### B. Präparation des Fragments 10

2 pmol des Plasmids pDS78/RBSII wurden mit dem Restriktionsenzym BamHI geschnitten. Nach Hitzeinaktivierung des Enzyms wurde die Probe mit Phenol extrahiert, ausgeäthert und die DNA, wie beschrieben, ausgefällt. Das Sediment wurde in 10 »l Ligasepuffer gelöst. 50 pmol des phosphorylierten Adaptors 7 (Abb.27), gelöst in Ligasepuffer, wurden dem geschnittenen Plasmid zugegeben und die Probe mit 2E Ligase inkubiert (22°C, 3 Stunden). Nach Hitzeinaktivierung der Ligase wurde die Probe mit Phenol extrahiert, ausgeäthert und die DNA, wie beschrieben, ausgefällt. Das Sediment wurde gelöst und die DNA mit den Restriktionsenzymen XhoI und BamHI geschnitten. Nach Zugabe von Probenpuffer, 7-minütigem Erhitzen der Mischung bei 65°C und Auftrennung der DNA in einem 6%igen Polyacrylamidgel wurde das XhoI/BamHI-Fragment mit dem Promotor N25OPSN25OP29, der Ribosomenbindungsstelle RBSII und dem für 6 Histidine kodierenden Bereich, wie beschrieben, isoliert. Dieses Fragment erhielt die Bezeichnung Fragment 10 (Abb.27).

### C. Präparation des BamHI/XhoI-Fragments des Plasmids pDS78/RBSII

2 pmol des Plasmids pDS78/RBSII wurden mit den Restriktionsenzymen XhoI und BamHI geschnitten. Nach Aufarbeitung der Probe und Gelektrophorese wurde das Fragment isoliert, welches die Replikationsregion enthält (Abb.28).

### D. Zusammensetzen des Plasmids p6xHis-DHFR

Je 0,1 pmol der besagten Fragmente wurden in Ligasepuffer mit 2E T4-DNA-Ligase inkubiert (22°C, 3 Stunden). Nach Hitzeinaktivierung des Enzyms wurde die Mischung, wie oben beschrieben, in den E. coli-Stamm M15, der das Plasmid pDMI,1 enthielt, transformiert, das Transformationsgemisch auf LB-Agarplatten, die 100 »g/ml Ampicillin und 25 »g/ml Kanamycin enthielten, ausplattiert und die Platten über Nacht bei 37°C inkubiert. Einzelne Kolonien wurden in 10 ml LB-Medium, wie beschrieben, aufgewachsen und die Plasmide nach der Methode von Birnboim und Doly (supra) isoliert. Eine Restriktionsanalyse mit den Enzymen XhoI und BamHI ergab, dass die Plasmide die 2 gewünschten Fragmente enthielten. Die Sequenzanalyse wurde, wie beschrieben (Beispiel 3,E), durchgeführt und bestätigte, dass der Adaptor 7 korrekt mit der Ribosomenbindungsstelle verknüpft worden war. Diese Plasmide, die das DHFR-Fusionsprotein (His)₆-mDHFR kodieren, erhielten die Bezeichnung p6xHis-DHFR (Abb.28).

### Beispiel 10

### Konstruktion des Plasmids p4xHis-DHFR

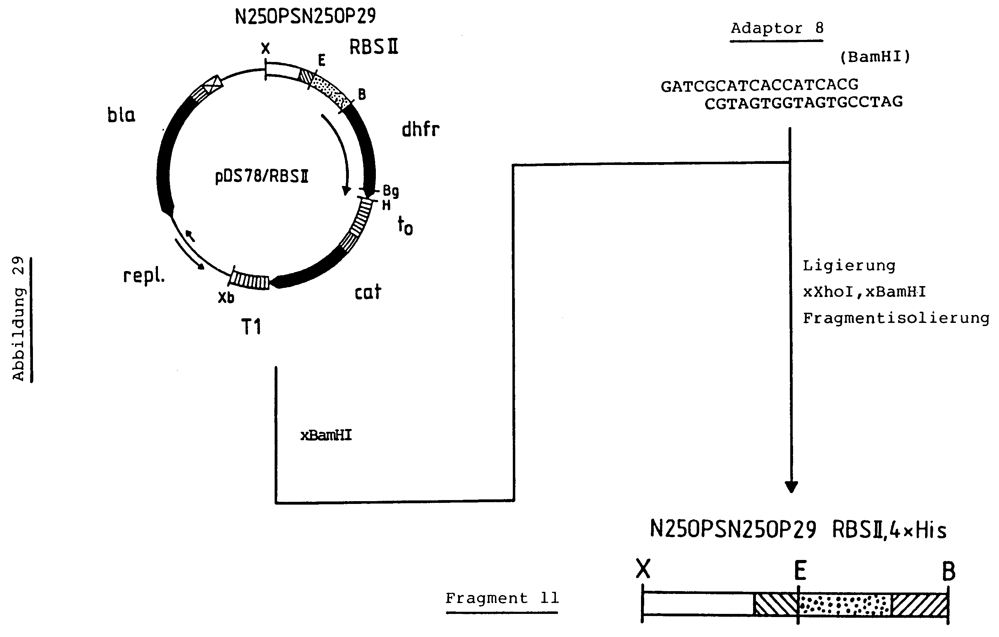

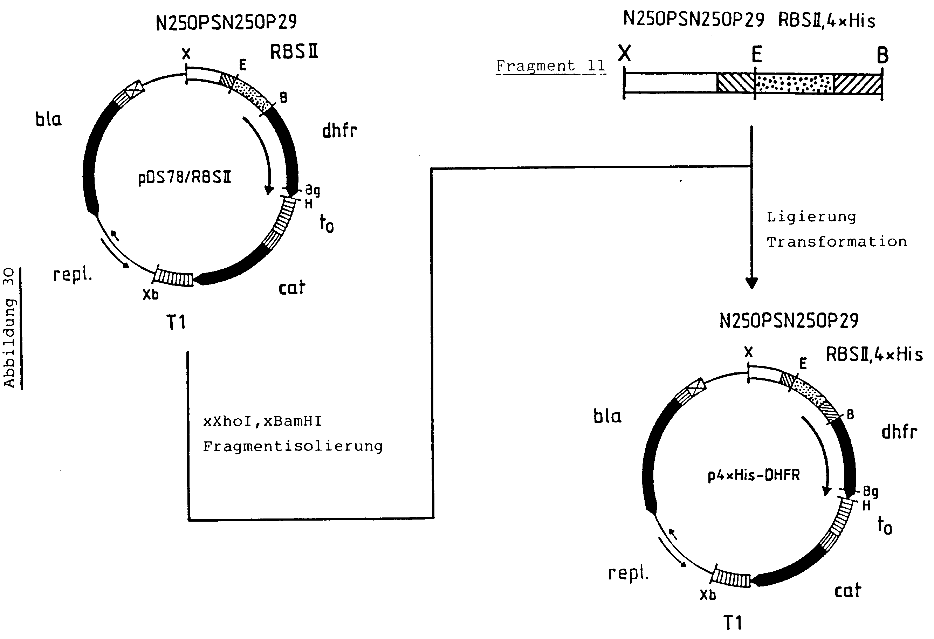

Die Konstruktion des Plasmids p4xHis-DHFR wurde analog zur Konstruktion des Plasmids p6xHis-DHFR (Beispiel 9) durchgeführt, wobei folgende DNA-Fragmente isoliert und miteinander verbunden wurden (Abb.30): 1., die mit dem Adaptor 8 (Abb. 11) verbundene Signaleinheit des Plasmids pDS78/RBSII mit dem Promotor N25OPSN25OP29 sowie der Ribosomenbindungsstelle RBSII (Fragment 11, Abb.29) und 2., das grössere der beiden XhoI/BamHI-Fragmente des Plasmids pDS78/RBsII (Abb.30). Das resultierende Plasmid p4xHis-DHFR kodiert das DHFR-Fusionsprotein (His)₄-mDHFR.

### Beispiel 11

### Konstruktion des Plasmids pRBSII-6xHis

### A. Prinzipien

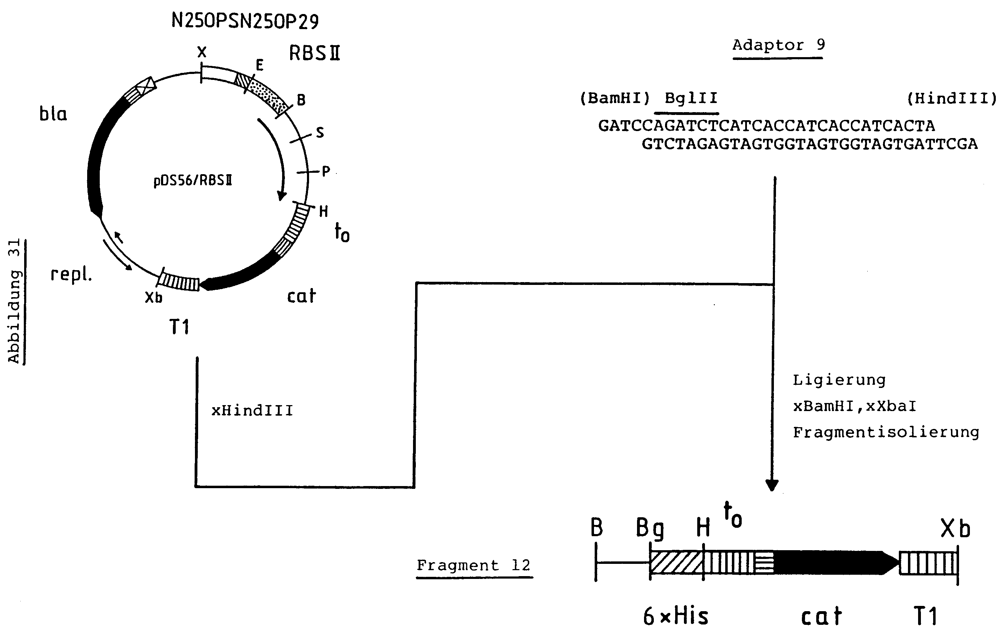

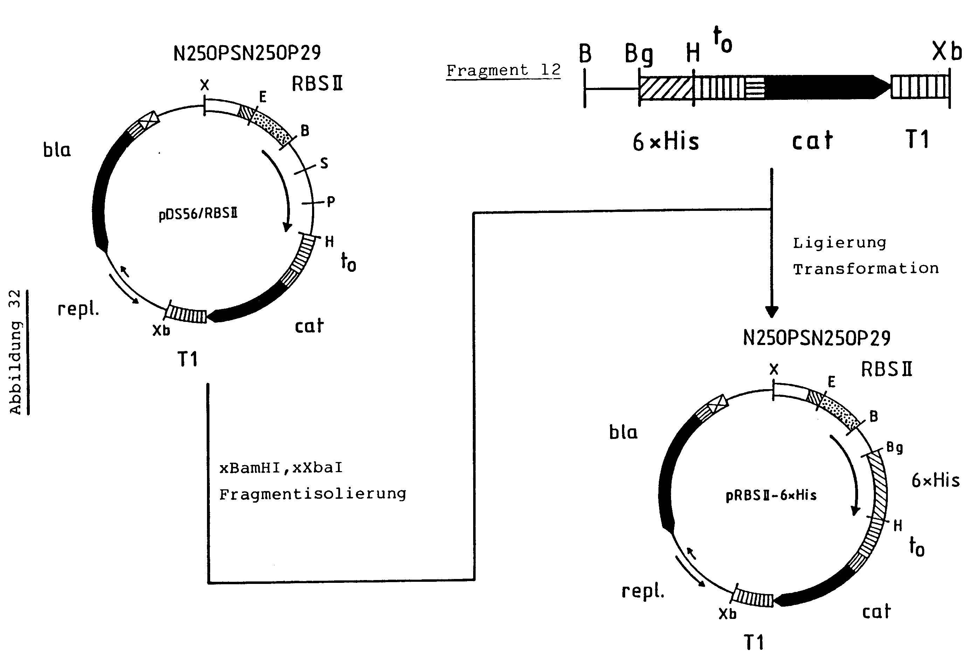

Zur Konstruktion des Plasmids pRBSII-6xHis wurden die folgenden DNA-Fragmente isoliert und miteinander verbunden (Abb.32): 1., die um den Adaptor 9 (der für 6 Histidine kodiert) verlängerte Region aus dem Plasmid pDS56/RBSII mit dem Terminator tₒ, dem cat-Gen und dem Terminator T1 (Fragment 12, Abb.31) und 2., das XbaI/BamHI-Fragment aus dem Plasmid pDS56/RBsII mit der Replikationsregion, dem bla-Gen, dem Promotor N25OPSN25OP29 und der Ribosomenbindungsstelle RBSII (Abb.32).

### B. Präparation des Fragments 12

2 pmol des Plasmids pDS56/RBsII wurden mit dem Restriktionsenzym HindIII geschnitten. Nach Aufarbeitung der Probe wurden dem geschnittenen Plasmid 50 pmol des phosphorylierten Adaptors 9 zugegeben und die Probe mit T4-DNA-Ligase, wie beschrieben, inkubiert. Nach Aufarbeitung des Ligierungsansatzes wurde die DNA mit den Restriktionsenzymen BamHI und XbaI geschnitten und das BamHI/XbaI-Fragment mit der für 6 Histidine kodierenden Region, dem Terminator tₒ, dem cat-Gen und dem Terminator T1, wie beschrieben, isoliert. Dieses Fragment erhielt die Bezeichnung Fragment 12 (Abb.31).

### C. Präparation des XbaI/BamHI-Fragments des Plasmids pDS56/RBSII

2 pmol des Plasmids pDS56/RBSII wurden mit den Restriktionsenzymen XbaI und BamHI geschnitten und das Fragment mit der Replikationsregion, dem bla-Gen dem Promotor N25OPSN25OP29 und der Ribosomenbindungsstelle RBSII wurde, wie beschrieben, isoliert (Abb.32).

### D. Zusammensetzen des Plasmids pRBSII-6xHis

Je 0,1 pmol der isolierten Fragmente wurden, wie beschrieben (Beispiel 9,D), ligiert und anschliessend in den E. coli-Stamm M15 (pDMI,1) transformiert. Nach Plattierung und Inkubation (Beispiel 9,D) wurden einzelnen Kolonien in 10 ml Medium, wie beschrieben, aufgewachsen und die Plasmide nach der Methode von Birnboim und Doly (supra) isoliert. Eine Restriktionsanalyse mit den Enzymen BamHI und XbaI ergab, dass die Plasmide die 2 gewünschten Fragmente enthielten. Die Sequenzanalyse (Beispiel 3,E) bestätigte, dass der Adaptor 9 korrekt in die Plasmid-DNA eingeführt wurde. Diese Plasmide erhielten die Bezeichnung pRBSII-6xHis.

### Beispiel 12

### Konstruktion des Plasmids pRBSII-4xHis

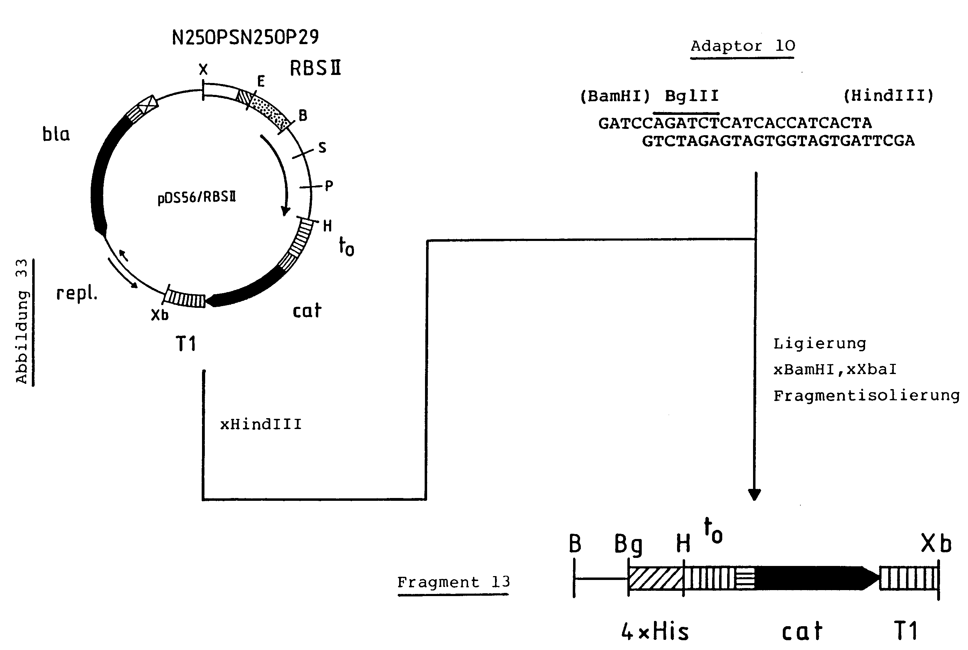

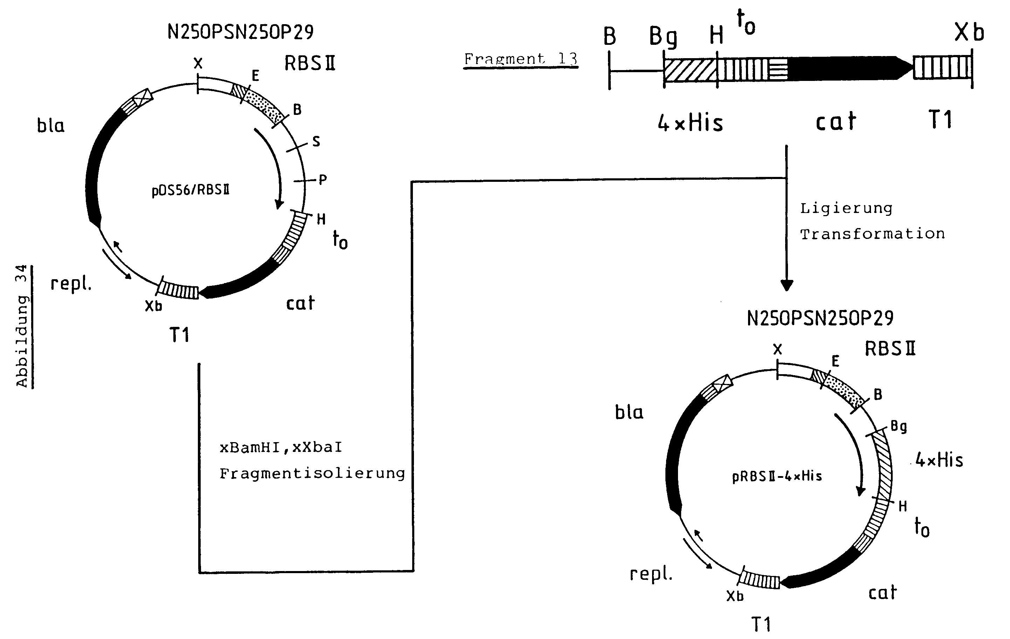

Die Konstruktion des Plasmids pRBSII-4xHis wurde analog zur Konstruktion des Plasmids pRBSII-6xHis (Beispiel 11) durchgeführt, wobei folgende DNA-Fragmente isoliert und miteinander verbunden wurden (Abb.34): 1., die um dem Adaptor 10 (der für 4 Histidine kodiert) verlängerte Region aus dem Plasmid pDS56/RBSII mit dem Terminator tₒ, dem cat-Gen und dem Terminator T1 (Fragment 13, Abb.33) und 2., das XbaI/BamI-Fragment aus dem Plasmid pDS56/RBSII mit der Replikationsregion, dem bla-Gen, dem Promotor N25OPSN25OP29 und der Ribosomenbindungsstelle RBSII (Abb. 34).

### Beispiel 13

### Konstruktion des Plasmids pRBSII-2xHis

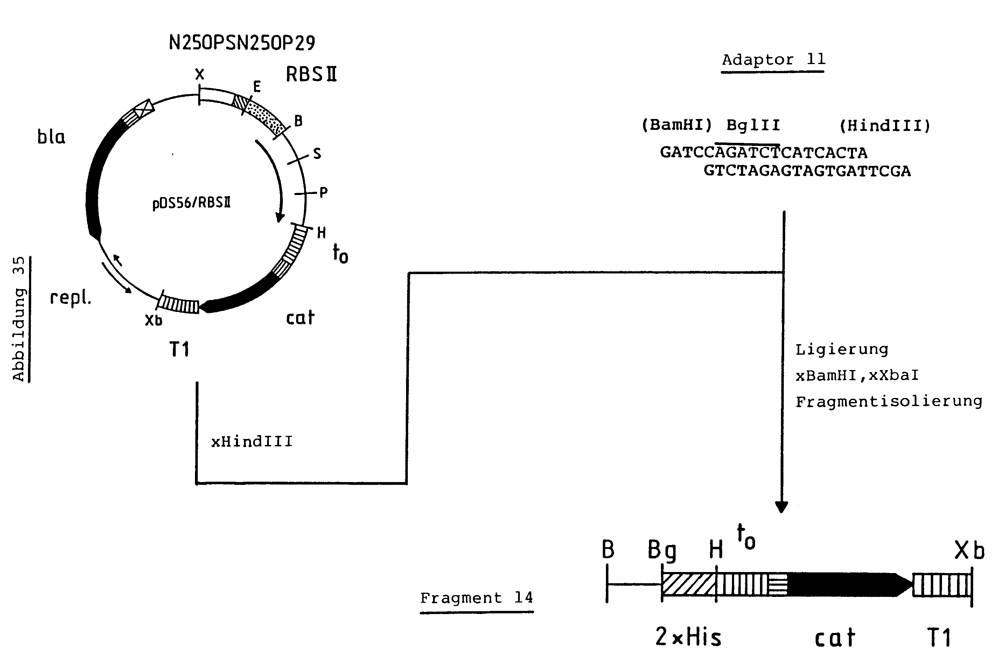

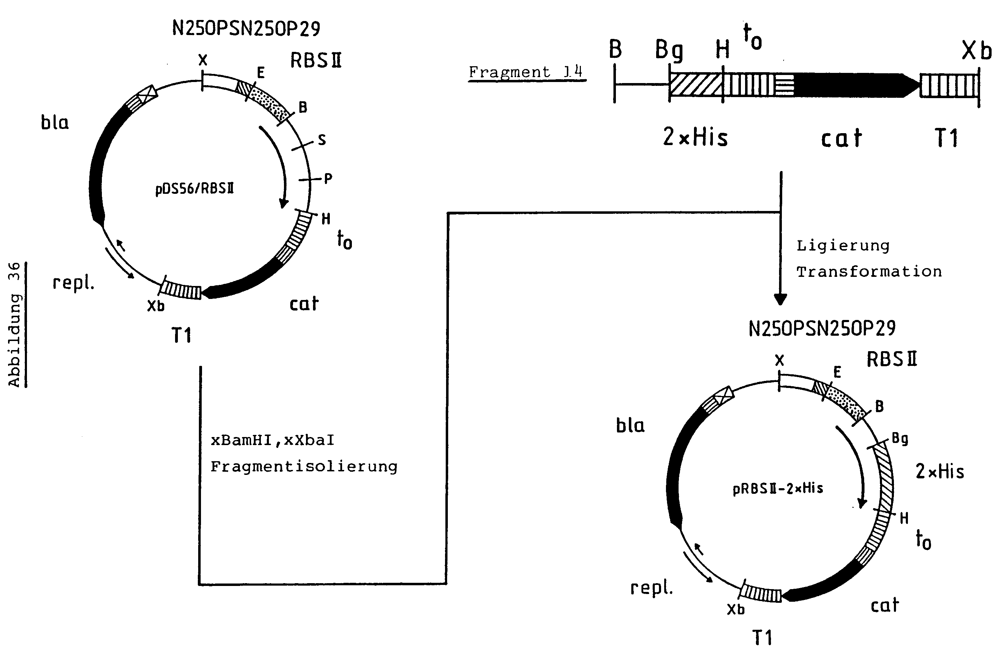

Die Konstruktion des Plasmids pRBSII-2xHis wurde analog zur Konstruktion des Plasmids pRBSII-6xHis (Beispiel 11) durchgeführt, wobei folgende DNA-Fragmente isoliert und miteinander verbunden wurden (Abb.36): 1., die um den Adaptor 11 (der für 2 Histidine kodiert) verlängerte Region aus dem Plasmid pDS56/RBSII mit dem Terminator tₒ, dem cat-Gen und dem Terminator T1 (Fragment 14, Abb.35) und 2., das XbaI-BamHI-Fragment aus dem Plasmid pDS56/RBSII mit der Replikationsregion, dem bla-Gen, dem Promotor N25OPSN25OP29 und der Ribosomenbindungsstelle RBSII (Abb.36).

### Beispiel 14

### Konstruktion des Plasmids pDHFR-6xHis

### A. Prinzipien

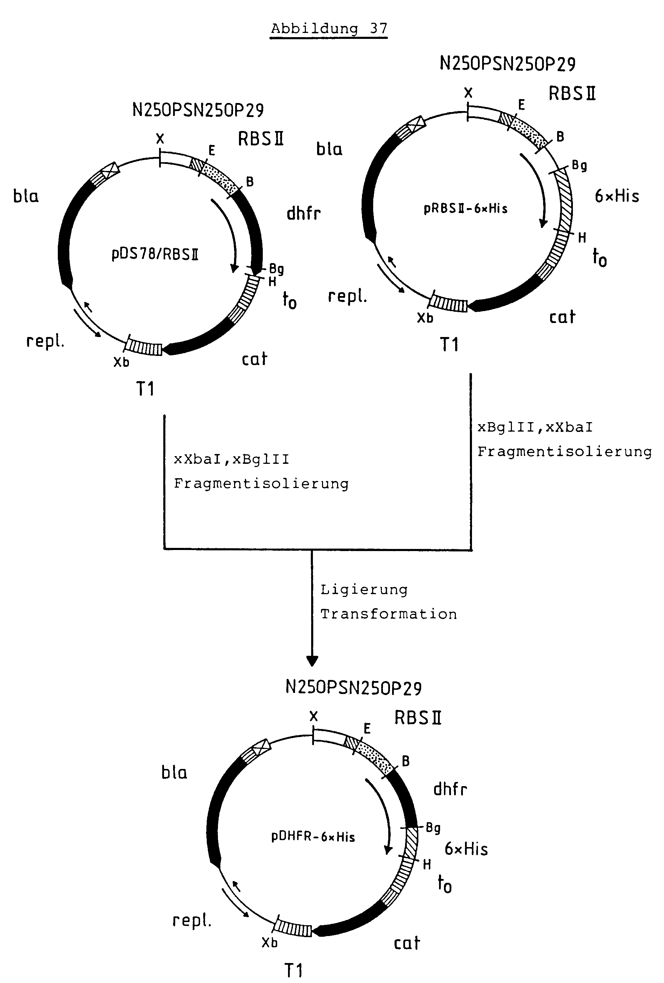

Zur Konstruktion des Plasmids pDHFR-6xHis wurden die folgenden DNA-Fragmente isoliert und miteinander verbunden (Abb.37): 1., das XbaI/BglII-Fragment aus dem Plasmid pDS78/RBSII mit der Replikationsregion, dem bla-Gen, dem Promotor N25OPSN25OP29, der Ribosomenbindungsstelle RBSII und dem dhfr-Gen und 2., das BglII/XbaI-Fragment aus dem Plasmid pRBSII-6xHis mit der für 6 Histidine kodierenden Region, dem Terminator tₒ, dem cat-Gen und dem Terminator T1. Das resultierende Plasmid pDHFR-6xHis kodiert das DHFR-Fusionsprotein Met-mDHFR-(His)₆.

### B. Präparation des XbaI/BglII-Fragments des Plasmids pDS78/RBSII

2 pmol des Plasmids pDS78/RBSII wurden mit den Restriktionsenzymen XbaI und BglII geschnitten. Nach der Aufarbeitung der Probe wurde das XbaI/BglII-Fragment mit der Replikationsregion, dem bla-Gen, dem Promotor N25OPSN25OP29, der Ribosomenbindungsstelle RBSII und dem dhfr-Gen, wie beschrieben, isoliert.

### C. Präparation des BglII/XbaI-Fragments des Plasmids pRBSII-6xHis

2 pmol des Plasmids pRBSII-6xHis wurden mit den Restriktionsenzymen BglII und XbaI geschnitten. Nach Aufarbeitung der Probe wurde das BglII/XbaI-Fragment mit der für 6 Histidine kodierenden Region, dem Terminator tₒ, dem cat-Gen und dem Terminator T1, wie beschrieben, isoliert.

### D. Zusammensetzen des Plasmids pDHFR-6xHis

Je 0,1 pmol der isolierten Fragmente wurden, wie beschrieben (Beispiel 9,D), ligiert und in den E. coli-Stamm M15 (pDMI,1) transformiert. Nach Plattierung und Inkubation (Beispiel 9,D) wurden einzelne Kolonien in 10 ml Medium wie beschrieben, aufgewachsen und die Plasmide nach der Methode von Birnboim und Doly (supra) isoliert. Eine Restriktionsanalyse mit den Enzymen XbaI und BglII ergab, dass die beiden Fragmente in der gewünschten Weise miteinander verbunden waren. Diese Plasmide erhielten die Bezeichnung pDHFR-6xHis.

### Beispiel 15

### Konstruktion des Plasmids pDHFR-2xHis

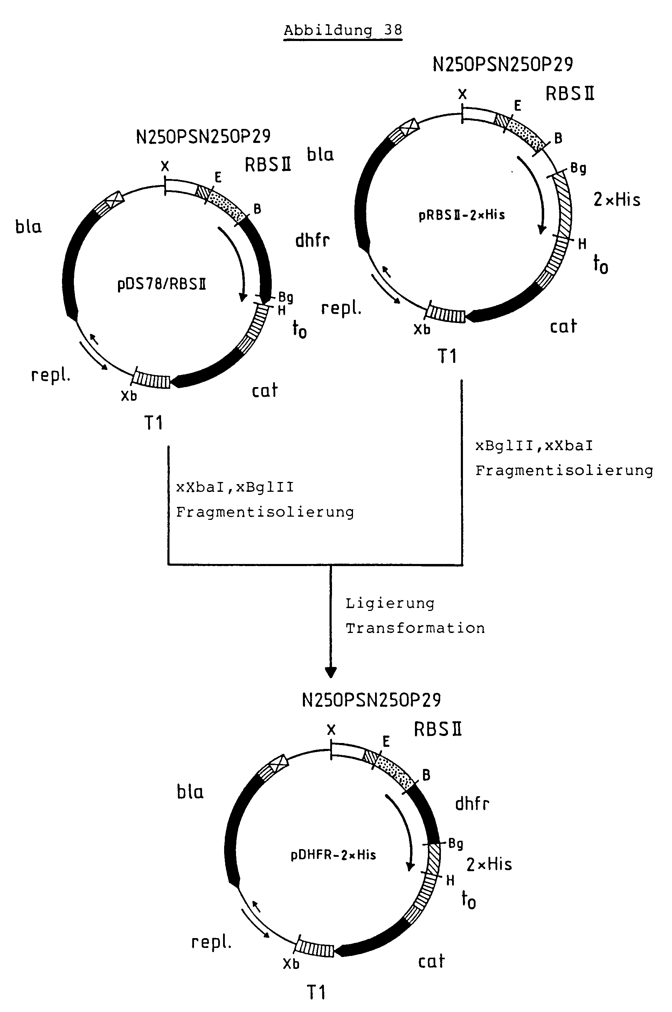

Die Konstruktion des Plasmids pDHFR-2xHis, welches das DHFR-Fusionsprotein Met-mDHFR-(His)₂ kodiert, wurde analog zur Konstruktion des Plasmids pDHFR-6xHis (Beispiel 14) durchgeführt, wobei folgende DNA-Fragmente isoliert und miteinander verbunden wurden (Abb.38): 1., das XbaI/BglII-Fragment aus dem Plasmid pDS78/RBSII mit der Replikationsregion, dem bla-Gen, dem Promotor N25OPSN25OP29, der Ribosomenbindungsstelle RBSII und dem dhfr-Gen und 2., BglII/XbaI-Fragment aus dem Plasmid pRBSII-2xHis mit der für 2 Histidine kodierenden Region, dem Terminator tₒ, dem cat-Gen und dem Terminator T1.

### Beispiel 16

### Konstruktion des Plasmids p4xHis-DHFR-4xHis

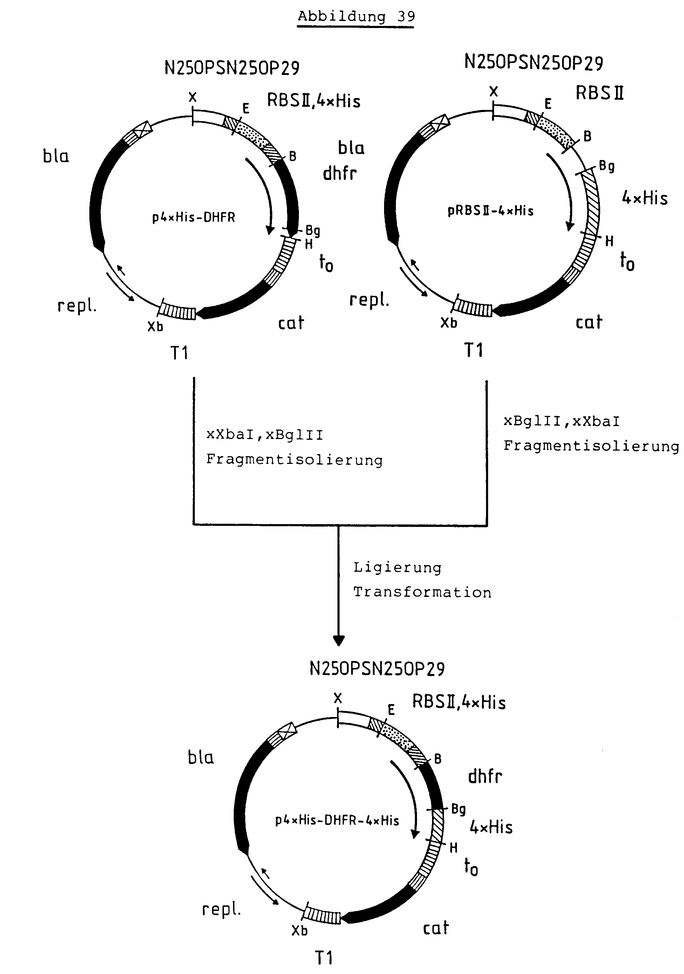

Die Konstruktion des Plasmids p4xHis-DHFR-4xHis, welches das DHFR-Fusionsprotein (His)₄-mDHFR-(His)₄ kodiert, wurde analog zur Konstruktion des Plasmids pDHFR-6xHis (Beispiel 14) durchgeführt, wobei folgende DNA-Fragmente isoliert und miteinander verbunden wurden (Abb.39): 1., das XbaI/BglII-Fragment aus dem Plasmid p4xHis-DHFR mit der Replikationsregion, dem bla-Gen, dem Promotor N25OPSN25OP29, der Ribosomenbindungsstelle RBSII,4xHis und dem dhfr-Gen und 2., das BglII/XbaI-Fragment aus dem Plasmid pRBSII-4xHis mit der für 4 Histidine kodierenden Region, dem Terminator tₒ, dem cat-Gen und dem Terminator T1.

### Beispiel 17

### Herstellung des NTA-Harzes

41,7 g Bromessigsäure wurden in 150 ml 2N Natronlauge gelöst und auf 0°C gekühlt. Dazu wurde unter Rühren eine Lösung von 42 g N^{ε}-Z-L-Lysin in 225 ml 2N Natronlauge bei 0°C langsam zugetropft. Nach 2 Stunden wurde die Kühlung abgestellt und über Nacht weitergerührt. Dann wurde das Reaktionsgemisch während 2 Stunden bei 50°C gehalten und anschliessend wurden 450 ml 1N Salzsäure zugesetzt. Nachdem das Gemisch abgekühlt war wurden die ausgeschiedenen Kristalle abfiltriert. Das Produkt wurde in 1N Natronlauge gelöst und mit der gleichen Menge 1N Salzsäure erneut gefällt und abfiltriert. Es wurden 40 g N-[5-Benzyloxycarbonylamino-1-carboxypentyl]-iminodiessigsäure in Form weisser Kristalle, Smp. 172-174°C (Zers.), [α]_{D} = +9,9° (c = 1; 0,1 N NaOH), erhalten.

7,9 g des erhaltenen Lysinderivats wurden in 49 ml 1N Natronlauge gelöst und nach Zusatz einer Spatelspitze 5% Pd/C bei Raumtemperatur und Normaldruck hydriert. Der Katalysator wurde abfiltriert und das Filtrat eingedampft. Es resultierten 6,2 g N-[5-Amino-1-carboxypentyl]-iminodiessigsäure, deren Struktur, NH₂-(CH₂)₄-CH(COOH)-N(CH₂COOH)₂, durch das NMR-Spektrum bestätigt wurde.

100 ml Sepharose® CL-6B (Pharmacia) wurden auf einer Glasfilternutsche zweimal mit ca. 500 ml Wasser gewaschen und dann in einem 500 ml Rundkolben mit 16 ml 4N Natronlauge und 8,22 ml Epibromhydrin 4 Stunden bei 30°C umgesetzt. Das Totalvolumen des Reaktionsgemisches war 200 ml. Anschliessend wurde die aktivierte Sepharose abfiltriert, mit Wasser neutral gewaschen und zurück in das Reaktionsgefäss transferiert. 6,5 g N-[5-Amino-1-carboxypentyl]-iminodiessigsäure wurden in 50 ml Wasser gelöst und zusammen mit 10,6 g festem Soda zur aktivierten Sepharose gegeben. Das Gemisch wurde bei 60°C über Nacht langsam gerührt. Das resultierende Chelatharz mit der Formel [Sepharose® CL-6B]-O-CH₂-CH(OH)-CH₂-NH-(CH₂)₄-CH(COOH)-N(CH₂COOH)₂ (NTA-Harz) wurde anschliessend in einer Chromatographiesäule nacheinander mit 500 ml Wasser, 100 ml wässrigem NiSO₄·6H₂O (2 Gew.-%), 200 ml Wasser, 200 ml 0,2M Essigsäure (enthaltend 0,2M NaCl und 0,1 Gew./Vol.% Tween 20) und 200 ml Wasser gewaschen. Die Nickelionenkonzentration des resultierenden Chelatharzes der Formel [Sepharose® CL-6B]-O-CH₂-CH(OH)-CH₂-NH-(CH₂)₄-CH(COOH)-N(CH₂COO⁻)₂Ni²⁺ betrug etwa 7,1 Mikromol/ml.

### Beispiel 18

### Metallchelat Affinitäts-Chromatographie mit gereinigtem IFN-γ

Eine Säule (⌀ 1,6 cm, Länge = 7,0 cm) wurde mit metallfreiem Chelatharz der Formel [Sepharose® CL-6B]-O-CH₂-CH(OH)-CH₂-NH-(CH₂)₄-CH(COOH)-N(CH₂COOH)₂ (NTA-Harz) gefüllt und das Harz durch Spülen mit dem dreifachen Säulenvolumen 0,1M NiSO₄·5H₂O und anschliessendes Waschen mit dem dreifachen Säulenvolumen 0,2M Essigsäure in die Nickelform gebracht. Anschliessend wurde mit 0,1M Tris·HCl Puffer (pH 7,5) und 0,5M NaCl äquilibriert (Durchfluss jeweils 60 ml/Std.).

1 mg gereinigtes IFN-γ (Beisp. 3, Aminosäuresequenz siehe Abb.40) wurde in 3 ml Aequilibrierungspuffer aufgenommen und auf die Säule aufgetragen. Mittels Enzymimmunoassay [Gallati, H., J. Clin. Chem. Clin. Biochem. 20, 907-914 (1982)] konnte nachgewiesen werden, dass trotz der beiden proteininternen Strukturelemente Gly-His-Ser und Ile-His-Glu keine Bindung an der NTA-Säule erfolgte.

### Beispiel 19

### Reinigung von His,His-Xa-IFN-γ mittels NTA-Harz

E. coli M15 Zellen, enthaltend die Plasmide pDMI,1 und pHis,His-Xa-IFN-γ (Beisp. 6), wurden in 1 Liter LB-Medium, enthaltend 100 »g/ml Ampicillin und 25 »g/ml Kanamycin, bei 37°C bis zu einer optischen Dichte von OD₆₀₀ = 0,6 wachsen gelassen. Dann wurde IPTG hinzugegeben (Endkonzentration 0,5mM) und die Zellen wurden für weitere 4 Stunden inkubiert. Anschliessend wurden die Zellen durch Zentrifugation (4000 x g, 10 min, 4°C) vom Kulturmedium abgetrennt (5 g Nassgewicht) und mit 15 ml 7M Guanidin·HCl und 0,01M Natriumborat (pH 8) aufgeschlossen (1 Std, 4°C, Magnetrührer). Der so gewonnene Rohextrakt wurde zentrifugiert (10'000 x g, 15′, 4°C), der Ueberstand 10-fach mit 0,1M Tris·HCl Puffer (pH 7,5) und 0,5M NaCl verdünnt, abermals zentrifugiert (10'000 x g, 15′, 4°C) und auf die gleiche, wie in Beispiel 18 beschriebene NTA-Säule aufgepumpt. Anschliessend wurde die Säule mit Aequilibrierungspuffer so lange gewaschen, bis der UV-Detektor (280 mm) wieder den Basiswert anzeigte. Die Elution des His,His-Xa-IFN-γ erfolgte durch Senken des pH-Wertes auf 5,5. Mittels Enzymimmunoassay [Gallati, H., supra] konnte nachgewiesen werden, dass dieses Protein quantitativ von der NTA-Säule absorbiert wurde und erst durch Senken des pH-Wertes eluiert wurde. Mittels SDS-Polyacrylamidgelelektrophorese und RP-18 HPLC konnte nachgewiesen werden, dass es sich bei dem erhaltenen Protein um reines His,His-IFN-γ (Reinheit > 90%) handelte. Die erwartete aminoterminale Sequenz Met-His-Ala-Gly-Ile-Glu-Gly-Arg-Gln... wurde durch Edman-Abbau bestätigt.

### Beispiel 20

### Reinigung von His,His-Ek-IFN-γ(-8) mittels NTA-Harz

His,His-Ek-IFN-γ(-8) (Beisp.7) wurde in zu Beispiel 19 analoger Weise in E. coli exprimiert, extrahiert und über die NTA-Säule gereinigt. Dieses Fusionsprotein wurde ebenfalls bei pH 7,5 an die NTA-Säule gebunden und durch Senkung des pH-Wertes auf 5,5 in reiner Form eluiert (Reinheit > 90%). Die erwartete Sequenz Met-His-His-Ala-Gly-Asp-Asp-Asp-Asp-Lys-Gln.... wurde durch Edman-Abbau bestätigt.

### Beispiel 21

### Reinigung von His-His-Xa-IFN-γ(-8)(Asn) mittels NTA-Harz

His,His-Xa-IFN-γ(-8)(Asn) (Beisp. 8) wurde in zu Beispiel 19 analoger Weise in E. coli exprimiert, extrahiert und über die NTA-Säule gereinigt. Dieses Protein wurde ebenfalls bei pH 7,5 an die NTA-Säule gebunden und durch Senkung des pH-Wertes auf 5,5 in reiner Form eluiert (Reinheit > 90%).

1 mg des so erhaltenen His-His-Xa-IFN-γ(-8)(Asn) wurde gegen 0,1M Tris·HCl (pH 7,5), 0,5M NaCl und 1mM CaCl₂ dialysiert. Das Dialysat (5 ml) wurde mit 100 »l (= 1U) Gerinnungsfaktor Xa versetzt (Boehringer/Mannheim) und für 16 Stunden bei 22°C inkubiert. Der enzymatische Abbau des His,His-Affinitätspeptids wurde mittels SDS-Polyacrylamidgelelektrophorese nachgewiesen.

Zwecks Abtrennung von Salzen, Rinderserumalbumin (Bestandteil des käuflichen Faktor Xa Präparates) und Faktor Xa wurde die Inkubationsmischung zuerst gegen Wasser dialysiert, dann lyophilisiert und anschliessend an einer RP-18 HPLC-Säule (Nucleosil 5C18 Säule von Brownlee Labs, Fliessmittel 0,1% Trifluoressigsäure, Gradient mit Acetonitril, Durchfluss 1 ml/min) chromatographiert. Das resultierende gereinigte Protein wurde dann vom Lösungsmittel befreit und einem Edman-Abbau unterworfen. Mittels dieser Methode konnte die erwartete aminoterminale Sequenz Gln-Asn-Pro-Tyr..... bestätigt werden.

Dieses Experiment zeigt, dass die Affinitätssequenz am NH₂-Terminus des His-His-Xa-IFN-γ(-8)(Asn) nach der Metallchelat-Affinitätschromatographie sauber abgespalten werden kann.

### Beispiel 22

### Reinigung von (His)₆-mDHFR mittels NTA-Harz in 6M Guanidin·HCl

(His)₆-mDHFR (Beispiel 9) wurde in zu Beispiel 19 analoger Weise in E. coli exprimiert. Die Zellen wurden mit 6M Guanidin·HCl in 0,1M Natriumphosphatpuffer (pH 8,0) aufgeschlossen (5 ml Pufferlösung pro 1 g Zellen, 1 Std., 22°C, Magnetrührer). Der so gewonnene Rohextrakt wurde anschliessend zentrifugiert und der Ueberstand auf die gleiche, wie in Beispiel 18 beschriebene NTA-Säule aufgepumpt. Mit Ausnahme der verwendet Pufferlösungen wurde die Chromatographie analog zu Beispiel 19 durchgeführt. Die verwendeten Puffer enthielten jeweils 6M Guanidin·HCl in 0,1M Natriumphosphatpuffer mit den folgenden pH-Werten: pH 8,0 zum Auftragen der Proteine, pH 6,0 zum Auswaschen der nicht gebundenen E. coli-Proteine und pH 4,5 zur Elution des (His)₆-mDHFR. Das erhaltene Eluat wurde gegen Wasser dialysiert und anschliessend lyophilisiert. Mittels SDS-Polyacrylamidgelektrophorese konnte nachgewiesen werden, dass es sich bei dem erhaltenen Protein um reines (His)₆-mDHFR (Reinheit >90%) handelte. Die erwartete Sequenz Met-Arg-Gly-Ser-His-His-His-His-His-His-Gly-Ser-Ile-Met... wurde durch Edmann-Abbau bestätigt.

### Beispiel 23

### Reinigung von (His)₄-mDHFR-(His)₄ mittels NTA-Harz in 6M Guanidin·HCl

(His)₄-mDHFR-(His)₄ (Beispiel 16) wurde in zu Beispiel 19 analoger Weise in E. coli exprimiert, extrahiert und über die NTA-Säule gereinigt. Anstelle des in Beispiel 22 verwendeten Stufengradienten wurde ein linearer pH-Gradient (pH 8,0 bis pH 4.0, 2 Stunden) zur Elution verwendet. (His)₄-mDHFR-(His)₄-Fusionsprotein wurde bei pH 4,9 eluiert und wies eine Reinheit von mindestens 90% auf.

### Beispiel 24

### Reinigung von Met-mDHFR-(His)₆ mittels NTA-Harz in 6M Harnstoff

Met-mDHFR-(His)₆ (Beispiel 14) wurde in zu Beispiel 19 analoger Weise in E. coli exprimiert. Die abzentrifugierten Zellen wurden mit 6M Harnstoff in 0,05M Natriumphosphatpuffer (pH 7,5) (1 g Zellen pro 10 ml Pufferlösung) und Ultraschall (10 Minuten) extrahiert. Nach dem Abzentrifugieren der Zelltrümmer wurde der Ueberstand auf eine mit Extraktionspuffer äquilibrierte NTA-Säule (4,5 cm x 2,6 cm) aufgetragen. Nach dem Waschen der Säule mit Extraktionspuffer wurde das Met-mDHFR-(His)₆-Fusionprotein mit einem linearen pH-Gradienten von pH 7,5 (Extraktionspuffer) bis pH 4,8 (0,05M Natriumphosphatpuffer enthaltend 6M Harnstoff) während 5 Stunden und einer Pumpgeschwindigkeit von 18 ml pro Stunde eluiert. Die Fraktionen, welche Protein enthielten, wurden mittels SDS-Polyacrylamidgelektrophorese analysiert. Es wurden 9 mg Met-mDHFR-(His)₆-Fusionsprotein mit einer Reinheit >90% erhalten.

### Beispiel 25

### Reinigung von Met-mDHFR-(His)₂ mittels NTA-Harz

Met-mDHFR-(His)₂ (Beispiel 15) wurde in zu Beispiel 19 analoger Weise in E. coli exprimiert. Die abzentrifugierten Zellen wurden in 0,05M Kaliumphosphatpuffer (pH 8,0), enthaltend 0,1M Kaliumchlorid und 0,1% Tween 20, während 15 Minuten im Eisbad mit Ultraschall behandelt (1 g Zellen pro 10 ml Pufferlösung). Anschliessend wurden die Zelltrümmer abzentrifugiert und der klare Ueberstand auf eine mit Extraktionspuffer äquilibrierte NTA-Säule (4,6 cm x 2,6 cm) aufgetragen. Die Säule wurde mit Extraktionspuffer gewaschen und das Met-mDHFR-(His)₂-Fusionsprotein mit einem linearen pH-Gradienten von pH 8,0 (Extraktionspuffer) bis pH 5,0 (0,05M Kaliumphosphatpuffer enthaltend 0,1M Kaliumchlorid und 0,1% Tween 20) während 10 Stunden und einer Pumpgeschwindigkeit von 50 ml pro Stunde eluiert. Die Gipfel-Fraktionen des Eluates wurden mittels SDS-Polyacrylamidgelelektrophorese analysiert. Es wurden 7 mg Met-mDHFR-(His)₂-Fusionsprotein mit einer Reinheit von >85% erhalten.

3 mg des so erhaltenen Fusionsproteins Met-mDHFR-(His)₂ wurden gegen 0,05M Tris·HCl (pH 6,0) bei 6°C dialysiert. Die Proteinlösung wurde dann mit 0,5M NaOH auf pH 9,0 eingestellt und bei 37°C in Gegenwart von 8,5 Einheiten Carboxypeptidase A aus Rinderpankreas (Serva, Feinbiochemica, Heidelberg BRD) inkubiert. Nach 0, 15, 30, 90 und 180 Minuten wurden Proben entnommen und mittels HPLC auf ihren Histidingehalt analysiert. Nach 480 Minuten wurde der pH-Wert auf 8,0 abgesenkt und das Reaktionsgemisch auf eine, mit 0,05M Kaliumphosphatpuffer (pH 8) äquilibrierte NTA-Säule gepumpt. Das im Reaktionsgemisch enthaltene Protein wurde mittels SDS-Polyacrylamidgelektrophorese im Durchfluss nachgewiesen. Zusätzlich wurde in den Proben, welche nach 15, 30, 90 und 180 Minuten aus der Proteinlösung entnommen worden waren, eine mit der Zeit zunehmende Menge von Histidinresten nachgewiesen.

Dieses Experiment zeigt, dass die Affinitätssequenz am Carboxylterminus nach der Reinigung am NTA-Harz sauber entfernt werden kann.

## Patentansprüche

1. Fusionsproteine bestehend aus einem oder zwei Affinitätspeptiden, welche mindestens zwei direkt nebeneinander stehende Histidinreste enthalten und eine hohe Affinität für einen Liganden haben, und einem an diese Affinitätspeptide direkt oder indirekt gebundenen biologisch aktiven Polypeptid oder Protein.

2. Fusionsproteine gemäss Anspruch 1, worin die Affinitätspeptide die Formel
R¹-(His)₂₋₄-R²
aufweisen,
worin R¹ Wasserstoff, eine Aminosäure oder eine Sequenz von mehreren Aminosäuren ist, R² Q, Q-Ile-Glu-Gly-Arg- oder Q-Asp-Asp-Asp-Asp-Lys- darstellt und Q eine Peptidbindung, eine Aminosäure oder eine Sequenz von mehreren, max. 30 Aminosäuren ist.

3. Fusionsproteine gemäss Anspruch 1 oder 2, worin die Affinitätspeptide eine Peptidsequenz der Formeln 
Met-His-His,
Met-His-His-His,
Met-His-His-His-His,
Met-His-His-His-His-His,
Met-His-His-His-His-His-His,
Met-His-His-Ala-Gly-Ile-Glu-Gly-Arg
oder Met-His-His-Ala-Gly-Asp-Asp-Asp-Asp-Lys
 aufweisen.

4. Fusionsproteine gemäss einem der Ansprüche 1-3, worin ein Affinitätspeptid direkt oder indirekt an die aminoterminale oder die carboxyterminale Aminosäure des biologisch aktiven Polypeptids oder Proteins gebunden ist.

5. Fusionsproteine gemäss einem der Ansprüche 1-3, worin ein Affinitätspeptid direkt oder indirekt an die aminoterminale Aminosäure und ein weiteres an die carboxyterminale Aminosäure des biologisch aktiven Polypeptids oder Proteins gebunden ist.

6. Fusionsproteine gemäss einem der Ansprüche 1-5, worin die Affinitätspeptide immobilisierte Nickelionen komplexieren.

7. Fusionsproteine gemäss einem der Ansprüche 1-6, worin das biologisch aktive Polypeptid oder Protein die Aminosäuresequenz eines Human-Immun-Interferons oder Teilsequenzen davon oder die Aminosäuresequenz der Dihydrofolatreduktase der Maus aufweist.

8. Ein bakteriell hergestelltes Fusionsprotein gemäss einem der Ansprüche 1-7.

9. Ein durch E. coli hergestelltes Fusionsprotein gemäss einem der Ansprüche 1-7.

10. Ein Fusionsprotein gemäss einem der Ansprüche 1-9 in homogener Form.

11. Gene, die für ein Fusionsprotein gemäss einem der Ansprüche 1-9 kodieren.

12. Expressionsvektoren, in denen ein Gen gemäss Anspruch 11 operativ an eine Expressionskontrollsequenz gebunden ist.

13. Expressionsvektoren gemäss Anspruch 12, die in einem gram-negativen Bakterium replizieren können.

14. Expressionsvektoren gemäss Anspruch 13, die in E. coli replizieren können.

15. Mit einem Expressionsvektor gemäss den Ansprüchen 12-14 transformiertes Bakterium.

16. Mit einem Expressionsvektor gemäss den Ansprüchen 12-14 transformierter E. coli Stamm.

17. Mit einem Expressionsvektor gemäss den Ansprüchen 12-14 transformierter E. coli M15 Stamm.

18. Verfahren zur Reinigung eines Fusionsproteins gemäss einem der Ansprüche 1-9, dadurch gekennzeichnet, dass man eine Lösung enthaltend genanntes Fusionsprotein mit einem Metallchelatharz folgender Struktur
Trägermatrix-Spacer-NH-(CH₂)_{X}-CH(COOH)-N(CH₂COO⁻)₂ Ni²⁺
worin X 2, 3 oder 4 bedeutet,
in Kontakt bringt und das genannte Fusionsprotein durch Behandlung des beladenen Harzes mit einer Waschflüssigkeit eluiert.

19. Verfahren gemäss Anspruch 18, dadurch gekennzeichnet, dass es sich bei der Trägermatrix um Sepharose® CL-6B handelt.

20. Verfahren gemäss Anspruch 18 oder 19, dadurch gekennzeichnet, dass der Spacer -O-CO- oder -O-CH₂-CH(OH)--CH₂- ist.

21. Verfahren zur Reinigung eines biologisch aktiven Polypeptids oder Proteins, dadurch gekennzeichnet, dass man es als Fusionsprotein gemäss einem der Ansprüche 1-9 mittels eines Verfahrens gemäss einem der Ansprüche 18-20 reinigt und dann durch selektive Abspaltung das Affinitätspeptid entfernt.

22. Verfahren gemäss Anspruch 21, dadurch gekennzeichnet, dass zur Abspaltung des Affinitätspeptids eine Protease verwendet wird.

23. Verfahren gemäss Anspruch 22, dadurch gekennzeichnet, dass es sich bei der Protease um den Faktor Xa handelt.

24. Impfstoffe enthaltend ein Fusionsprotein gemäss einem der Ansprüche 1-10 und ein physiologisch verträgliches Trägermaterial.

25. Reagenzien zur Bestimmung von Infektionskrankheiten enthaltend ein Fusionsprotein gemäss einem der Ansprüche 1-10.

26. Verwendung eines Fusionsproteins gemäss einem der Ansprüche 1-9 zur Reinigung eines biologisch aktiven Polypeptids mittels eines Verfahrens gemäss einem der Ansprüche 21-23.

27. Expressionsvektoren, in denen eine Nukleotidsequenz, die für ein Affinitätspeptid gemäss Anspruch 3 kodiert, operativ an eine Expressionskontrollsequenz gebunden ist.

## Claims

1. Fusion proteins consisting of one or two affinity peptides, which contain at least two directly neighbouring histidine residues and which have high affinity for a ligand, and a biologically active polypeptide or protein linked directly or indirectly to this/these affinity peptide(s).

2. Fusion proteins in accordance with claim 1, wherein the affinity peptide has the formula
R¹-(His)₂₋₄-R²
wherein R¹ represents hydrogen, an amino acid or a sequence of several amino acids, R² represents Q, Q-Ile-Glu-Gly-Arg-or Q-Asp-Asp-Asp-Asp-Lys- and Q is a peptide bond, an amino acid or a sequence of several, max. 30, amino acids.

3. Fusion proteins in accordance with claim 1 or 2, wherein in the affinity peptide has a peptide sequence of the formula

Met-His-His
Met-His-His-His
Met-His-His-His-His
Met-His-His-His-His-His
Met-His-His-His-His-His-His
Met-His-His-Ala-Gly-Ile-Glu-Gly-Arg
or Met-His-His-Ala-Gly-Asp-Asp-Asp-Asp-Lys.

4. Fusion proteins in accordance with any one of claims 1-3, wherein one affinity peptide is linked directly or indirectly to the amino terminal or the carboxy terminal amino acid of the biologically active polypeptide or protein.

5. Fusion proteins in accordance with any one of claims 1-3, wherein one affinity peptide is linked directly or indirectly to the amino terminal amino acid of the biologically active polypeptide or protein and a further affinity peptide is linked to the carboxy terminal amino acid of the biologically active polypeptide or protein.

6. Fusion proteins in accordance with any one of claims 1-5, wherein the affinity peptides complex immobilized nickel ions.

7. Fusion proteins in accordance with any one of claims 1-6, wherein the biologically active polypeptide or protein has the amino acid sequence of a human immune interferon or partial sequences thereof or the amino acid sequence of mouse dihydrofolate reductase.

8. A bacterially prepared fusion protein in accordance with any one of claims 1-7.

9. A fusion protein in accordance with any one of claims 1-7 prepared by E. coli.

10. A fusion protein in accordance with any one of claims 1-9 in homogeneous form.

11. Genes which code for a fusion protein in accordance with any one claims 1-9.

12. Expression vectors in which a gene in accordance with claim 11 is operatively linked to an expression control sequence.

13. Expression vectors in accordance with claim 12 which can replicate in a gram-negative bacterium.

14. Expression vectors in accordance with claim 13 which can replicate in E. coli.

15. A bacterium transformed with an expression vector in accordance with claims 12-14.

16. An E. coli strain transformed with an expression vector in accordance with claims 12-14.

17. E. coli M15 strain transformed with an expression vector in accordance with claims 12-14.

18. A process for the purification of a fusion protein in accordance with any one of claims 1-9, characterized by bringing a solution containing the said fusion protein into contact with a metal chelate resin of the following structure
Carrier matrix-spacer-NH-(CH₂)_{X}-CH(COOH)-N(CH₂COO⁻)₂ Ni²⁺
wherein X signifies 2, 3 or 4,
and eluting the said fusion protein by treating the loaded resin with a wash liquid.

19. A process in accordance with claim 18, wherein the carrier matrix is Sepharose® CL-6B.

20. A process in accordance with claim 18 or 19, wherein the spacer is -O-CO-or -O-CH₂-CH(OH)-CH₂-.

21. A process for the purification of a biologically active polypeptide or protein, characterized by purifying it as a fusion protein in accordance with any one of claims 1-9 by means of a process in accordance with any one of claims 18-20 and then removing the affinity peptide by selective cleavage.

22. A process in accordance with claim 21, characterized in that a protease is used for the cleavage of the affinity peptide.

23. A process in accordance with claim 22, characterized in that the protease is factor Xa.

24. Vaccines containing a fusion protein in accordance with any one of claims 1-10 and a physiologically compatible carrier material.

25. Reagents for the determination of infectious diseases, containing a fusion protein in accordance with any one of claims 1-10.

26. The use of a fusion protein in accordance with any one of claims 1-9 for the purification of a biologically active polypeptide by means of a process in accordance with any one of claims 21-23.

27. Expression vectors in which a nucleotide sequence which codes for an affinity peptide in accordance with claim 3 is operatively linked to an expression control sequence.

## Revendications

1. Protéines fusionnées consistant en un ou deux peptides d'affinité, qui contiennent au moins deux restes histidine directement vicinaux et ont une affinité élevée pour un ligand, et en un polypeptide ou une protéine biologiquement actif, lié directement ou indirectement à ces peptides d'affinité.

2. Protéines fusionnées selon la revendication 1, dans lesquelles les peptides d'affinité répondent à la formule
R¹ - (His)₂₋₄ - R²
où R¹ est l'hydrogène, un acide aminé ou une séquence de plusieurs acides aminés, R² représente Q, Q-Ile-Glu-Gly-Arg- ou Q-Asp-Asp-Asp-Asp-Lys- et Q est une liaison peptidique, un acide aminé ou une séquence de plusieurs , au maximum 30, acides aminés.

3. Protéines fusionnées selon l'une des revendications 1 ou 2, dans lesquelles les peptides d'affinité présentent une séquence peptidique répondant aux formules

Met-His-His,
Met-His-His-His,
Met-His-His-His-His,
Met-His-His-His-His-His,
Met-His-His-His-His-His-His,
Met-His-His-Ala-Gly-Ile-Glu-Gly-Arg
ou Met-His-His-Ala-Gly-Asp-Asp-Asp-Asp-Lys.

4. Protéines fusionnées selon l'une quelconque des revendications 1-3, dans lesquelles un peptide d'affinité est lié directement ou indirectement à l'acide aminé aminoterminal ou à l'acide aminé carboxyterminal du polypeptide ou de la protéine biologiquement actif.

5. Protéines fusionnées selon l'une quelconque des revendications 1-3, dans lesquelles un peptide d'affinité est lié directement ou indirectement à l'acide aminé aminoterminal et un autre à l'acide aminé carboxyterminal du polypeptide ou de la protéine biologiquement actif.

6. Protéines fusionnées selon l'une quelconque des revendications 1-5, dans lesquelles les peptides d'affinité complexent des ions nickel immobilisés.

7. Protéines fusionnées selon l'une quelconque des revendications 1-6, dans lesquelles le polypeptide ou la protéine biologiquement actif présente la séquence d'acides aminés d'un interféron immunitaire humain ou des séquences partielles de celui-ci ou la séquence d'acides aminés de la dihydrofolate-réductase de souris.

8. Protéine fusionnée préparée par voie bactérienne selon l'une quelconque des revendications 1-7.

9. Protéine fusionnée préparée au moyen d'E. coli selon l'une quelconque des revendications 1-7.

10. Protéine fusionnée selon l'une quelconque des revendications 1-9 sous une forme homogène.

11. Gènes, qui codent pour une protéine fusionnée selon l'une quelconque des revendications 1-9.

12. Vecteurs d'expression, dans lesquels un gène selon la revendication 11 est lié de façon opérationnelle à une séquence de régulation d'expression.

13. Vecteurs d'expression selon la revendication 12, qui peuvent se répliquer dans une bactérie Gram négative.

14. Vecteurs d'expression selon la revendication 13, qui peuvent se répliquer dans E. coli.

15. Bactérie transformée avec un vecteur d'expression selon l'une quelconque des revendications 12-14.

16. Souche d'E. coli transformée avec un vecteur d'expression selon l'une quelconque des revendications 12-14.

17. Souche M15 d'E. coli transformée avec un vecteur d'expression selon l'une quelconque des revendications 12-14.

18. Procédé pour la purification d'une protéine fusionnée selon l'une quelconque des revendications 1-9, caractérisée en ce qu'on met en contact une solution contenant ladite protéine fusionnée avec une résine de chélate de métal ayant la structure suivante
matrice support-espaceur-NH-(CH₂)ₓ-CH(COOH)-N(CH₂COO⁻)₂ Ni²⁺
où X est le nombre 2, 3 ou 4,
et on élue la protéine fusionnée susdite par traitement de la résine chargée avec un fluide de lavage.

19. Procédé selon la revendication 18, caractérisé en ce qu'il s'agit pour la matrice support de Sepharose® CL-6B.

20. Procédé selon l'une des revendications 18 ou 19, caractérisé en ce que l'espaceur est -O-CO- ou -O-CH₂-CH(OH)-CH₂-.

21. Procédé pour la purification d'un polypeptide ou d'une protéine biologiquement actif, caractérisé en ce qu'on le purifie sous forme de protéine fusionnée selon l'une quelconque des revendications 1-9 au moyen d'un procédé selon l'une quelconque des revendications 18-20 et ensuite on élimine le peptide d'affinité par séparation sélective.

22. Procédé selon la revendication 21, caractérisé en ce qu'on utilise une protéase pour la séparation du peptide d'affinité.

23. Procédé selon la revendication 22, caractérisé en ce qu'il s'agit, en ce qui concerne la protéase, du facteur Xa.

24. Substances inoculantes contenant une protéine fusionnée selon l'une quelconque des revendications 1-10 et une matière support physiologiquement acceptable.

25. Réactifs pour la détermination de maladies infectieuses contenant une protéine fusionnée selon l'une quelconque des revendications 1-10.

26. Utilisation d'une protéine fusionnée selon l'une quelconque des revendications 1-9 pour la purification d'un polypeptide biologiquement actif au moyen d'un procédé selon l'une quelconque des revendications 21-23.

27. Vecteurs d'expression, dans lesquels une séquence nucléotidique, qui code pour un peptide d'affinité selon la revendication 3, est lié de façon opérationnelle à une séquence de régulation d'expression.
